# EUROPEAN PATENT APPLICATION

(11) **EP 2 186 893 A1**
(43) Date of publication of application: **19.05.2010**
(21) Application number: 08778269.4
(22) Date of filing: 11.07.2008
(51) Int. Cl.: C12N 15/09, A61K 39/29, A61P 1/16, A61P 31/12, C07K 16/08, C12N 5/10, C12N 7/00, C12N 7/02

(54) **PRODUCTION AND USE OF EPITOPE-TAGGED HEPATITIS C VIRUS PARTICLE**

(30) Priority: 13.07.2007 JP 2007184587
(71) Applicant: JAPAN as represented by DIRECTOR GENERAL OF NATIONAL INSTITUTE OF INFECTIOUS DISEASES, Tokyo 162-8640 (JP); Tokyo Metropolitan Organization for Medical Research, Tokyo 163-8001 (JP); Toray Industries, Inc., Tokyo 103-8666 (JP)
(72) Inventor: WAKITA, Takaji, Tokyo 107-0062 (JP); SUZUKI, Tetsuro, Nishitokyo-shi Tokyo 202-0022 (JP); TAKAHASHI, Hitoshi, Kamakura-shi Kanagawa 248-8555 (JP)
(74) Representative: Kador, Ulrich
(86) International application number: PCT/JP2008/062977
(87) International publication number: WO 2009/011413

(57) **Abstract**

This invention relates to a nucleic acid comprising a nucleic acid sequence encoding an epitope tag peptide in the hypervariable region 1 of the E2 protein of naturally occurring or chimeric hepatitis C virus (HCV) genome, an epitope-tagged HCV particle encoded by such nucleic acid, and a method for purifying the HCV particles at a high purity in a simple manner with the use of an anti-epitope tag antibody-immobilized support.

## Description

### Technical Field

The present invention relates to epitope-tagged hepatitis C virus particles, a vector used for producing such viruses, and a cell line that produces such virus particles. The present invention also relates to a method for purifying such virus particles, a vaccine obtained by inactivating such virus particles, and an anti-hepatitis C virus antibody to such virus particles.

### Background Art

Hepatitis C virus (which may be simply referred to as "HCV" hereinafter) was found and identified as a major causative virus of non-A and non-B hepatitis (Choo, QL. et al., Science, 244: 359-362, 1989), and highly sensitive methods for detecting HCV had been established. Thus, the number of new HCV patients caused by blood transfusion has dramatically decreased. However, the number of virus carriers in Japan is deduced to be over 2,000,000 and over 170,000,000 in the world, including so-called virus carriers who have not yet developed hepatitis symptoms. This is mainly because the rate of chronicity of hepatitis due to HCV infection is as high as 70-80% and because there are no effective antiviral agents other than interferon at present. Further, hepatitis C is a serious infection with poor prognosis, and approximately half of the patients with chronic hepatitis C would unexceptionally experience worsening of symptoms from hepatitis C to cirrhosis and hepatic cancer. Accordingly, development of antiviral agents or vaccines aimed at preventing virus carriers from developing the disease or at eliminating viruses has been awaited.

In order to develop therapeutic agents for HCV, a cell culture system used for studying infectious HCV particles or a mechanism of HCV particle growth is needed. In the past, an available experimentation system that can effectively grow HCV was limited to an animal experimentation system using chimpanzees, which constitutes a major cause for delayed development of therapeutic agents for HCV. In 2005, however, Wakita et al. developed a method for preparing infectious viruses in a cell culture system (WO 05080575 A1, WO 06022422 A1, and Wakita, T. et al., Nat. Med., 11:791-796, 2005), which enabled development of therapeutic agents for HCV using infectious HCV particles.

Under the above circumstances, there are needs for development of a novel technique that can purify infectious HCV particles in a more simple manner with higher purity than conventional techniques for purifying infectious HCV particles, which were produced in a culture cell system (WO 06022422 A1 and Zahn, A. & Allain1, J.P., J. Gen. Virol., 86: 677-685, 2005).

As the method for purifying a protein obtained in a cell culture system at a high purity in a simple manner, a method wherein a protein of interest is expressed as a fusion protein with protein label and the fusion protein is purified with the use of an antibody to the protein label or a molecule that specifically binds thereto, is known.

The protein label is referred to as an epitope tag or simply as a tag. Examples of known epitope tags include FLAG peptide, 3XFLAG peptide, HA peptide, 3XHA peptide, myc peptide, 6XHis peptide, GST polypeptide, MBP polypeptide, PDZ domain polypeptide, tandem affinity purification (TAP) peptide, alkaline phosphatase, and avidin. Such peptides or polypeptides are generally fused to the N- or C-terminus of the protein of interest, and such peptides or polypeptides can be optionally inserted into the protein of interest.

When an epitope tag is added to a protein, however, a biological activity or conformation inherent to the protein might not be always maintained, depending on the type of a protein, the type of an epitope tag, a site of a protein to which an epitope tag is added, or the like. Accordingly, it is necessary to study biological functions of epitope-tagged proteins, for every type of proteins, for every type of epitope tags, or for every site to which an epitope tag is added.

The HCV genome is a single-stranded (+) RNA comprising approximately 9,600 nucleotides. This genomic RNA comprises 5'-untranslated region (also referred to as "5' NTR" or "5' UTR"), a translational region composed of structural regions and non-structural regions, and 3'-untranslated region (also referred to as "3' NTR" or "3' UTR"). In the structural regions, HCV structural proteins are encoded and, in the non-structural regions, a plurality of non-structural proteins are encoded.

Such HCV structural proteins (Core, E1, E2, and p7) and non-structural proteins (NS2, NS3, NS4A, NS4B, NS5A, and NS5B) are first translated as a series of polyproteins from the translational region, subjected to limited degradation by protease, and then released. Among structural proteins, Core is a core protein, and E1 and E2 are envelope proteins. It is known that non-structural proteins are associated with replication of the virus itself, NS2 has metalloprotease activity, and NS3 has serine protease activity (one third of the N-terminal side) and helicase activity (two thirds of the C-terminal side). Further, it is reported that NS4A is a cofactor for protease activity of NS3, and NS5B has RNA-dependent RNA polymerase activity.

HCV is coated with a coat called envelope. The envelope comprises a component derived from the membrane of a host cell and proteins derived from the virus. The proteins that constitute the HCV envelope comprise envelope protein 1 (referred to as "E1"), envelope protein 2 (referred to as "E2"), and p7. In particular, E1 and E2 each have a transmembrane region at the C terminus, E1 and E2 are anchored to the HCV membrane through such transmembrane region, and E1 and E2 are associated with HCV particle formation or infection (Voisset, C. & Dubuisson, Biology of the Cell, 96: 413-420, 2004; and Op De Beeck, A. et al., J. Gen. Virol., 82: 2589-2595, 2001).

There are two regions, at the amino acid terminal side of the E2 protein, having significantly different sequences among virus strains, and they are named hypervariable region 1 (referred to as HVR1) and hypervariable region 2 (referred to as HVR2) (Hijikata, M. et al., Biochem Biophys Res Commun., 175: 220-228, 1991). HVR1 comprises 27 amino acid residues at the amino terminus of the E2 protein, and it is exposed on the surface of the E2 protein, serving as a B cell epitope. While amino acid sequence diversity of HVR1 is 80% at maximum, there are several regions in which amino acids are preserved. Thus, it is considered that a given secondary structure is necessary for virus survival. Further, there are substantially no cysteine residues or no signals for addition of asparagine-binding sugar chains in HVR1. Meanwhile, HVR2 comprises 7 amino acids and it is located in the region at positions 91-97 from the amino terminus of the E2 protein. While HVR2 exists in the genome of the HCV 1b type, it does not exist in HCV of other genotypes.

As an example of HCV in which epitope tags have been inserted into virus particles, the JFH-1 strain (type HCV2a) in which HVR1 of the E2 protein has been substituted with HA peptide is known, and it is confirmed to have infectivity (Wakita, T. et al., Nat. Med., 11: 791-796, 2005). However, there is no report showing that such infectious epitope-tagged HCV particles are highly purified.

### Disclosure of the Invention

The present invention is intended to provide epitope-tagged HCV particles that can be simply purified at a high purity, a method for purifying the same, and a vaccine prepared using such highly purified HCV particles.

The present inventors have studied epitope tag types, which would not affect productivity and infectivity when used for cell culture of HCV particles, and combinations of a HCV protein to which an epitope tag is added with a site of addition, thereby succeeding in attaining the first object of the present invention; i.e., preparation of epitope-tagged HCV particles. Further, the present inventors prepared clones, which produce HCV particles at high levels, and confirmed that the prepared epitope-tagged HCV particles could be highly purified via a single step with the use of an anti-epitope tag antibody column. This has led to the completion of the present invention.

Specifically, the present invention includes (1) to (15) below.
(1) A nucleic acid comprising (a), (b), or (c) below and comprising a nucleic acid sequence encoding an epitope tag peptide at hypervariable region 1 (HVR1) in an E2 protein coding sequence of the (a), (b), or (c):
   (a) the genome of hepatitis C virus (HCV) JFH-1 strain;
   (b) a chimeric hepatitis C virus (HCV) genome comprising: 5'-untranslated region, a Core protein coding sequence, an E1 protein coding sequence, an E2 protein coding sequence, and a p7 protein coding sequence of HCV J6CF strain; and a NS2 protein coding sequence, a NS3 protein coding sequence, a NS4A protein coding sequence, a NS4B protein coding sequence, a NS5A protein coding sequence, a NS5B protein coding sequence, and 3'-untranslated region of the HCV JFH-1 strain; or
   (c) a chimeric hepatitis C virus (HCV) genome comprising: 5'-untranslated region of the JFH-1 strain; a Core protein coding sequence, an E1 protein coding sequence, an E2 protein coding sequence, and a p7 protein coding sequence of HCV TH1 strain; and a NS2 protein coding sequence, a NS3 protein coding sequence, a NS4A protein coding sequence, a NS4B protein coding sequence, a NS5A protein coding sequence, a NS5B protein coding sequence, and 3'- untranslated region of the HCV JFH-1 strain.
(2) The nucleic acid according to (1), wherein the epitope tag peptide comprises the amino acid sequence DYKDDDDK or DYKDHDGDYKDHDIDYKDDDDK.
(3) The nucleic acid according to (1) or (2), wherein the epitope tag peptide further comprises a linker sequence.
(4) The nucleic acid according to (3), wherein the linker sequence comprises GGG sequence, GGGGS sequence, or (GGGGS)₃ sequence, or a sequence comprising deletion, substitution or addition of 1-10 amino acids in a part of the sequence GGG, GGGGS or (GGGGS)₃.
(5) The nucleic acid according to any one of (1) to (4), which is shown in SEQ ID NO: 11, 12, 30, 31, 41, or 42, wherein nucleotide "T" shown in the sequence listing is read as "U" when the nucleic acid is RNA.
(6) The nucleic acid according to any one of (1) to (5), which, when the amino acid subsequent to the end of the sequence of the hypervariable region 1 (HVR1) is designated as an amino acid at position 1, comprises a mutation which is a substitution of asparagine at position 122 or 124 with lysine.
(7) The nucleic acid according to (6), which is shown in SEQ ID NO: 20 or 21 wherein nucleotide "T" shown in the sequence listing is read as "U" when the nucleic acid is RNA.
(8) A vector comprising the nucleic acid according to any one of (1) to (7).
(9) An epitope-tagged hepatitis C virus (HCV) particle comprising the nucleic acid according to any one of (1) to (7) as the genome.
(10) A cell comprising the epitope-tagged hepatitis C virus (HCV) particle according to (9).
(11) The cell according to (10), which is Huh7 or a cell line derived therefrom.
(12) A method for purifying epitope-tagged hepatitis C virus (HCV) particle comprising:
   a step of preparing epitope-tagged HCV particles from the cell according to (10) or (11);
   a step of bringing a solution containing the epitope-tagged HCV particles into contact with an anti-epitope tag antibody-immobilized support and then adsorbing the epitope-tagged HCV particles to the anti-epitope tag antibody-immobilized support; and
   a step of releasing the epitope-tagged HCV particles from the anti-epitope tag antibody-immobilized support to obtain the epitope-tagged HCV particles.
(13) The method of purification according to (12), wherein the releasing step comprises releasing the epitope-tagged HCV particles from the anti-epitope tag antibody-immobilized support using any of a buffer containing epitope tag peptide, a buffer containing no calcium, 0.1M glycine buffer (pH 2.5-4.0), 1M arginine HCl buffer (pH 4.0-5.0), or 0.1-1 mM HCl.
(14) An hepatitis C virus (HCV) vaccine, which is obtained by inactivating the epitope-tagged HCV particle according to (9).
(15) An anti-hepatitis C virus (HCV) antibody to the epitope-tagged HCV particle according to (9).

This description includes all or part of the contents as disclosed in the description and/or drawings of Japanese Patent Application No. 2007-184587, which is a priority document of the present application.

### Brief Description of the Drawings

Fig. 1 shows the structure of HCV E2. N1 to N11 each represent a sugar chain addition site, HVR represents a hypervariable region, and TMD represents a transmembrane domain. Part of the amino acid sequence of E2 HVR1 of J6/JFH-1 (the underlined portion) was removed, and the FLAG-tag sequence (the underlined portion) was inserted therein.
Fig. 2A, Fig. 2B, and Fig. 2C each show the structures of pJ6/JFH-1 (1XFLAG) and pJ6/JFH-1 (3XFLAG) plasmids. Fig. 2B is a continuation of Fig. 2A and Fig. 2C is a continuation of Fig. 2B.
Fig. 3 shows changes in the amount of the HCV Core proteins in the culture supernatant after J6/JFH-1 RNA and J6/JFH-1 (3XFLAG) RNA were introduced into the Huh-7 cells. The amount of the produced Core protein did not change when J6/JFH-1 was introduced. When J6/JFH-1 (3XFLAG) was introduced, the amount of the produced Core protein decreased until day 22 after introduction, but thereafter increasing to an almost same level as that of J6/JFH-1 on day 35.
Fig. 4 shows the expression of HCV Core and E2 proteins on days 4, 17, 30 and 43 after J6/JFH-1 RNA or J6/JFH-1 (3XFLAG) RNA were introduced into Huh-7 cells. When J6/JFH-1 (3XFLAG) was introduced, the molecular masses of the E2 protein on days 30 and 43 were found to be lower than that on day 4.
Fig. 5 shows behavior of the HCV E2 protein which was treated with endoglycosidase (PNGase F) on days 4, 17, 30 and 43 after J6/JFH-1 (3XFLAG) RNA was introduced the Huh-7 cells,. By treating the protein with endoglycosidase, differences in molecular mass as observed in Fig. 4 were no longer observed.
Fig. 6 shows the results of the sequence analysis of the HCV genome 8 and 39 days after introduction of J6/JFH-1 (3XFLAG) RNA. Asparagine (N6), which is a sugar chain addition site in the E2 region, was already substituted with lysine (K) 39 days after RNA introduction.
Fig. 7 shows the amount of HCV Core protein in the solution of purified J6/JFH-1 (3XFLAG) virus. Since HCV Core protein was detected after elution of 1XFLAG peptide or 3XFLAG peptide, purification of HCV particles was confirmed.
Fig. 8 shows contamination of proteins contained in the solution of purified J6/JFH-1 (3XFLAG) virus. The solution of the purified virus contained almost no contaminant proteins.
Fig. 9 shows infectivity of purified J6/JFH-1 (3XFLAG) virus. HCV RNA in the cells was quantified 4 days after infection with viruses. The purified virus was found to have infectivity equivalent to that of unpurified virus.
Fig. 10A, Fig. 10B, and Fig. 10C each show the structures of pJ6/JFH-1 (1XFLAG) N → K and pJ6/JFH-1 (3XFLAG) N → K plasmids. Fig. 10B is a continuation of Fig. 10A and Fig. 10C is a continuation of Fig. 10B.
Fig. 11 shows the structure of HCV E2. N1 to N11 each represent a sugar chain addition site, HVR represents a hypervariable region, and TMD represents a transmembrane domain. Part of the amino acid sequence of E2 HVR1 of JFH-1 (the underlined portion) was removed, and the FLAG-tag sequence (the underlined portion) was inserted therein.
Fig. 12A, Fig. 12B, and Fig. 12C each show the structures of pJFH-1 (1XFLAG) and pJFH-1 (3XFLAG) plasmids. Fig. 12B is a continuation of Fig. 12A and Fig. 12C is a continuation of Fig. 12B.
Fig. 13 shows the structure of HCV E2. N1 to N11 each represent a sugar chain addition site, HVR represents a hypervariable region, and TMD represents a transmembrane domain. Part of the amino acid sequence of E2 HVR1 of pTH/JFH-1 (the underlined portion) was removed, and the FLAG-tag sequence (the underlined portion) was inserted therein.
Fig. 14A, Fig. 14B, and Fig. 14C each show the structures of pTH/JFH-1 (1XFLAG) and pTH/JFH-1 (3XFLAG) plasmids. Fig. 14B is a continuation of Fig. 14A and Fig. 14C is a continuation of Fig. 14B.

### Best Modes for Carrying out the Invention

When implementing the present invention, conventional molecular biological and immunological techniques within the technical scope in the art are to be utilized. Such techniques are obvious to those skilled in the art and have already been thoroughly reported in the published documents (see, for example, Sambrook et al., Molecular Cloning: A Laboratory Manual, vol. 3, 2001; Ed Harlow et al., Antibodies: A Laboratory Manual, 1988).

All publications, patents, and patent applications cited herein are incorporated herein by reference in their entirety.

### 1. Epitope-tagged HCV particles

The term "epitope-tagged HCV particle" as used herein refers to a HCV particle in which an epitope tag peptide has been added in a HCV structural protein. Such epitope-tagged HCV particle can be produced in a cell culture system, and such particle preferably has infectivity.

Production of HCV particles in a cell culture system requires the use of a full-length gene derived from the JFH-1 strain or a chimera gene of the JFH-1 strain and another HCV strain. The fundamental technique thereof is described in WO 04104198 A1, WO 06022422 A1, Wakita, T. et al., Nat. Med. 11: 791-796, 2005, and Lindenbach, BD. et al., Science 309: 623-626, 2005.

Specifically, an embodiment of the gene that can produce HCV particles is the JFH-1 virus genomic RNA comprising 5'- untranslated region, a Core protein coding sequence, an E1 protein coding sequence, an E2 protein coding sequence, a p7 protein coding sequence, a NS2 protein coding sequence, a NS3 protein coding sequence, a NS4A protein coding sequence, a NS4B protein coding sequence, a NS5A protein coding sequence, a NS5B protein coding sequence, and 3'-untranslated region, in order in the 5' →3' direction.

Another embodiment of the gene that can generate HCV particles is a chimeric gene comprising virus genomic RNAs of 2 or more types of HCV strains. An example of such RNA is RNA comprising 5'-untranslated region, a Core protein coding sequence, an E1 protein coding sequence, an E2 protein coding sequence, a p7 protein coding sequence, and a NS2 protein coding sequence of an HCV strain other than the JFH-1; and a NS3 protein coding sequence, a NS4A protein coding sequence, a NS4B protein coding sequence, a NS5A protein coding sequence, a NS5B protein coding sequence, and 3'-untranslated region of the JFH-1 strain, in order in the 5' →3' direction.

Specific examples of genes that can generate HCV particles include the nucleic acids (a), (b), and (c) below:
(a) the genome of the JFH1 strain (i.e., a nucleic acid comprising 5'-untranslated region, a Core protein coding sequence, an E1 protein coding sequence, an E2 protein coding sequence, a p7 protein coding sequence, a NS2 protein coding sequence, a NS3 protein coding sequence, a NS4A protein coding sequence, a NS4B protein coding sequence, a NS5A protein coding sequence, a NS5B protein coding sequence, and 3'-untranslated region of the JFH-1 strain, in order in the 5' →3' direction (see SEQ ID NO: 30: pJFH-1 (1XFLAG), SEQ ID NO: 31: pJFH-1 (3XFLAG));
(b) a chimeric HCV genome comprising: 5'-untranslated region, a Core protein coding sequence, an E1 protein coding sequence, an E2 protein coding sequence, and a p7 protein coding sequence of J6CF strain; and a NS2 protein coding sequence, a NS3 protein coding sequence, a NS4A protein coding sequence, a NS4B protein coding sequence, a NS5A protein coding sequence, a NS5B protein coding sequence, and 3' -untranslated region of the JFH-1 strain, in order in the 5' →3' direction (preferably a chimeric HCV genome comprising: 5'-untranslated region, a Core protein coding sequence, an E1 protein coding sequence, an E2 protein coding sequence, a p7 protein coding sequence, and a sequence encoding the N-terminal 16 amino acids of the NS2 protein encoding region; and a sequence encoding a region from the N-terminal 17^{th} amino acid to the C terminus of the NS2 protein encoding region, a NS3 protein coding sequence, a NS4A protein coding sequence, a NS4B protein coding sequence, a NS5A protein coding sequence, a NS5B protein coding sequence, and 3' -untranslated region of the JFH-1 strain, in order in the 5' →3' direction (see SEQ ID NO: 10: pJ6/JFH-1, SEQ ID NO: 11: pJ6/JFH-1 (1XFLAG), SEQ ID NO: 12: pJ6/JFH-1 (3XFLAG)); and
(c) a chimeric HCV genome comprising: 5'-untranslated region of the JFH1 strain; a Core protein coding sequence, an E1 protein coding sequence, an E2 protein coding sequence, and a p7 protein coding sequence of TH1 strain; and a NS2 protein coding sequence, a NS3 protein coding sequence, a NS4A protein coding sequence, a NS4B protein coding sequence, a NS5A protein coding sequence, a NS5B protein coding sequence, and 3' -untranslated region of the JFH-1 strain, in order in the 5' →3' direction (preferably, a chimeric HCV genome comprising: 5'-untranslated region of the JFH-1 strain; a Core protein coding sequence, an E1 protein coding sequence, an E2 protein coding sequence, a p7 protein coding sequence, and a sequence encoding the N-terminal 33 amino acids of the NS2 protein encoding region of the TH strain; and a sequence encoding a region from the N-terminal 34^{th} amino acid to the C terminus of the NS2 protein encoding region, a NS3 protein coding sequence, a NS4A protein coding sequence, a NS4B protein coding sequence, a NS5A protein coding sequence, a NS5B protein coding sequence, and 3' -untranslated region of the JFH-1 strain, in order in the 5' →3' direction (see SEQ ID NO: 40: pTH/JFH1, SEQ ID NO: 41: pTH/JFH1 (1XFLAG), SEQ ID NO: 42: pTH/JFH1 (3XFLAG)).

In the present invention, a nucleic acid sequence encoding an epitope tag peptide, i.e. DYKDDDDKGGG (SEQ ID NO: 49) or DYKDHDGDYKDHDIDYKDDDDKGGG (SEQ ID NO: 50), is inserted in frame into the hypervariable region 1 (HVR1) of the E2 protein coding sequence of the above HCV genome. Alternatively, part of said region is substituted with said epitope tag peptide to prepare epitope-tagged HCV genome DNA. The "epitope-tagged HCV particles" are produced in a cell culture system using such DNA to obtain highly purified epitope-tagged HCV particles.

### 2. Preparation of epitope-tagged HCV particle

(1) Preparation of HCV genome DNA having an epitope tag in the HCV structural protein

The term "epitope tag" (or simply referred to as "tag") as used herein is not particularly limited, provided that it can be used for labeling HCV. Examples thereof include the FLAG peptide (which may be referred to as the flag peptide or Flag peptide), the 3XFLAG peptide (which may be referred to as the 3xFLAG peptide, 3xFlag peptide, or 3Xflag peptide), the HA peptide, the 3XHA peptide, the myc peptide, the 6XHis peptide, the GST polypeptide, the MBP polypeptide, the PDZ domain polypeptide, the tandem affinity purification (TAP) peptide, alkaline phosphatase, and avidin. In the present invention, the FLAG peptide (the amino acid sequence: DYKDDDDK (SEQ ID NO: 51)) or the 3XFLAFG peptide (the amino acid sequence: DYKDHDGDYKDHDIDYKDDDDK (SEQ ID NO: 52)) is used as the epitope tag peptide, and among them, the 3XFLAG peptide is particularly preferable.

The position at which the epitope tag is added is a HVR1 region of the E2 protein of HCV particle. The epitope tag peptide is inserted in frame (i.e., so that the reading frame is not misaligned) into a region between the amino acids at positions 8 and 19 counted from the N-terminus of the E2 protein having low homology between HCV strains, in the HVR1 region, or alternatively, preferably the epitope tag peptide is inserted into the position at which the N-terminal amino acids 11-17 have been removed.

Where necessary, a suitable linker may be added to the C-terminus of the epitope tag peptide. Used as the linker are the amino acid sequences GGG (SEQ ID NO: 53), GGGGS (SEQ ID NO: 54), and (GGGGS) ×3 (also referred to as (GGGGS)₃), or peptides comprising a deletion, substitution, or addition of about 1-10, preferably 1-5, more preferably 1-2 amino acids in part of said amino acid sequences. Preferable examples are GGG, GGGGS (SEQ ID NO: 54), and (GGGGS)×3, preferably GGG.

An example of a method for introducing a nucleic acid encoding the epitope tag into the HCV genome is a method wherein polymerase chain reaction (PCR) is carried out using synthetic primers containing a nucleic acid sequence encoding the epitope tag and cDNA of the HCV genome as a template, and the resulting PCR product is adequately subjected to restriction enzyme treatment and ligation treatment to recombine the above cDNA into cDNA of the HCV genome of interest. Examples of such procedures are described in Examples below.

HCV strains used are, but not limited to, strains belonging to any of 6 genotypes classified by lineage analysis using the nucleotide sequences of the HCV strains: genotype 1a (e.g., H77 strain (GenBank Accession No. AF011751)), genotype 1b (e.g., J1 strain (GenBank Accession No. D89815), Con1 strain (GenBank Accession No. AJ238799, which may be referred to as the strain Con-1 or con1), TH strain (Wakita, T. et al., J. Biol. Chem., 269, 14205-14210, 1994, JP Patent Publication (kokai) No. 2004-179 A)), genotype 2a (e.g., the JFH-1 strain (GenBank Accession No. AB047639, which may be referred to as "JFH1 strain"), the J6CF strain (GenBank Accession No. AF177036), JCH-1 (GenBank Accession No. AB047640), JCH-2 (GenBank Accession No. AB047641), JCH-3 (GenBank Accession No. AB047642), JCH-4 (GenBank Accession No. AB047643), JCH-5 (GenBank Accession No. AB047644), JCH-6 (GenBank Accession No. AB047645)), genotype 2b (e.g., HC-J8 strain (GenBank Accession No. D01221)), genotype 3a (e.g., NZL1 strain (GenBank Accession No. D17763)), and genotype 3b (e.g., Tr-Kj (GenBank Accession No. D49374)). In the Examples later, a nucleic acid sequence comprising the non-structural protein-encoding sequences and 3'-untranslated region derived from JFH-1 and the 5'- untranslated region and structural protein-encoding sequences derived from TH1, JFH-1, or J6CF, is used as a preferable example of the present invention.

To insert an epitope tag peptide of interest into HVR of HCV, a cDNA of the HCV genomic RNA is cloned into a vector, and PCR is carried out using the vector as a template and using synthetic DNAs encoding an epitope tag peptide as primers. Thus, the cDNA encoding an envelope protein in which an epitope tag has been added to or inserted into a target site, can be obtained.

Furthermore, a cDNA fragment encoding an envelope protein, which has an epitope tag, is isolated after digestion with a suitable restriction enzyme, cDNA of full-length HCV genomic RNA is cloned into a site downstream of a promoter such as a T7 promoter to prepare a vector, which is then digested with the same restriction enzyme as above, and a cDNA fragment encoding an envelope protein, which has the isolated epitope tag sequence, is inserted at the same digestion site. Thereby being able to obtain a full-length HCV cDNA.

The vector into which the cDNA of full-length HCV genomic RNA as used herein has been cloned is capable of producing HCV particles, when the RNA transcribed from the vector is introduced into HCV permissive cells, such as Huh7 cell. The details regarding the structure and preparation thereof are described in WO 04104198 A1, WO 06022422 A1, Wakita, T. et al., Nat. Med. 11: 791-796, 2005, and Lindenbach, BD. et al., Science 309: 623-626, 2005.

### (2) Production of epitope-tagged HCV particles in cell culture system

The RNA may be synthesized from a HCV cDNA having an epitope tag coding sequence under the control of a promoter, which RNA is introduced into a cell to obtain a cell containing epitope-tagged HCV particles, and subsequently the obtained cells are cultured to prepare epitope-tagged HCV particles. The promoter includes, but is not limited to, T7 promoter, SP6 promoter, and T3 promoter, preferably T7 promoter.

To prepare the RNA *in vitro* using, as a template, a nucleic acid into which a HCV cDNA has been introduced under control of T7 promoter and then cloned, a kit such as MEGAscript T7 kit (Ambion) can be employed.

Cells for introducing the RNA are any cells capable of producing HCV particles, such as Huh7, HepG2, IMY-N9, HeLa or 293 cells, or cells which express CD81 and/or Claudin1 genes and are derived from Huh7, HepG2, IMY-N9, HeLa or 293 cells. Among such cells, Huh7 cell or Huh7-derived cell lines are preferably used. Examples of the Huh7-derived cell lines include Huh7.5 and Huh7.5.1.

Examples of methods for introducing the RNA into cells include calcium phosphate coprecipitation, a DEAE-dextran method, lipofection, microinjection, and electroporation. Lipofection and electroporation are preferable, and electroporation is more preferable.

A cDNA may be introduced into cells instead of RNA. In such a case, the HCV cDNA to which an epitope tag coding sequence has been added is operably inserted into a vector comprising an RNA polymerase I promoter and a terminator (Neumann, G. et al., Virology, 202: 477-479, 1994, WO 2007/037428 A1), and the vector is introduced into cells in the same manner as in the case of RNA to express the cDNA in the cells.

The capacity of the cells for virus particle production can be evaluated by use of antibodies to proteins that constitute a HCV particle released in culture solution, such as a Core protein, an E1 protein, and an E2 protein. Also, the capacity of the cells for HCV virus particle production may be indirectly evaluated by amplifying a HCV genomic RNA contained in the HCV virus particles in a culture medium via RT-PCR using specific primers, to detect the RNA.

Whether or not the prepared viruses are infectious can be evaluated by culturing cells into which HCV RNA has been introduced, treating HCV permissive cells (e.g., Huh7) with the supernatant from said culture, and immunologically staining the cells with an anti-Core antibody 48 hours later to count the number of infected cells. Alternatively, the evaluation can be carried out by subjecting the cell extract to electrophoresis on SDS-polyacrylamide gel and detecting Core proteins via Western blotting.

### (3) Obtaining epitope-tagged HCV particles-producing cell lines

For efficient replication of the HCV genome, it is necessary that a mutation occurs in the nucleotide sequence of the genome (Lohmann, V. et al., J. Virol. 75: 1437-1449, 2001). Mutation that enhances replication is referred to as "adaptive mutation." The cells into which the HCV genomic RNA has been introduced as prepared in (2) above may be subjected to subculture to obtain cell lines that continuously produce HCV particles. When such culture is continued, there is a case where adaptive mutation takes place in the HCV genome, resulting in significantly enhancing the production of HCV particles.

When an amino acid subsequent to the end of the HVR1 sequence (Puntoriero, G. et al., EMBO J. 17: 3521-3533, 1998) in the nucleic acids (a) to (c) is designated as the 1^{st} amino acid, the mutation which is a substitution of asparagine, a 122^{nd} or 124^{th} amino acid, with lysine is suitable as a mutation when producing epitope-tagged HCV particles. In the case of the nucleic acid (a) or (b), particularly, the mutation which is a substitution of asparagine, a 124^{th} amino acid, with lysine is more preferable. In the nucleic acid (c), the mutation which is a substitution of asparagine, a 122^{nd} amino acid, with lysine is more preferable. Asparagine at these positions is considered to be modified with a sugar chain, and it is suggested that there is a correlation between no addition of a sugar chain and high productivity of HCV particles.

In the present invention, the adaptive mutation can be attained via subculture as described above, or can be artificially introduced. When it is artificially introduced, mutagenic primers may be designed, and PCR may be carried out using such primers to synthesize DNA into which mutation has been introduced. Also, the commercially available Quick Change II XL Site-Directed Mutagenesis kit (Stratagene) may be used to prepare a mutant.

In the present invention, it is difficult to detect a nucleic acid mutation that is necessary for mutating one amino acid, by hybridization techniques; however, the adaptive mutation can be confirmed by determining the sequence of a HCV gene.

### (4) Purification of epitope-tagged HCV particles

Epitope-tagged HCV particles are purified using a support to which a protein capable of binding to the epitope tag has been bound. The most general protein is an antibody to epitope tag peptide. In the present invention, a preferable epitope tag is FLAG or 3XFLAG peptide, and a support to which an anti-FLAG antibody capable of binding to said peptide, such as an M2 antibody or M5 antibody, has been bound (or immobilized) (available from Sigma) or the like can be used.

A culture supernatant of FLAG epitope-tagged HCV particle-producing cells or 3XFLAG epitope-tagged HCV particle-producing cells or a solution containing HCV particles is applied to a column containing the anti-FLAG antibody-bound support, then the column is washed with phosphate buffer, and HCV particles bound to the anti-FLAG antibody-bound support can be eluted with a buffer containing the FLAG or 3XFLAG peptide. When a support to which an M5 antibody that binds to the FLAG peptide in a calcium-dependent manner has been bound is used, HCV particles are bound with the use of a calcium-containing buffer, and HCV particles can be eluted from the support with the use of a calcium free buffer.

As other elution techniques, general techniques for eluting proteins from antibody-bound supports can be employed (Antibodies: A Laboratory Manual, Cold Spring Harbor Laboratory, 1988). For example, 0.1M glycine buffer (pH 2.5-4.0) or 1M arginine HCl buffer (pH 4.0-5.0) can be used as an elution solution (Ejima, D. et al., Anal. Biochem. 345: 250-257, 2005). Further, 0.1 mM-1 mM hydrochloric acid can be used. To prevent the eluted HCV particles from denaturation, the pH of the buffer after elution is preferably set at around neutral (pH 6.0-8.0).

The thus-purified HCV particles are subjected to electrophoresis on SDS-polyacrylamide gel and HCV-derived proteins are detected by Western blot, whereby it can be determined whether or not the HCV particles are purified.

### 3. Utilization of epitope-tagged HCV particles

### (1) Vaccine

Highly purified epitope-tagged HCV particles are preferably used as a vaccine or as an antigen for preparing anti-HCV antibodies.

Specifically, the prepared epitope-tagged HCV particles can be inactivated by a method known in the art for use thereof. The virus can be inactivated by adding and mixing an inactivator such as formalin, β-propiolactone, or glutardialdehyde in, for example, a virus suspension and allowing the inactivator to react with the virus (Appaiahgari et al., Vaccine, 22: 3669-3675, 2004).

Furthermore, the virus may be irradiated with ultraviolet to eliminate infectivity of virus, thereby being immediately inactivated. Irradiation of ultraviolet leads to inactivation of the virus with little influence on constituted proteins. A source of ultraviolet rays used for inactivation can be a commercially available germicidal lamp, particularly a 15w germicidal lamp, although the source is not limited thereto. In the present invention, HCV particles that are purified by the method described above are used, and methods of inactivation are not limited by a purified or unpurified state. Preferably, a solution containing epitope-tagged HCV particles may be irradiated with ultraviolet at 20 mW/cm² at room temperature for at least 5 minutes to inactivate epitope-tagged HCV particles.

The vaccine of the present invention is in the form of a solution or suspension, containing 10%-95%, preferably 25%-70% active ingredient (virus particles or part thereof), and is formulated for administration. The vaccine can be prepared in a solid state that is suitable for dissolving or suspending it in a solution. The preparation may be emulsified or may be encapsulated in liposomes.

An active immunogenic ingredient, such as HCV particle, is often mixed with a pharmaceutically acceptable excipient which is compatible with the active ingredient. Examples of adequate excipients include water, physiological saline, dextrose, glycerol, ethanol, and mixtures thereof. Further, the vaccine can contain a minor amount of an auxiliary agent(s) (e.g., a humidifier or emulsifier), a pH buffer, and/or an adjuvant that enhances vaccine efficacy, where needed. Examples of the effective adjuvant include, but are not limited to, aluminum hydroxide, N-acetyl-muramyl-L-threonyl-D-isoglutamine (thr-MDP), N-acetyl-nor-muramyl-L-alanyl-D-isoglutamine (referred to as CGP11637 or nor-MDP), N-acetylmuramyl-L-alanyl-D-isoglutaminyl-L-alanine-2-(1'-2'-dipalmitoyl-sn-glycero-3 -hydroxyphosphoryloxy)-ethylamine (referred to as CGP19835A or MTP-PE), and RIBI. RIBI contains three components extracted from bacteria; i.e. monophosphoryl lipid A, trehalose dimycolate, and a cell wall skeleton (HPL+TDM+CWS), in 2% squalene/Tween^{®} 80 emulsion. Efficacy of an adjuvant can be determined by assaying the amount of antibodies resulting from administration of a vaccine comprising HCV particles.

The vaccine of the present invention may generally be administered parenterally, by injection such as subcutaneous injection or intramuscular injection, for example. Examples of other formulations that are suitable as other dosage forms for administration include suppositories and, optionally, oral preparations.

If necessary, one or more compounds having adjuvant activity can be added to the HCV vaccine. An adjuvant is a non-specific stimulant to the immune system. Such substance enhances the immune response of a host against HCV vaccines. Specific examples of adjuvants that are known in the art include Freund's complete and incomplete adjuvants, vitamin E, a nonionic block copolymer, muramyl dipeptide, saponin, mineral oil, vegetable oil, and Carbopol. Examples of adjuvants that are particularly suitable for mucosal application include *E. coli* thermolabile toxin (LT) and Cholera toxin (CT). Examples of other adequate adjuvants include aluminum hydroxide, aluminum phosphate or aluminum oxide, an oil emulsion (e.g., Bayol^{®} or Marcol 52^{®}), saponin, and a vitamin E solubilizate. Accordingly, the vaccine of a preferable embodiment of the present invention comprises an adjuvant.

For example, injections for subcutaneous, intracutaneous, intramuscular, or intravenous administration can be administered, which injections comprise the vaccine of the invention and a pharamaceutically acceptable carrier or diluent, in combination with others such as a stabilizer, a carbohydrate (e.g., sorbitol, mannitol, starch, sucrose, glucose, or dextran), a protein (e.g., albumin or casein), a protein-containing substance (e.g., bovine serum or skimmed milk), and buffer (e.g., phosphate buffer).

The vaccine of the present invention is administered in a manner suitable for a dosage form and in an amount that can exert preventive and/or therapeutic effects. The amount to be administered is generally 0.01 µg-100,000 µg antigen per dose. Such amount varies depending on patients to be treated, the capacity of patients for antibody synthesis in the immune system, and the desired degree of protection. Also, the amount varies depending on the route of administration, such as oral, subcutaeous, intracutaneous, intramuscular, or intravenous administration.

The vaccine of the present invention can be administered according to a single-administration schedule, and preferably according to a multiple-administration schedule. In the multiple-administration schedule, 1-10 separate administrations are performed at the time of initiation of inoculation, and another administration can then be performed with the time interval that is necessary for maintaining and/or enhancing the immune response. For example, the second administration can be performed 1-4 months later. Where needed, administration may be subsequently performed several months later. The administration regimens are, at least partially, determined according to the necessity of an individual, and the regimens depend on the judgment made by a doctor.

The vaccine of the present invention may be administered with another immunosuppressant agent (e.g., immunoglobulin).

Further, the vaccine of the present invention may be administered to a healthy individual to induce an immune response to HCV in such healthy individual, and the vaccine may be used for preventing new HCV infection. Furthermore, the vaccine may be administered to a patient infected with HCV to induce a potent immune response to HCV *in vivo*, and thus the vaccine may be used as a therapeutic vaccine which eliminates HCV.

### (2) Anti-HCV antibody induced with the use of epitope-tagged HCV particle antigens

The epitope-tagged HCV particles of the present invention can be easily purified and are of high purity. Thus, such particles are useful as antigens for preparing antibodies.

Specifically, animal blood serum contains antibodies to impurities. When the HCV particle sample used for animal immunization contains impurities, accordingly, the serum needs to be purified using an HCV antigen column in order to eliminate antibodies to such impurities. In the present invention, however, because the epitope-tagged HCV particles are obtained as highly purified HCV, any purification step is not needed.

The anti-HCV antibody can be prepared by administering the epitope-tagged HCV particles of the present invention to mammalians or birds. Examples of mammalians include mice, rats, rabbits, goats, sheep, horses, cattle, guinea pigs, dromedaries, Bactrian camel, and lama. Dromedaries, Bactrian camel, and lama are suitable for preparing an antibody consisting of the H chain alone. Examples of birds include chickens, geese, and ostriches. The blood serum may be taken from an animal to which the particles of the present invention have been administered, in order to obtain antibodies of interest by well-known methods.

With the use of animal cells immunized with the epitope-tagged HCV particles of the present invention, hybridomas that produce monoclonal antibody-producing cells can be prepared. Methods for producing hybridomas are well-known in the art, and the method described in Antibodies: A Laboratory Manual (Cold Spring Harbor Laboratory, 1988) can be employed.

Monoclonal antibody-producing cells may be prepared via cell fusion or via other means such as introduction of an oncogene (DNA) or immortalization of B lymphocytes via infection with Epstein-Barr virus.

Monoclonal or polyclonal antibodies thus obtained are useful for diagnosis, treatment, and prevention of HCV.

The antibodies prepared using the epitope-tagged HCV particles of the present invention are used for treating or preventing hepatitis C. Further, they are used for preventing HCV infection from donor organs at the time of organ transplantation. Further, the above antibodies can be used in combination with existing antiviral agents, such as interferon and ribavirin.

An effective dose of the anti-HCV antibody can be selected from between 0.001 mg and 1,000 mg per kg body weight. Alternatively, the amount of administration may be 0.01 to 100,000 mg/patient' s body. It should be noted, however, that the amount of a therapeutic agent comprising the anti-HCV antibody prepared using the epitope-tagged HCV particles of the invention is not limited to the above-mentioned amounts.

The administration route of the above therapeutic agent includes, but is not limited to, preferably subcutaneous, intracutaneous, or intramuscular administration, more preferably intravenous administration. Also, the timing for administration of the therapeutic agent is not limited and may be either before or after patients develop clinical symptoms of the disease.

The therapeutic agent comprising, as an active ingredient, the anti-HCV antibody prepared using the epitope-tagged HCV particles of the present invention can be formulated by conventional techniques (Remington's Pharmaceutical Science, Latest Edition, Mark Publishing Company, Easton, U.S.A.), in combination with pharmaceutically acceptable carriers or additives.

Examples of such carriers and pharmaceutical additives include water, pharmaceutically acceptable organic solvents, collagen, polyvinyl alcohol, polyvinyl pyrrolidone, carboxyvinyl polymer, sodium carboxymethylcellulose, sodium polyacrylate, sodium alginate, water-soluble dextran, sodium carboxymethyl starch, pectin, methylcellulose, ethylcellulose, xanthan gum, gum Arabic, casein, agar, polyethylene glycol, diglycerine, glycerine, propylene glycol, vaseline, paraffin, stearyl alcohol, stearic acid, human serum albumin (HSA), mannitol, sorbitol, lactose, and surfactants acceptable as pharmaceutical additives.

Actually employed additives are selected from among the above-mentioned additives depending upon dosage forms of the therapeutic agent of the invention, and such additives can be used alone or optionally in combination, although the additives, of course, are not limited to the above-mentioned additives. When the additive is used for an injection preparation, for example, the anti-HCV antibody prepared using the purified epitope-tagged HCV particles is dissolved in a solvent, such as physiological saline, buffer, or a glucose solution, followed by addition of an adsorption preventing agent, such as Tween 80, Tween 20, gelatin, or human serum albumin. Alternatively, the additive may be lyophilized to be prepared in a dosage form dissolved and reconstituted before use. Examples of excipients for lyophilization include sugar alcohols, such as mannitol or dextrose, and sugars.

Hereafter, the present invention will be described in more detail with reference to the following examples. It should be noted that these examples are provided for illustrative purposes and the technical scope of the present invention is not limited to these examples.

### Examples

### Example 1: Construction of J6/JFH-1 plasmid with FLAG-tag sequence inserted in HCV E2 HVR1 region

Used as a cDNA of HCV genomic RNA was the cDNA of a J6/JFH-1 chimera comprising a genotype 2a strain J6CF-derived sequence from 5'-UTR to N-terminal 16^{th} amino acid residue of NS2 (GenBank Accession No. AF177036, Yanagi, M. et al., Virology, 262, 1999, pp. 250-263) and a genotype 2a strain JFH-1-derived sequence from N-terminal 17^{th} amino acid residue of NS2 to 3' -UTR (GenBank Accession No. AB047639, Kato, T. et al., Gastroenterology, 125, 2003, pp. 1808-1817). As the FLAG-tag, cDNA comprising 1 or 3 repeats of the FLAG sequence was inserted. The resulting plasmids are referred to as pJ6/JFH-1 (1XFLAG) and pJ6/JFH-1 (3XFLAG), respectively.

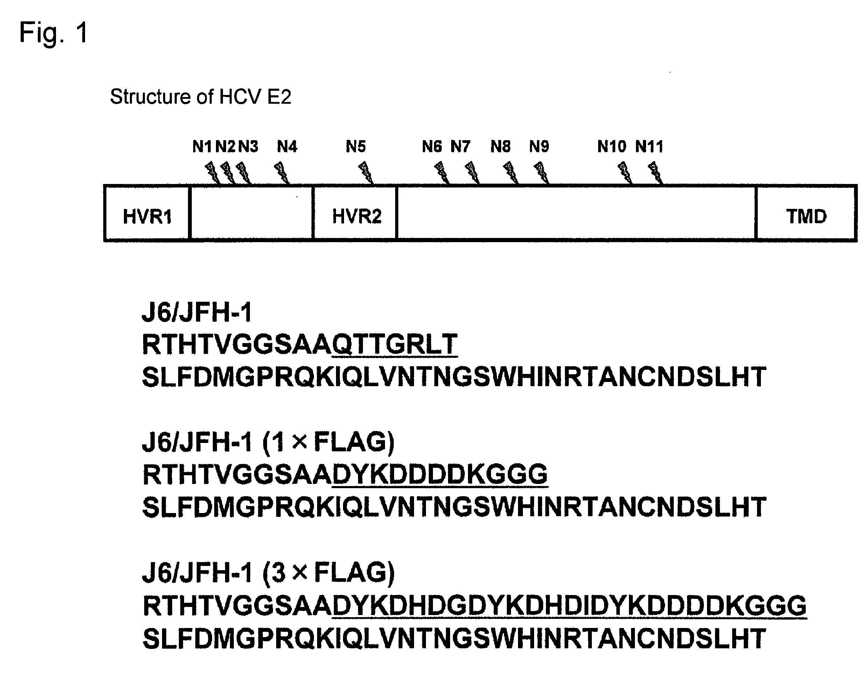

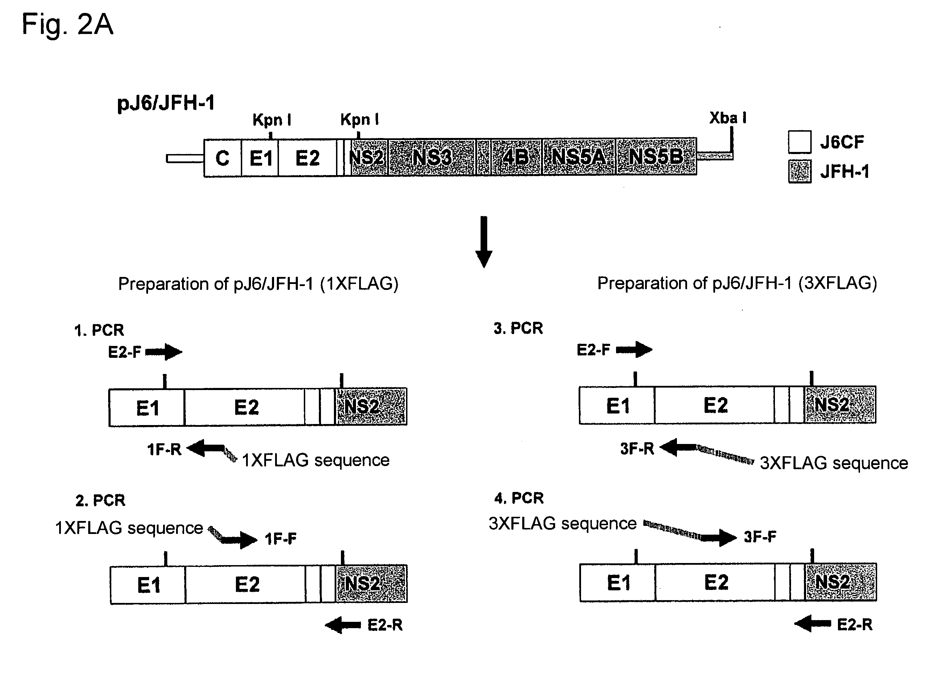

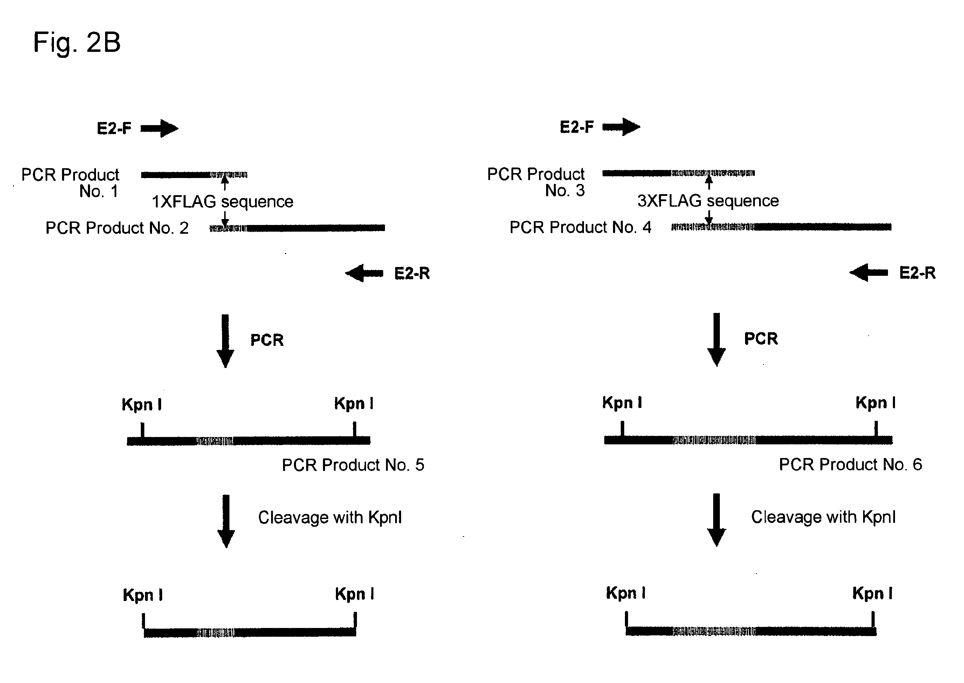

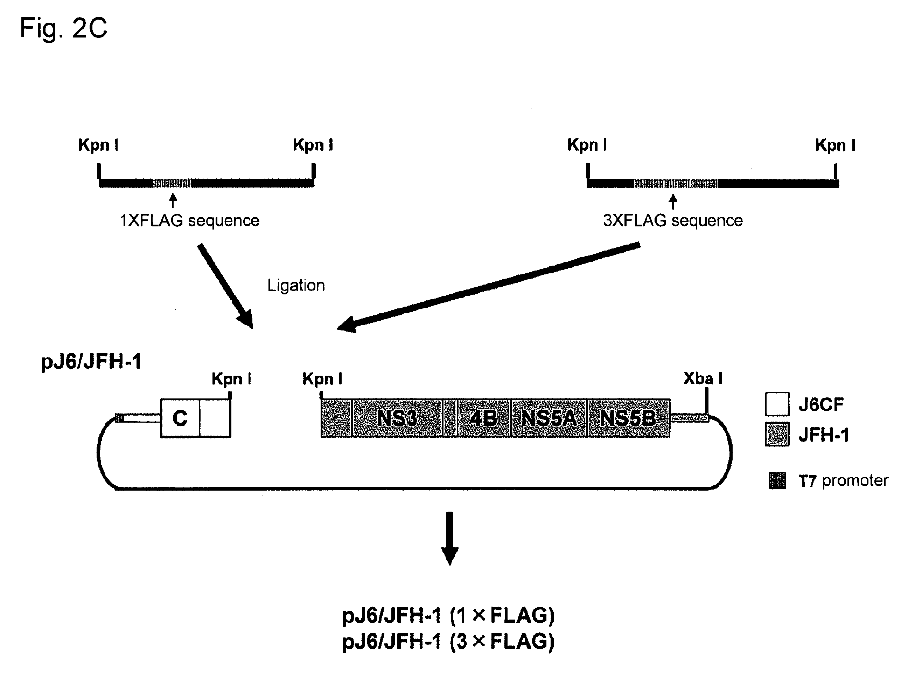

Fig. 1 shows the amino acid sequence which is in the vicinity of the FLAG tag-insertion site translated from those genes, starting from the N-terminal amino acid of the E2 protein. The N-terminal amino acid sequence of the E2 protein encoded by J6/JFH1 is shown in SEQ ID NO: 1, the N-terminal amino acid sequence of the E2 protein encoded by pJ6/JFH-1 (1XFLAG) is shown in SEQ ID NO: 2, and the N-terminal amino acid sequence of the E2 protein encoded by pJ6/JFH-1 (3XFLAG) is shown in SEQ ID NO:3. The methods for preparing plasmids are shown in Fig. 2 (A) to (C).

Specifically, cDNA corresponding to the entire JFH1 strain-derived genomic RNA region was cloned into pUC19 plasmid to construct a plasmid DNA, pJFH1 (Wakita, T. et al. Nat. Med., 11, 2005, pp. 791-796, WO 2004/104198), which was digested with EcoRI and then partially digested with BclI, and a plasmid DNA fragment from which a fragment (about 2,840 bp) from the EcoRI site to the first BclI site had been removed was purified. Separately, a J6CF strain-derived genomic cDNA which had been cloned into pUC19 plasmid, i.e. pJ6CF (GenBank Accession No. AF177036, Yanagi, M., et al., Virology 262: 250-263, 1999), was partially digested with EcoRI and BclI to obtain an about 2,840-bp fragment, which was then ligated to the above purified fragment to obtain J6/JFH1.

Subsequently, to the J6/JFH1 cDNA as a template were added 10 µl of 10× buffer, 4 µl of 2 mM dNTPs mixture, and 1 µl each of 10 µM primers E2-F (SEQ ID NO: 4) and 1F-R (SEQ ID NO: 5), which were from the Phusion High-Fidelity DNA Polymerase kit (FINNZYMES), then deionized water to bring the total amount to 49.5 µl. Thereafter, 0.5 µl of Phusion DNA Polymerase (FINNZYMES) was added, and PCR was then carried out for 30 cycles where one cycle consists of 98°C for 10 seconds, 55°C for 15 seconds, and 72°C for 30 seconds.. The resulting PCR product was designated as PCR product No. 1.

To the J6/JFH1 cDNA as a template were added 10 µl of 10× buffer, 4 µl of 2 mM dNTPs mixture, and 1 µl each of 10 µM primers E2-R (SEQ ID NO: 6) and 1F-F (SEQ ID NO: 7), which were from the Phusion High-Fidelity DNA Polymerase kit (FINNZYMES), then deionized water to bring the total amount to 49.5 µl. Thereafter, 0.5 µl of Phusion DNA Polymerase (FINNZYMES) was added, and PCR was then carried out for 30 cycles where one cycle consists of 98°C for 10 seconds, 55°C for 15 seconds, and 72°C for 1.5 minutes. The resulting PCR product was designated as PCR product No. 2.

To the J6/JFH1 cDNA a template were added 10 µl of 10× buffer, 4 µl of 2 mM dNTPs mixture, and 1 µl each of 10 µM primers E2-F (SEQ ID NO: 4) and 3F-R (SEQ ID NO: 8), which were from the Phusion High-Fidelity DNA Polymerase kit (FINNZYMES), then deionized water to bring the total amount to 49.5 µl. Thereafter, 0.5 µl of Phusion DNA Polymerase (FINNZYMES) was added, and PCR was then carried out for 30 cycles where one cycle consists of 98°C for 10 seconds, 55°C for 15 seconds, and 72°C for 30 seconds. The resulting PCR product was designated as PCR product No. 3.

To the J6/JFH1 cDNA as a template were added 10 µl of 10× buffer, 4 µl of 2 mM dNTPs mixture, and 1 µl each of 10 µM primers E2-R (SEQ ID NO: 6) and 3F-F (SEQ ID NO: 9), which were from the Phusion High-Fidelity DNA Polymerase kit (FINNZYMES), then deionized water to bring the total amount to 49.5 µl. Thereafter, 0.5 µl of Phusion DNA Polymerase (FINNZYMES) was added, and PCR was then carried out for 30 cycles where one cycle consists of 98°C for 10 seconds, 55°C for 15 seconds, and 72°C for 1.5 minutes.. The resulting PCR product was designated as PCR product No. 4.

Each PCR product was purified and dissolved in 15 µl of H₂O. DNA (1 µl) of the PCR product No. 1 was mixed with DNA (1 µl) of the PCR product No. 2. To the mixture, which was used as a template, were added 10 µl of 10× buffer, 4 µl of the 2 mM dNTPs mixture, and 1 µl each of 10 µM primers E2-F (SEQ ID NO: 4) and E2-R (SEQ ID NO: 6), which were from the Phusion High-Fidelity DNA Polymerase kit (FINNZYMES), then deionized water to bring the total amount to 49.5 µl. Thereafter, 0.5 µl of Phusion DNA Polymerase (FINNZYMES) was added, and PCR was then carried out for 30 cycles where one cycle consists of 98°C for 10 seconds, 55°C for 15 seconds, and 72°C for 2 minutes. The resulting PCR product was designated as PCR product No. 5.

DNA (1 µl) of the PCR product No. 3 was mixed with DNA (1 µl) of the PCR product No. 4. To the mixture, which was used as a template, were added 10 µl of 10× buffer, 4 µl of 2 mM dNTPs mixture, and 1 µl each of 10 µM primers E2-F (SEQ ID NO: 4) and E2-R (SEQ ID NO: 6), which were from the Phusion High-Fidelity DNA Polymerase kit (FINNZYMES), then deionized water to bring the total amount to 49.5 µl. Thereafter, 0.5 µl of Phusion DNA Polymerase (FINNZYMES) was added, and PCR was then carried out for 30 cycles where one cycle consists of 98°C for 10 seconds, 55°C for 15 seconds, and 72°C for 2 minutes. The resulting PCR product was designated as PCR product No. 6. The PCR products were purified and dissolved in 30 µl of H₂O.

The J6/JFH-1 cDNA and the purified PCR product No. 5 were separately digested with the KpnI restriction enzyme, and each HCV cDNA fragment was fractionated by agarose gel electrophoresis, followed by purification. The two DNA fragments were mixed with Ligation Mix (TAKARA BIO INC, Japan) to ligate the DNA fragments. The vector was designated as pJ6/JFH-1 (1XFLAG). The pJ6/JFH-1 (1XFLAG) comprises a nucleotide sequence that encodes the chimera gene, which comprises: in order from 5'-side to 3'-side, the 5'-untranslated region, the Core protein-encoding sequence, the E1 protein-coding sequence, the E2 protein-coding sequence, the p7 protein-coding sequence, and a sequence encoding the N-terminal 16 amino acids of the NS2 protein region, of the J6CF strain; and a sequence encoding a region from the N-terminal 17^{th} amino acid to the C-terminus of the NS2 protein region, the NS3 protein-coding sequence, the NS4A protein-coding sequence, the NS4B protein-coding sequence, the NS5A protein-coding sequence, the NS5B protein region, and the 3'-untranslated region, of the JFH-1 strain, wherein it comprises, in the E2 HVR1 region, a nucleotide sequence that encodes the FLAG peptide sequence.

The J6/JFH-1 cDNA and the purified PCR product No. 6 were digested with the KpnI restriction enzyme, and each HCV cDNA fragment was fractionated by agarose gel electrophoresis, followed by purification. The two DNA fragments were mixed with Ligation Mix (TAKARA BIO INC, Japan) to ligate the two DNA fragments. The obtained vector was designated as pJ6/JFH-1 (3XFLAG). The pJ6/JFH-1 (3XFLAG) comprises a nucleotide sequence that encodes the chimera gene, which comprises: in order from 5'-side to 3'-side, the 5'-untranslated region, the Core protein-coding sequence, the E1 protein-coding sequence, the E2 protein-coding sequence, the p7 protein-coding sequence, and a sequence encoding the N-terminal 16 amino acids of the NS2 protein region, of the J6CF strain; and a sequence encoding a region from the N-terminal amino acid 17 to the C-terminus of the NS2 protein region, the NS3 protein-coding sequence, the NS4A protein-coding sequence, the NS4B protein-coding sequence, the NS5A protein-coding sequence, the NS5B protein-coding region, and the 3'-untranslated region, of the JFH-1 strain, wherein it comprises, in the E2 HVR1 region, a nucleotide sequence that comprises 3 repeats of the FLAG peptide sequence.

The nucleotide sequences of pJ6/JFH-1, pJ6/JFH-1 (1XFLAG), and pJ6/JFH-1 (3XFLAG) are shown in SEQ ID NO: 10, SEQ ID NO: 11, and SEQ ID NO: 12, respectively, in the Sequence Listing.

### Example 2: In vitro RNA synthesis and introduction of RNA into cells

The pJ6/JFH-1, pJ6/JFH-1 (IXFLAG), and pJ6/JFH-1 (3XFLAG) were separately cleaved with XbaI and subjected to phenol/chloroform extraction then to ethanol precipitation. The cleaved plasmids each was used as a template to synthesize a HCV RNA using the MEGAscript T7 kit (Ambion).

The Huh7 cells (3 × 10⁶ cells) and 5 µg of the HCV RNA were suspended in 400 µl of the Cytomix solution (120 mM KCl, 0.15 mM CaCl₂, 10 mM K₂HPO₄/KH₂PO₄, 25 mM Hepes, 2 mM EGTA, 5 mM MgCl₂, 20 mM ATP, and 50 mM glutathione), transferred into a 4-mm cuvette, and subjected to electroporation using the Gene Pulser (BioRad) at 260 V and 950 µF. Thereafter, the cells into which the HCV RNA had been introduced were seeded on a 10 cm² dish and then subcultured.

### Example 3: Production of HCV particles using J6/JFH-1 (3XFLAG) RNA introduced cells

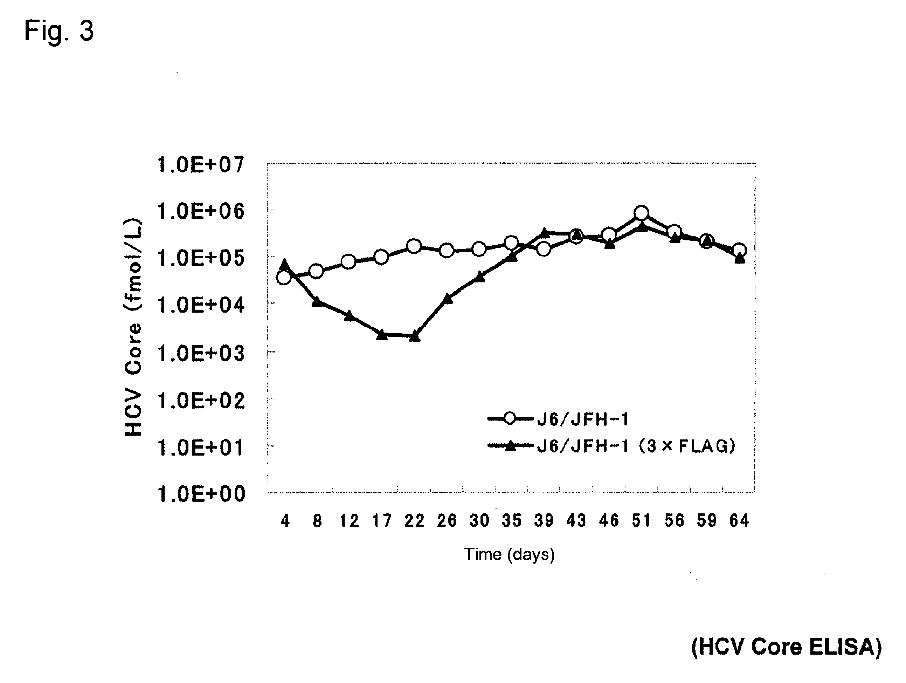

When the cells into which the J6/JFH-1 or J6/JFH-1 (3XFLAG) RNA had been introduced were subcultured, each HCV Core protein contained in the culture supernatant was quantified using the HCV antigen ELISA test kit (Ortho) to confirm the production of HCV particles. As a result, the amount of production of the HCV particles was found to remain unchanged at substantially constant levels when the J6/JFH-1 RNA was introduced. When the J6/JFH-1 (3XFLAG) RNA was introduced, however, the amount of HCV Core protein in the culture supernatant decreased with time until day 21 after the introduction, and then began to increase on day 22 after the introduction and reached an almost same level as that of J6/JFH-1 on day 35 after the introduction (Fig. 3). This suggested that, although the J6/JFH-1 (3XFLAG) RNA did not have a high capacity for virus production when the Huh7 cells were introduced, it had a capacity for virus production similar to that of J6/JFH-1 RNA after an adaptive mutation, which is necessary for virus production, was introduced into the virus genome.

### Example 4: Expression of HCV protein in J6/JFH-1 (3XFLAG) RNA introduced cells

Expression of HCV proteins in the cells was confirmed by Western blotting on days 4, 17, 30 and 43 after the introduction of J6/JFH-1 or J6/JFH-1 (3XFLAG) RNA. In this analysis, a cell extract sample obtained from the Huh7 cells with no introduced RNA was used as a negative control. Samples extracted from the cells were subjected to SDS-PAGE and blotted onto a PVDF membrane (Immobilon-P, Millipore). Thereafter, anti-Core monoclonal antibody, anti-FLAG monoclonal antibody (M2 antibody, Sigma), anti-E2 monoclonal antibody, and HRP-labeled secondary antibody that recognizes these antibodies were used to detect the Core proteins and the E2 proteins translated in the cells using the ECL Plus (GE Healthcare).

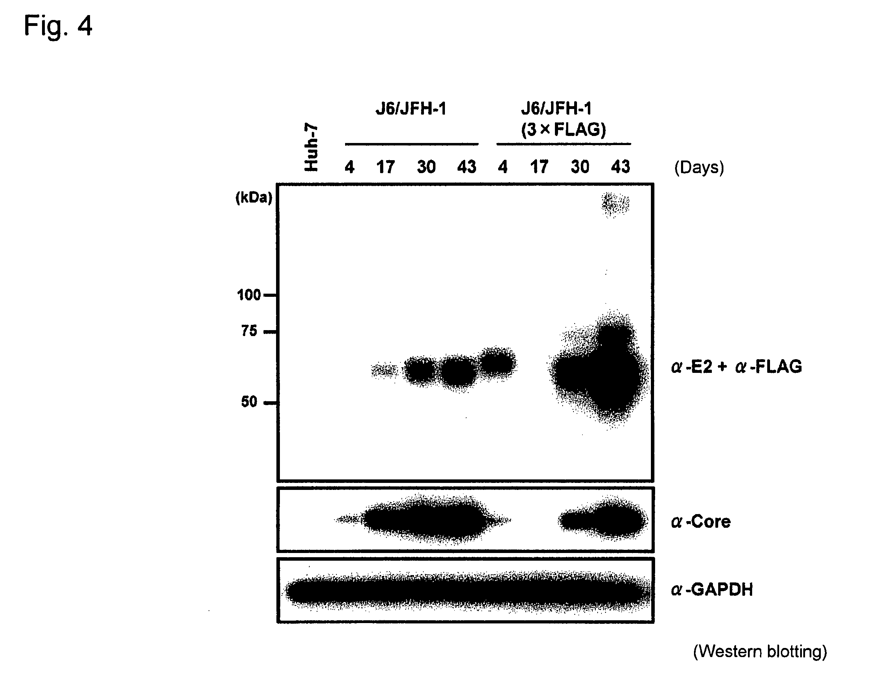

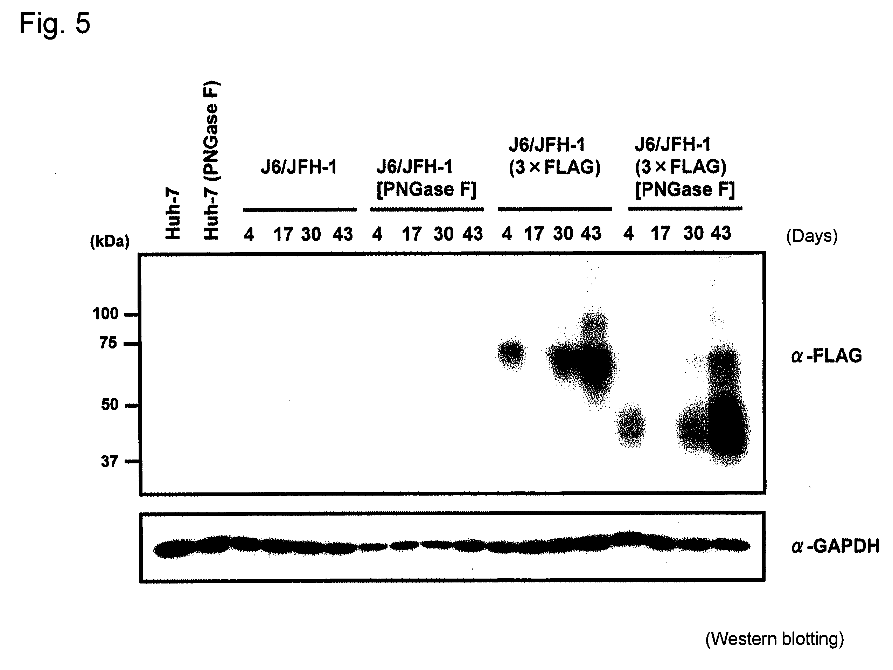

As a result, the molecular mass of the E2 protein 30 and 43 days after the introduction of J6/JFH-1 (3XFLAG) was found to be smaller than that of the E2 protein just when RNA was introduced (Fig. 4). Such change was not observed in the Core proteins. So, these E2 proteins were treated with the deglycosylation enzyme PNGase F, and as a result, such difference in molecular mass was no longer observed (Fig. 5). This suggested that the E2 region of the J6/JFH-1 (3XFLAG) RNA genome might cause a mutation in the genome by repeating subculture of cells and that the site of mutation might be a sugar chain-binding site.

### Example 5: Analysis of HCV genome sequence in cells infected with J6/JFH-1 (3XFLAG) virus repeatedly subcultured

To examine an adaptive mutation, which is necessary for the J6/JFH-1 (3 XFLAG) virus to have high infectivity, total RNA was extracted from the infecte d cells 8 days and 39 days after RNA introduction, and the sequence of the HC V genome contained therein was analyzed.

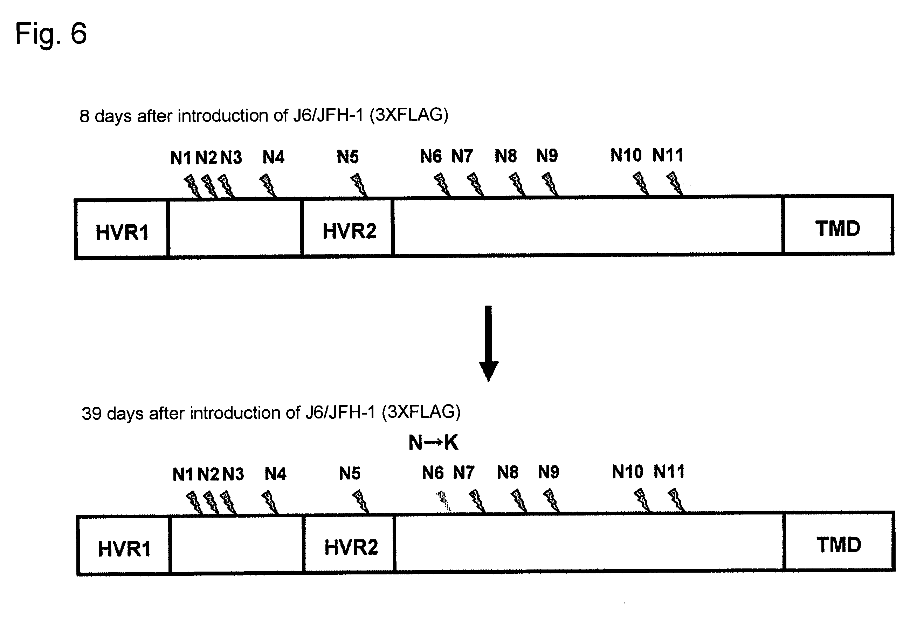

As a result, it was found that asparagine AAU (N), which is a sugar-chain addition site of the E2 region, was substituted with lysine AAA (K), and that, when the sugar chain modification at this site was removed, the molecular mass became smaller than that of the E2 protein at the time of introduction of RNA (Fig. 6). This asparagine corresponds to the 534^{th} amino acid when that the methionine is counted as a first amino acid,the methionine being at the N-terminus in the Core protein of the strain J6CF (GenBank Accession No. AF177036, Yanagi, M. et al., Virology, 262, 1999, pp. 250-263).

### Example 6: Purification of J6/JFH-1 (3XFLAG) virus particles

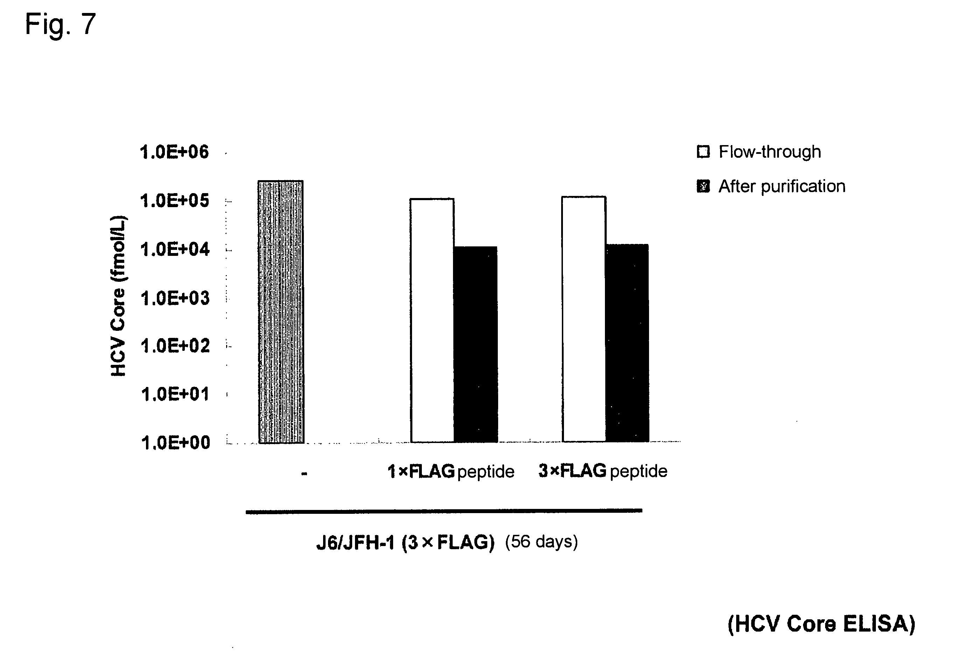

In order to purify J6/JFH-1 (3XFLAG) virus particles, the culture supernatant 56 days after the introduction of J6/JFH-1 (3XFLAG) RNA was used. To 600 µl of this culture supernatant in a container, 500 µl of M2 antibody agarose (Sigma) that had been pretreated with 0.1 M glycine-HCl (pH 3.5) was added, and for mixing, the top and bottom of the container were repeatedly inverted at 4°C for 2 hours. Flow-through fraction was recovered, while the M2 antibody agarose was washed three times with TBS buffer, and elution was then carried out three times using 200 µl of a 1XFLAG peptide (Sigma) solution or 3XFLAG peptide (Sigma) solution. The amounts of the HCV Core protein in the elution samples were quantified using the HCV antigen ELISA test kit (Ortho). As a result, it was found that elution could be carried out with either of the FLAG peptides, and that purification efficiency was about 5% (Fig. 7).

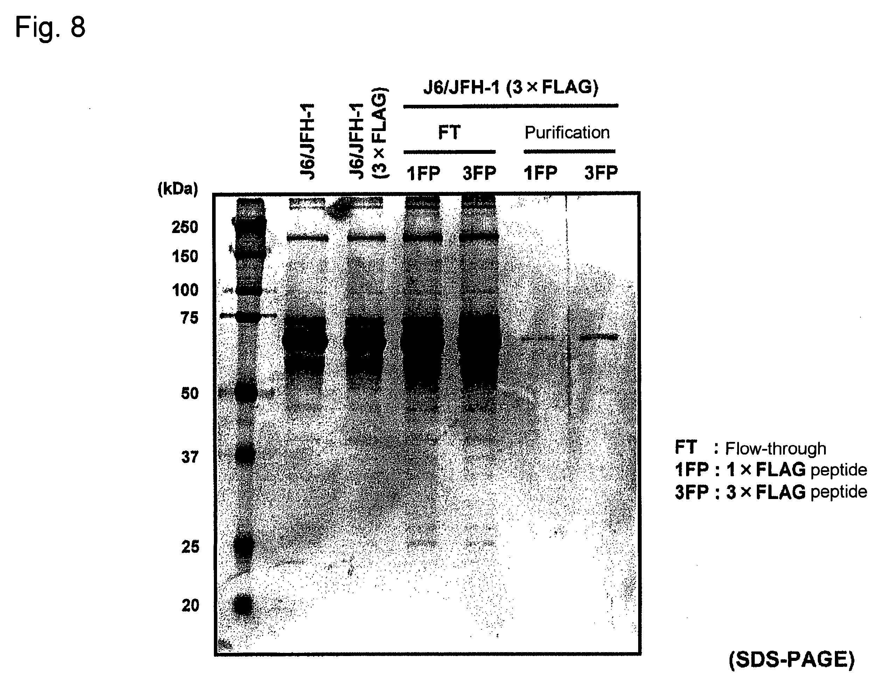

To examine the purity of the elution samples, the elution samples equivalent to 0.1 fmol HCV Core and the unpurified culture supernatant were subjected to SDS-PAGE. Thereafter, silver staining was carried out to confirm the presence of contaminant proteins contained in each solution; however, almost no contaminant proteins were observed in the elution samples (Fig. 8). Thus, it was confirmed that the HCV particles in the elution samples were highly purified.

### Example 7: Evaluation of infectivity of purified J6/JFH-1 (3XFLAG) virus

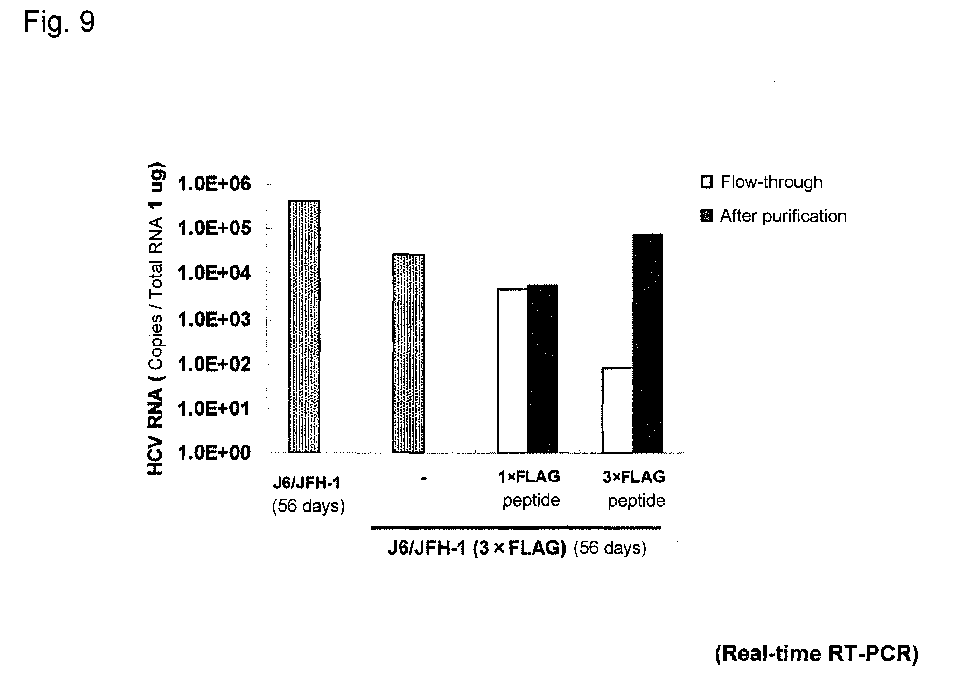

In order to evaluate the infectivity of the J6/JFH-1 (3XFLAG) virus purified in Example 6, infection experiment was carried out using cultured cells. The Huh7 cells were seeded on a 8-well glass chamber at 1 × 10⁴ cells/well, cultured for 24 hours, and then infected with the virus solution equivalent to 1 fmol HCV Core for 3 hours. After infection, the cells were washed twice with DMEM (Sigma) and cultured for further 4 days. Total RNA was extracted from the cells 4 days later in accordance with the manufacturer's instructions appended to the Trizol Reagent (Invitrogen). The amount of HCV RNA in total RNA was assayed via quantitative RT-PCR. The sense primer S-17 (SEQ ID NO: 13), antisense primer R-19 (SEQ ID NO: 14), and TaqMan probe (SEQ ID NO: 15), which target the 5'-UTR of HCV RNA, were used. The primers, the probe, and extracted total RNA were mixed together to prepare a RT-PCR reaction solution in accordance with the manufacturer's instructions appended to the TaqMan EZ RT-PCR Core Reagents (Applied Biosystems), followed by detection using the 7500 Fast Real-Time PCR System (Applied Biosystems). As a result, the purified J6/JFH-1 (3XFLAG) virus solution was found to have infectivity on the Huh7 cells (Fig. 9). This suggests that the viruses with inserted FLAG-tag sequence in the HVR1 region of HCV E2 can be highly purified and that such purified viruses have infectivity.

### Example 8: Construction of J6/JFH-1 (1XFLAG) E2 N→K and J6/JFH-1 (3XFLAG) E2 N→K mutant plasmids

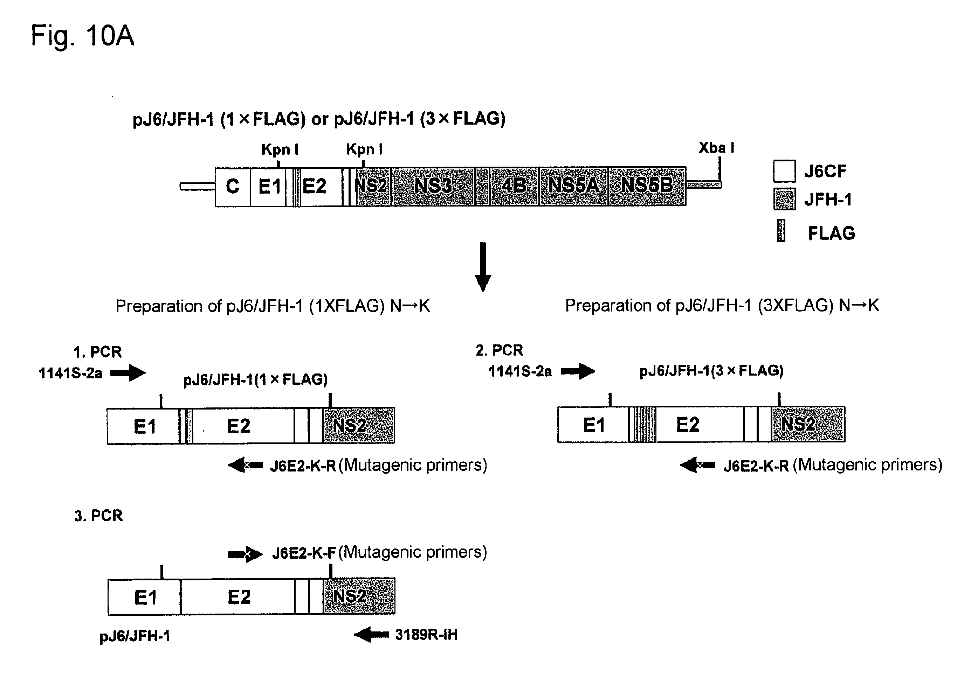

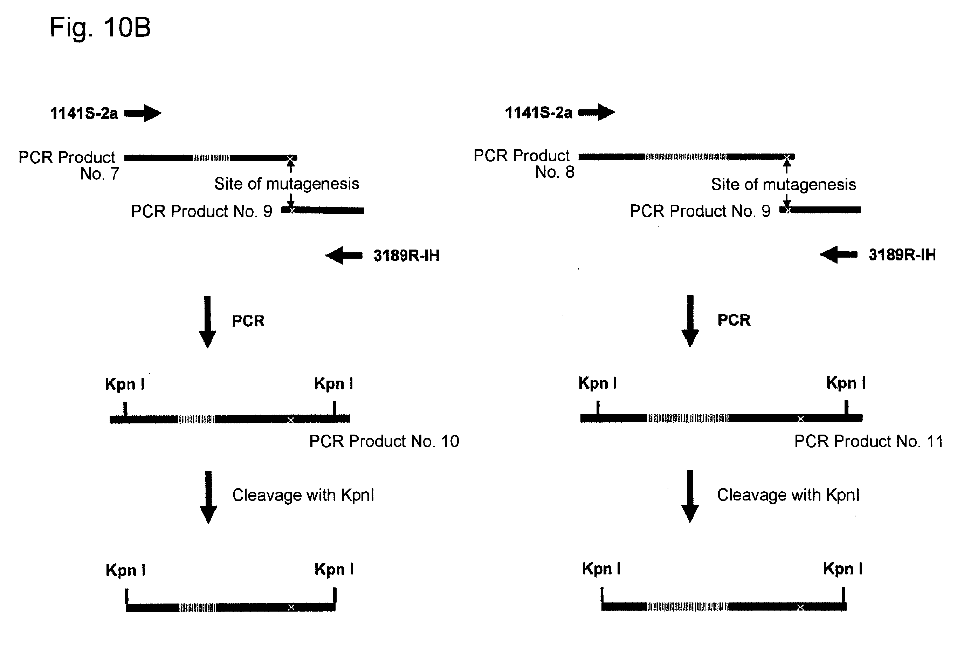

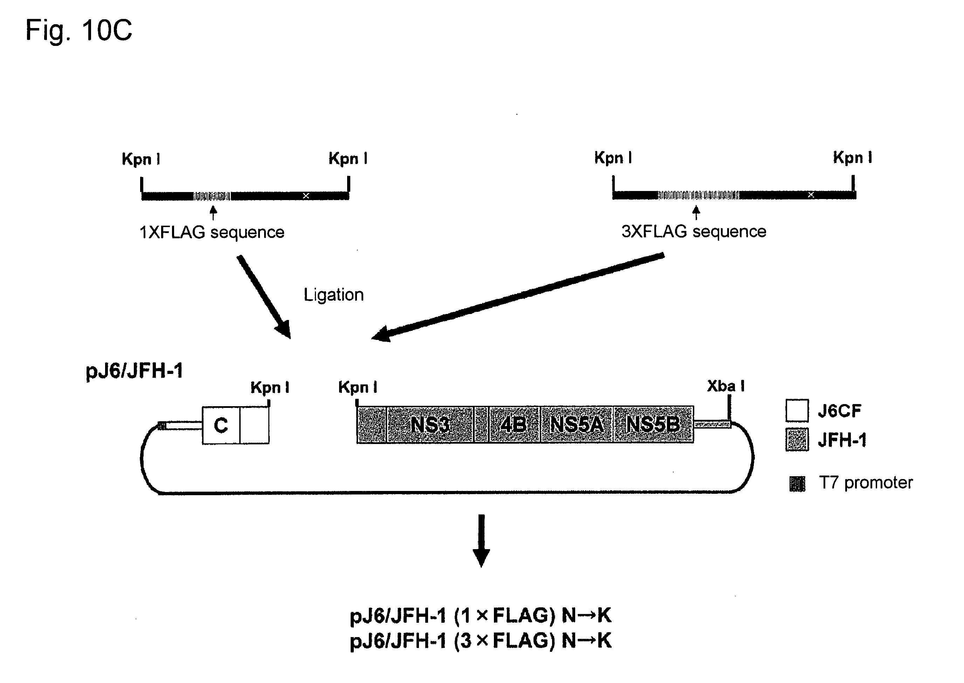

The pJ6/JFH-1 (1XFLAG) N→K and the pJ6/JFH-1 (3XFLAG) N→K plasmids described in Example 5 that have an adaptive mutation necessary for the J6/JFH-1 (3XFLAG) virus to be highly infectious (i.e., a substitution of asparagine AAT (N6) with lysine AAA (K) at the sugar chain addition site of the E2 region) were constructed. The methods for preparing such plasmids are shown in Fig. 10 (A) to (C).

Specifically, to the pJ6/JFH-1 (1XFLAG) as a template were added 10 µl of 10× buffer, 4 µl of 2 mM dNTPs mixture, and 1 µl each of 10 µM primers 1141 S-2a (SEQ ID NO: 16) and J6E2-K-R (SEQ ID NO: 17), which were from the Phusion High-Fidelity DNA Polymerase kit (FINNZYMES), then deionized water to bring the total amount to 49.5 µl. Thereafter, 0.5 µl of Phusion DNA Polymerase (FINNZYMES) was added, and PCR was then carried out for 30 cycles wherein one cycle consists of 98°C for 10 seconds, 55°C for 15 seconds, and 72°C for 1.5 minutes. The resulting PCR product was designated as PCR product No. 7.

Subsequently, to the pJ6/JFH1 (3XFLAG) as a template were added 10 µl of 10× buffer, 4 µl of 2 mM dNTPs mixture, and 1 µl each of 10 µM primers 1141 S-2a (SEQ ID NO: 16) and J6E2-K-R (SEQ ID NO: 17), which were from the Phusion High-Fidelity DNA Polymerase kit (FINNZYMES), then deionized water to bring the total amount to 49.5 µl. Thereafter, 0.5 µl of Phusion DNA Polymerase (FINNZYMES) was added, and PCR was then carried out for 30 cycles wherein one cycle consists of 98°C for 10 seconds, 55°C for 15 seconds, and 72°C for 1.5 minutes. The resulting PCR product was designated as PCR product No. 8.

To the pJ6/JFH-1 as a template were added 10 µl of 10× buffer, 4 µl of 2 mM dNTPs mixture, and 1 µl each of 10 µM primers J6E2-K-F (SEQ ID NO: 18) and 3189R-IH (SEQ ID NO: 19), which were from the Phusion High-Fidelity DNA Polymerase kit (FINNZYMES), then deionized water to bring the total amount to 49.5 µl. Thereafter, 0.5 µl of Phusion DNA Polymerase (FINNZYMES) was added, and PCR was then carried out for 30 cycles wherein one cycle consists of 98°C for 10 seconds, 55°C for 15 seconds, and 72°C for 1.5 minutes. The resulting PCR product was designated as PCR product No. 9.

Each PCR product was purified and dissolved in 50 µl of H₂O. DNA (1 µl) of the PCR product No. 7 was mixed with DNA (1 µl) of the PCR product No. 9. To the mixture, which was used as a template, were added 10 µl of 10× buffer, 4 µl of the 2 mM dNTPs mixture, and 1 µl each of 10 µM primers 1141S-2a (SEQ ID NO: 16) and 3189R-IH (SEQ ID NO: 19), which were from the Phusion High-Fidelity DNA Polymerase kit (FINNZYMES), then deionized water to bring the total amount to 49.5 µl. Thereafter, 0.5 µl of Phusion DNA Polymerase (FINNZYMES) was added, and PCR was then carried out for 30 cycles wherein one cycle consists of 98°C for 10 seconds, 55°C for 15 seconds, and 72°C for 2 minutes. The resulting PCR product was designated as PCR product No. 10.

Subsequently, DNA (1 µl) of the PCR product No. 8 was mixed with DNA (1 µl) of the PCR product No. 9. To the mixture, which was used as a template, were added 10 µl of 10× buffer, 4 µl of 2 mM dNTPs mixture, and 1 µl each of 10 µM primers 1141S-2a (SEQ ID NO: 16) and 3189R-IH (SEQ ID NO: 19), which were from the Phusion High-Fidelity DNA Polymerase kit (FINNZYMES), then deionized water to bring the total amount to 49.5 µl. Thereafter, 0.5 µl of Phusion DNA Polymerase (FINNZYMES) was added, and PCR was then carried out for 30 cycles wherein one cycle consists of 98°C for 10 seconds, 55°C for 15 seconds, and 72°C for 2 minutes. The resulting PCR product was designated as PCR product No. 11. Each PCR product was purified and dissolved in 30 µl of H₂O.

The J6/JFH-1 and the purified PCR product No. 10 were separately digested with the KpnI restriction enzyme, and the HCV cDNA fragments were fractionated by agarose gel electrophoresis, followed by purification. The two DNA fragments were mixed with Ligation Mix (TAKARA BIO INC, Japan) to ligate the two DNA fragments. The vector was designated as pJ6/JFH-1 (1XFLAG) N→K. The pJ6/JFH-1 (1XFLAG) N→K comprises a nucleotide sequence that encodes the chimera gene which comprises: in order from 5'-side to 3'-side, the 5'-untranslated region, the Core protein-coding sequence, the E1 protein-coding sequence, the E2 protein-coding sequence, the p7 protein-coding sequence, and a sequence encoding the N-terminal 16 amino acids of the NS2 protein region, of the J6CF strain; and a sequence encoding a region from the N-terminal 17^{th} amino acid to the C-terminus of the NS2 protein region, the NS3 protein-coding sequence, the NS4A protein-coding sequence, the NS4B protein-coding sequence, the NS5A protein-coding sequence, the NS5B protein-coding region, and the 3'-untranslated region, of the JFH-1 strain, wherein the pJ6/JFH-1 (IXFLAG) N→K comprises, in the E2 HVR1 region, a nucleotide sequence encoding the FLAG peptide sequence and a nucleotide sequence comprising lysine at position 534 when the methionine is counted as a first aminoacid, the methione being at the N-terminus in the Core protein.

The J6/JFH1 and the purified PCR product No. 11 were separately digested with the KpnI restriction enzyme, and each HCV cDNA fragment was fractionated by agarose gel electrophoresis, followed by purification. The two DNA fragments were mixed with Ligation Mix (TAKARA BIO INC, Japan) to ligate the two DNA fragments. The vector was designated as pJ6/JFH-1 (3XFLAG) N→K. The pJ6/JFH-1 (3XFLAG) N→K comprises a nucleotide sequence that encodes the chimera gene which comprises: in order from 5'-side to 3'-side, the 5'-untranslated region, the Core protein-coding sequence, the E1 protein-coding sequence, the E2 protein-coding sequence, the p7 protein-coding sequence, and a sequence encoding the N-terminal 16 amino acids of the NS2 protein region, of the J6CF strain; and a sequence encoding a region from the N-terminal 17^{th} amino acid to the C-terminus of the NS2 protein region, the NS3 protein-coding sequence, the NS4A protein-coding sequence, the NS4B protein-coding sequence, the NS5A protein-coding sequence, the NS5B protein-coding region, and the 3'-untranslated region, of the JFH-1 strain, wherein the pJ6/JFH-1 (3XFLAG) N→K comprises, in the E2 HVR1 region, a nucleotide sequence encoding 3 repeats of the FLAG peptide sequence and a nucleotide sequence comprising lysine at position 534 when the methionine is counted as a first amino acid, the methionine being at the N-terminus in the Core protein.

The nucleotide sequences of pJ6/JFH-1 (1XFLAG) N→K and pJ6/JFH-1 (3XFLAG) N→K are shown in SEQ ID NO: 20 and SEQ ID NO: 21, respectively, in the Sequence Listing.

### Example 9: Preparation of J6/JFH-1 (1XFLAG) E2 N→K and J6/JFH-1 (3XFLA G) E2 N→K viruses

The plasmids prepared in Example 8 were cleaved with XbaI and subjected to phenol/chloroform extraction then to ethanol precipitation. The cleaved plasmids each was used as a template to synthesize HCV RNA using the MEGAscript T7 kit (Ambion).

The Huh7 cells (3 × 10⁶ cells) and 5 µg of the HCV RNA were suspended in 400 µl of the Cytomix solution (120 mM KCl, 0.15 mM CaCl₂, 10 mM K₂HPO₄/KH₂PO₄, 25 mM Hepes, 2 mM EGTA, 5 mM MgCl₂, 20 mM ATP, and 50 mM glutathione), and transferred into a 4-mm cuvette, and then electroporation was carried out using the Gene Pulser (BioRad) at 260 V and 950 µF. Thereafter, the cells into which the HCV RNA had been introduced were seeded on a 10 cm² dish and then subcultured.

When the cells into which the J6/JFH-1 (1XFLAG) E2 N→K or J6/JFH-1 (3XFLAG) E2 N→K RNA had been introduced were subcultured, each HCV Core protein contained in the culture supernatant was quantified using the HCV antigen ELISA test kit (Ortho), thereby confirming the production of HCV particles. Over the period from the initial stage to the later stage of culture, the amount of HCV Core protein contained in the culture supernatant of cells into which RNA without mutation had been introduced was compared with that contained in the culture supernatant of cells into which RNA with mutation had been introduced. As a result, the latter amount was found to be higher.

### Example 10: Construction of JFH-1 plasmid with FLAG-tag sequence inserted into HCV E2 HVR1 region

In the same manner as in Example 1, an epitope tag sequence was introduced into HVR1 of the genotype 2a strain JFH-1 (GenBank Accession No. AB047639, Kato, T. et al., Gastroenterology, 125, 2003, pp. 1808-1817). The cDNA comprising 1 or 3 repeats of the FLAG sequence was inserted as the epitope. The resulting plasmids are referred to as JFH-1 (1XFLAG) and JFH-1 (3XFLAG), respectively.

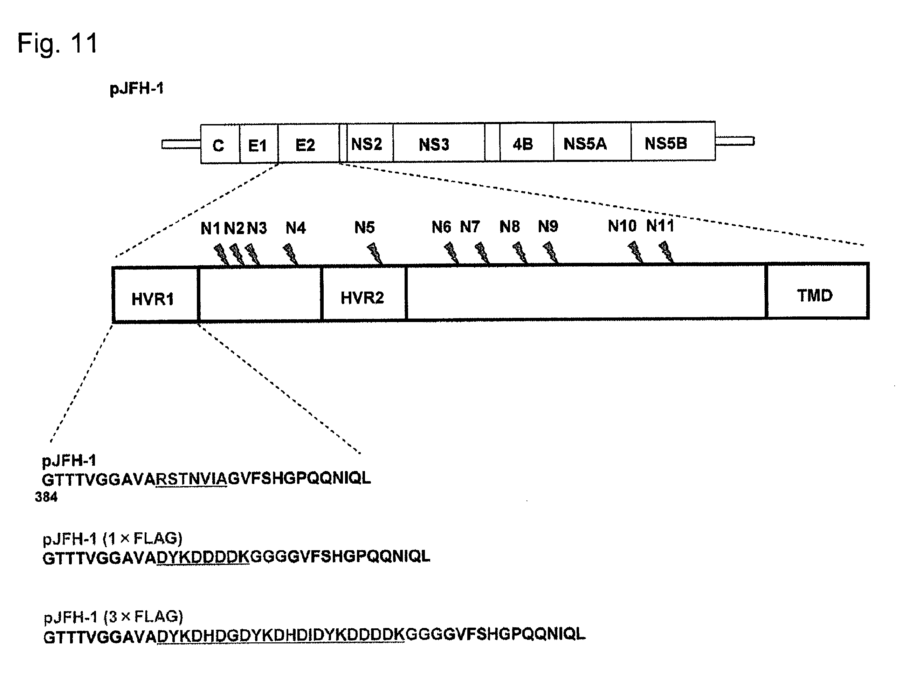

Fig. 11 shows the amino acid sequences, which are in the vicinity of the FLAG-tag insertion sites translated from said genes, starting from the N-terminal amino acid of the E2 protein.

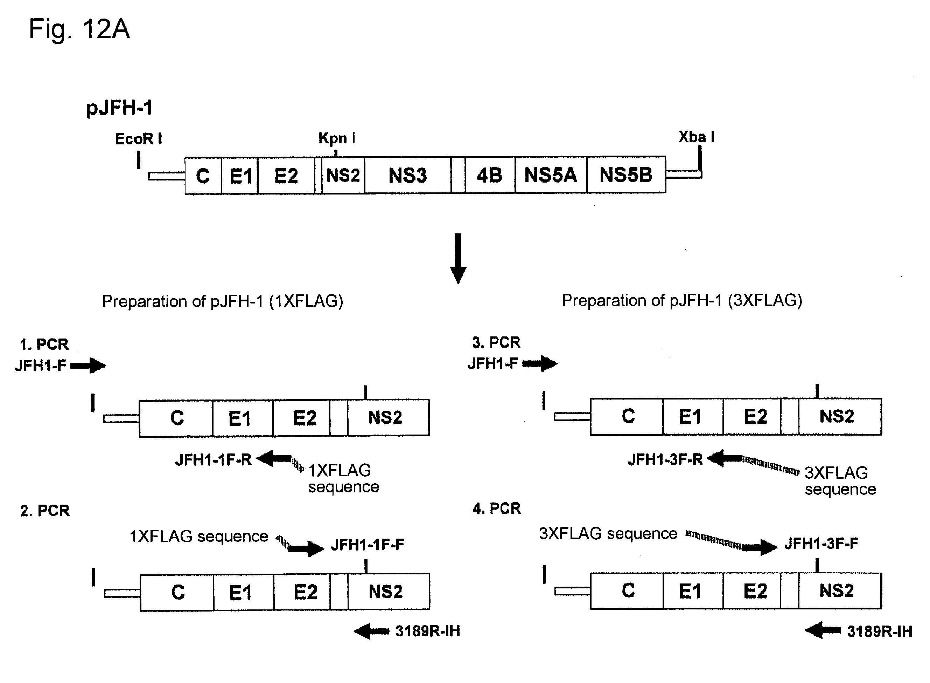

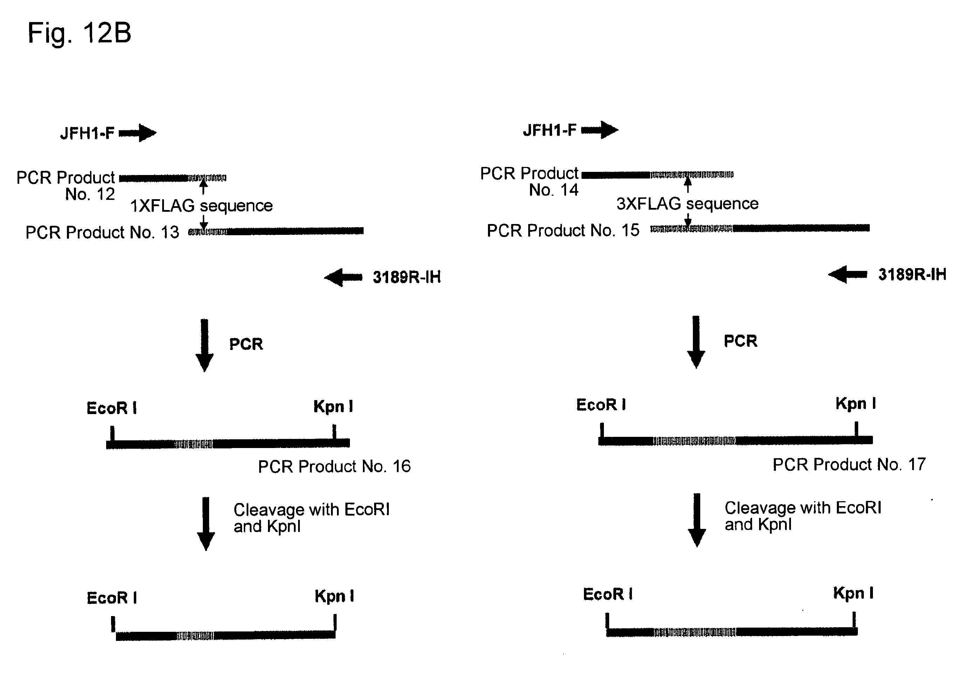

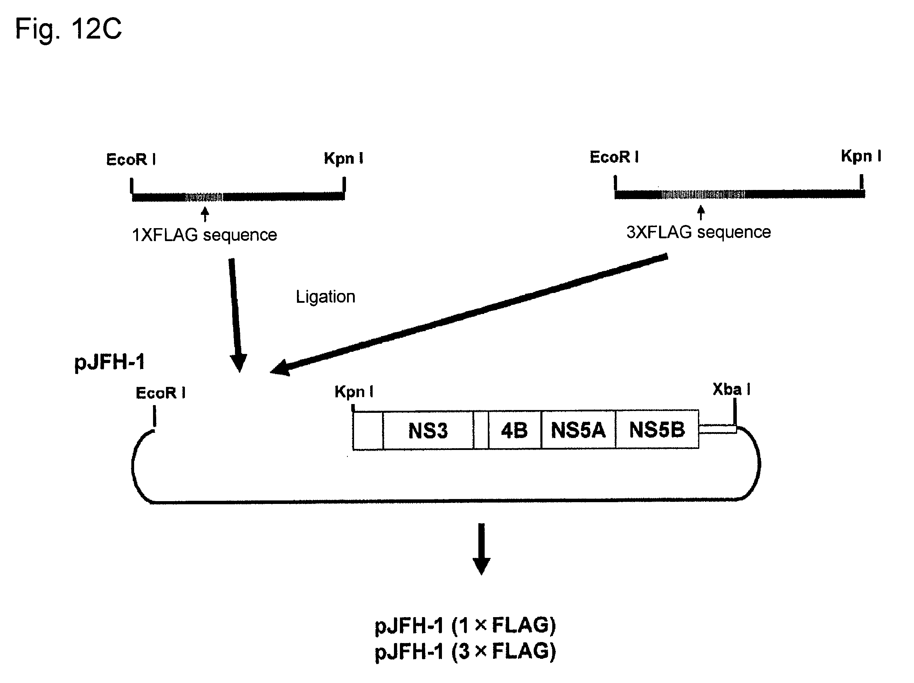

The N-terminal sequence of the E2 protein encoded by pJFH-1 (also referred to as "pJFH1"), the N-terminal sequence of the E2 protein encoded by pJFH-1 (1XFLAG), and the N-terminal sequence of the E2 protein encoded by pJFH-1 (3XFLAG) are shown in SEQ ID NO: 22, SEQ ID NO: 23, and SEQ ID NO: 24, respectively. The methods for preparing these plasmids are shown in Fig. 12 (A) to (C).

Specifically, to the pJFH-1 cDNA as a template were added 10 µl of 10× buffer, 4 µl of 2 mM dNTPs mixture, and 1 µl each of 10 µM primers JFH1-F (SEQ ID NO: 25) and JFH1-1F-R (SEQ ID NO: 26), which were from the Phusion High-Fidelity DNA Polymerase kit (FINNZYMES), then deionized water to bring the total amount to 49.5 µl. Thereafter, 0.5 µl of Phusion DNA Polymerase (FINNZYMES) was added, and PCR was then carried out for 30 cycles, wherein one cycle consists of 98°C for 10 seconds, 55°C for 15 seconds, and 72°C for 30 seconds. The resulting PCR product was designated as PCR product No. 12.

Subsequently, to the pJFH-1 cDNA as a template were added 10 µl of 10× buffer, 4 µl of 2 mM dNTPs mixture, and 1 µl each of 10 µM primers 3189R-IH (SEQ ID NO: 19) and JFH1-1F-F (SEQ ID NO: 27), which were from the Phusion High-Fidelity DNA Polymerase kit (FINNZYMES), then deionized water to bring the total amount to 49.5 µl. Thereafter, 0.5 µl of Phusion DNA Polymerase (FINNZYMES) was added, and PCR was then carried out for 30 cycles wherein one cycle consists of 98°C for 10 seconds, 55°C for 15 seconds, and 72°C for 1 minute and 30 seconds. The resulting PCR product was designated as PCR product No. 13.

To the JFH-1 cDNA as a template were added 10 µl of 10× buffer, 4 µl of 2 mM dNTPs mixture, and 1 µl each of 10 µM primers JFH1-F (SEQ ID NO: 25) and JFH-1-3F-R (SEQ ID NO: 28), which were from the Phusion High-Fidelity DNA Polymerase kit (FINNZYMES), then deionized water to bring the total amount to 49.5 µl. Thereafter, 0.5 µl of Phusion DNA Polymerase (FINNZYMES) was added, and PCR was then carried out for 30 cycles wherein one cycle consists of 98°C for 10 seconds, 55°C for 15 seconds, and 72°C for 30 seconds. The resulting PCR product was designated as PCR product No. 14.

To the pJFH-1 cDNA as a template were added 10 µl of 10× buffer, 4 µl of 2 mM dNTPs mixture, and 1 µl each of 10 µM primers 3189R-IH (SEQ ID NO: 19) and JFH1-3F-F (SEQ ID NO: 29), which were from the Phusion High-Fidelity DNA Polymerase kit (FINNZYMES), then deionized water to bring the total amount to 49.5 µl. Thereafter, 0.5 µl of Phusion DNA Polymerase (FINNZYMES) was added, and PCR was then carried out for 30 cycles wherein one cycle consists of 98°C for 10 seconds, 55°C for 15 seconds, and 72°C for 1.5 minutes. The resulting PCR product was designated as PCR product No. 15.

The PCR products were purified and dissolved in 15 µl of H₂O. DNA (1 µl) of the PCR product No. 12 was mixed with DNA (1 µl) of the PCR product No. 13. To the mixture, which was used as a template, were added 10 µl of 10× buffer, 4 µl of the 2 mM dNTPs mixture, and 1 µl each of 10 µM primers JFH1-F (SEQ ID NO: 25) and 3189R-IH (SEQ ID NO: 19), which were from the Phusion High-Fidelity DNA Polymerase kit (FINNZYMES), then deionized water to bring the total amount to 49.5 µl Thereafter, 0.5 µl of Phusion DNA Polymerase (FINNZYMES) was added, and PCR was then carried out for 30 cycles wherein one cycle consists of 98°C for 10 seconds, 55°C for 15 seconds, and 72°C for 2 minutes. The resulting PCR product was designated as PCR product No. 16.

The DNA (1 µl) of the PCR product No. 14 was mixed with DNA (1 µl) of the PCR product No. 15. To the mixture, which was used as a template, were added 10 µl of 10× buffer, 4 µl of 2 mM dNTPs mixture, and 1 µl each of 10 µM primers JFH1-F (SEQ ID NO: 25) and 3189R-IH (SEQ ID NO: 19), which were from the Phusion High-Fidelity DNA Polymerase kit (FINNZYMES), then deionized water to bring the total amount to 49.5 µl. Thereafter, 0.5 µl of Phusion DNA Polymerase (FINNZYMES) was added, and PCR was then carried out for 30 cycles where one cycle consists of 98°C for 10 seconds, 55°C for 15 seconds, and 72°C for 2 minutes. The resulting PCR product was designated as PCR product No. 17. Each PCR product was purified and dissolved in 30 µl of H₂O.

The pJFH-1 cDNA and the purified PCR product No. 16 were separately digested with the EcoRI and KpnI restriction enzymes, and the HCV cDNA fragments were fractionated by agarose gel electrophoresis, followed by purification. The two DNA fragments were mixed with Ligation Mix (TAKARA BIO INC, Japan) to ligate the two DNA fragments. The vector was designated as pJFH-1 (1XFLAG). The pJFH-1 (1XFLAG) comprises a nucleotide sequence that encodes the gene of the strain JFH1, wherein it comprises, in the E2 HVR1 region, a nucleotide sequence that encodes the FLAG peptide sequence. Subsequently, the pJFH1 cDNA and the purified PCR product No. 17 were separately digested with the EcoRI and KpnI restriction enzymes, and the HCV cDNA fragments were fractionated by agarose gel electrophoresis, followed by purification. The two DNA fragments were mixed with Ligation Mix (TAKARA BIO INC, Japan) to ligate the two DNA fragments. The vector was designated as pJFH-1 (3XFLAG). The pJFH-1 (3XFLAG) comprises a nucleotide sequence that encodes the gene of the strain JFH-1, wherein it comprises, in the E2 HVR1 region, a nucleotide sequence that comprises 3 repeats of the FLAG peptide sequence.

The nucleotide sequences of the pJFH-1 (1XFLAG) and pJFH-1 (3XFLAG) are shown in SEQ ID NO: 30 and SEQ ID NO: 31, respectively, in the Sequence Listing.

### Example 11: Construction of plasmid TH/JFH-1 with FLAG-tag sequence inserted in HCV E2 HVR1 region

Used as a cDNA of HCV genomic RNA was the cDNA of the TH/JFH-1 chimera which comprises a sequence from 5'-UTR to the N-terminal 16^{th} amino acid of NS2 of the genotype 1b strain TH1 (Wakita, T. et al., J. Biol. Chem., 269, 14205-14210, 1994, JP Patent Publication (kokai) No. 2004-179 A) and a sequence from the N-terminal 17^{th} amino acid of NS2 to 3'-UTR of the genotype 2a strain JFH-1 (GenBank Accession No. AB047639, Kato, T. et al., Gastroenterology, 125, 2003, pp. 1808-1817). An epitope tag sequence was introduced into HVR1 of TH/JFH-1. The cDNA comprising 1 or 3 repeats of the FLAG sequence was inserted as the epitope tag. The resulting plasmids are referred to as pTH/JFH-1 (1XFLAG) and pTH/JFH-1 (3XFLAG), respectively.

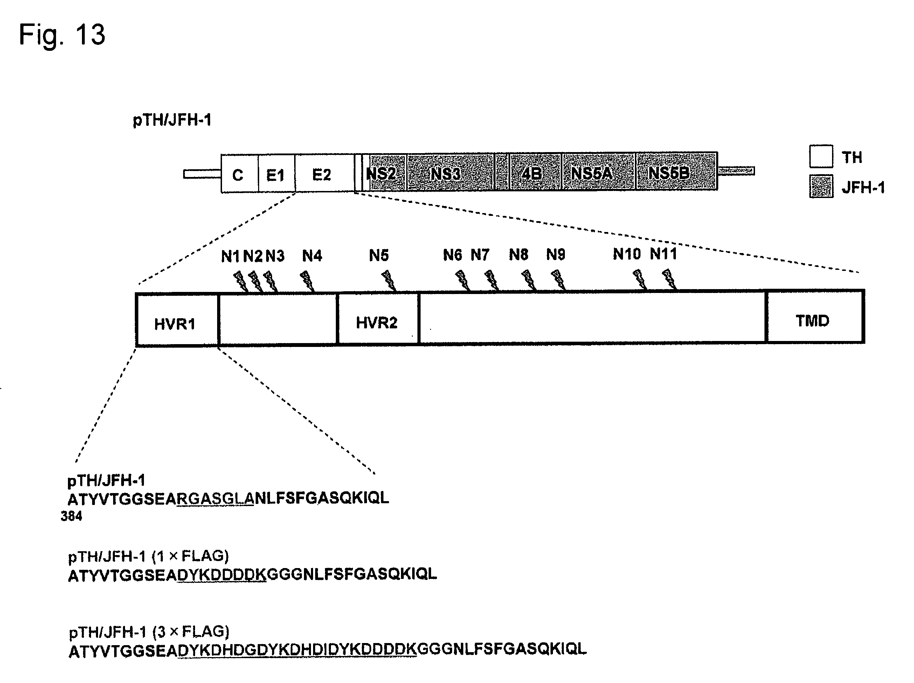

Fig. 13 shows the amino acid sequences which were in the vicinity of the FLAG-tag insertion sites translated from said genes and were from the N-terminal amino acid sequences of the E2 protein.

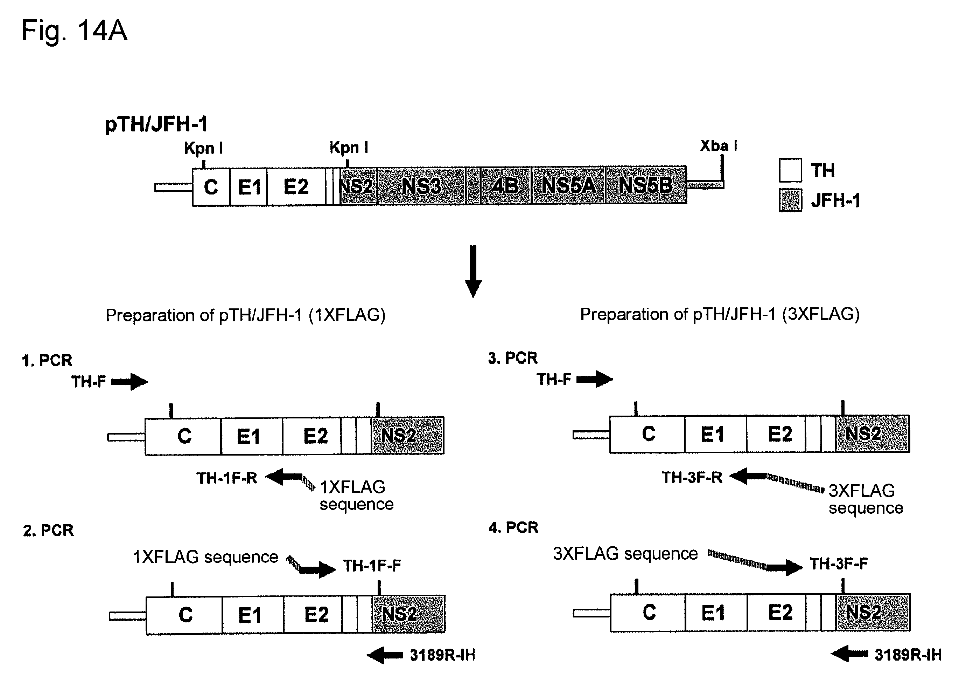

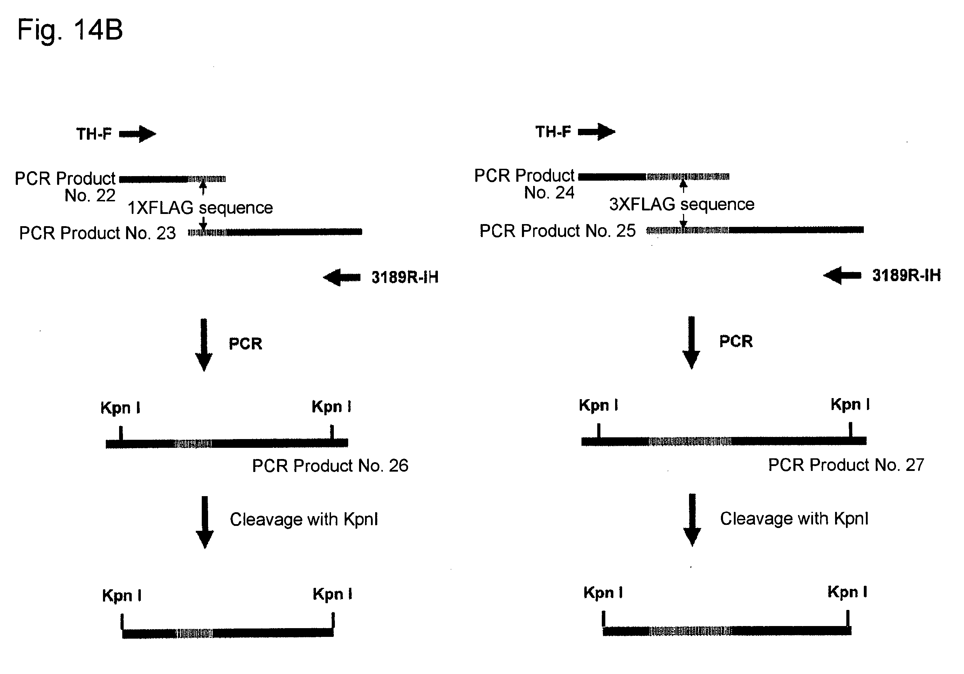

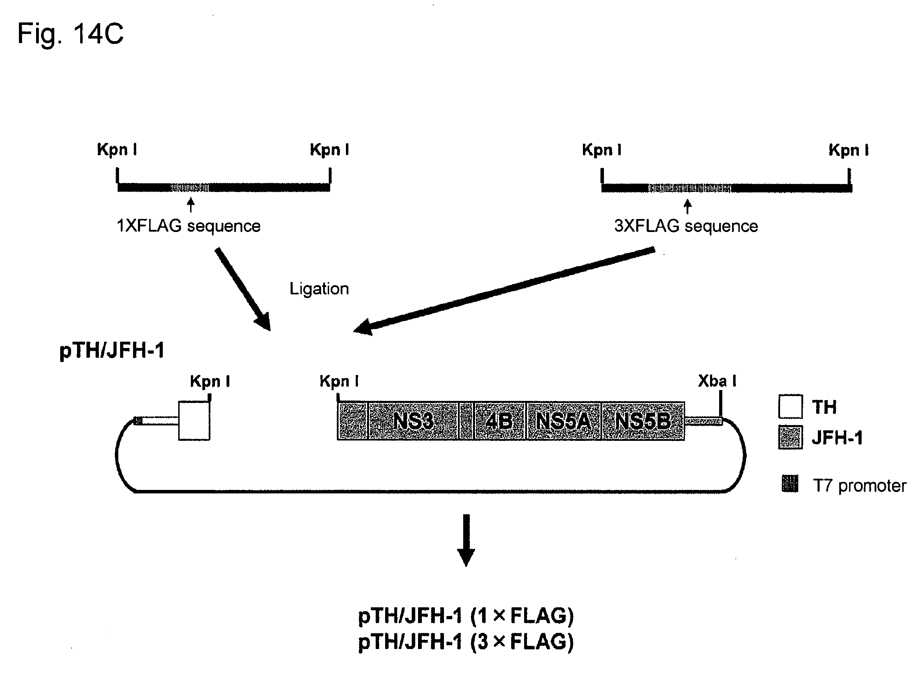

The N-terminal sequence of the E2 protein encoded by pTH/JFH-1, the N-terminal sequence of the E2 protein encoded by pTHJJFH-1 (1XFLAG), and the N-terminal sequence of the E2 protein encoded by pTH/JFH-1 (3XFLAG) are shown in SEQ ID NO: 32, SEQ ID NO: 33, and SEQ ID NO: 34, respectively. The methods for preparing the plasmids are shown in Fig. 14.

The TH/JFH-1 was first prepared. To the pJFH-1 cDNA as a template were added 10 µl of 10× buffer, 4 µl of 2 mM dNTPs mixture, and 1 µl each of 10 µM primers JFH1-A (SEQ ID NO: 43) and JFH1-B (SEQ ID NO: 44), which were from the Phusion High-Fidelity DNA Polymerase kit (FINNZYMES), then deionized water to bring the total amount to 49.5 µl. Thereafter, 0.5 µl of Phusion DNA Polymerase (FINNZYMES) was added, and PCR was then carried out to amplify the 5'- untranslated region of JFH-1 and a part of TH Core contained in the primer JFH1-B. PCR was carried out for 30 cycles where one cycle consists of 98°C for 10 seconds, 55°C for 15 seconds, and 72°C for 30 seconds. The resulting PCR product was designated as PCR product No. 18.

Subsequently, to the pTH as a template (Wakita, T. et al., J. Biol. Chem., 269, 14205-14210, 1994 and Moradpour et al., Biochem. Biophys. Res. Commun., 246: 920-924, 1998) were added 10 µl of 10× buffer, 4 µl of 2 mM dNTPs mixture, and 1 µl each of 10 µM primers TH-C (SEQ ID NO: 45) and TH-D (SEQ ID NO: 46), which were from the Phusion High-Fidelity DNA Polymerase kit (FINNZYMES) , then deionized water to bring the total amount to 49.5 µl. Thereafter, 0.5 µl of Phusion DNA Polymerase (FINNZYMES) was added, and PCR was then carried out to amplify Core to a part of NS2 of TH, a part of the 5'- untranslated region of JFH-1 contained in the primer TH-C, and a part of NS2 of JFH-1 contained in the primer YH-D. PCR was carried out for 30 cycles where one cycle consists of 98°C for 10 seconds, 55°C for 15 seconds, and 72°C for 30 seconds. The resulting PCR product was designated as PCR product No. 19.

To the pJFH-1 as a template were added 10 µl of 10× buffer, 4 µl of t2 mM dNTPs mixture, and 1 µl each of 10 µM primers JFH1-E (SEQ ID NO: 47) and JFH1-F (SEQ ID NO: 48), which were from the Phusion High-Fidelity DNA Polymerase kit (FINNZYMES), then deionized water to bring the total amount to 49.5 µl. Thereafter, 0.5 µl of Phusion DNA Polymerase (FINNZYMES) was added, and PCR was then carried out to amplify NS2 and a part of NS3 of JFH-1 and a part of NS2 of TH contained in the primer JFH1-E. PCR was carried out for 30 cycles where one cycle consists of 98°C for 10 seconds, 55°C for 15 seconds, and 72°C for 30 seconds. The resulting PCR product was designated as PCR product No. 20.

PCR was carried out using the PCR product Nos. 18, 19, and 20 and the JFH-A and JFH-E primers to obtain a fragment consisting of the 5' -translated region of the strain JFH-1 to the Core to a part of NS2 of the strain TH, and NS2 to a part of NS3 of JFH-1 (PCR product No. 21).

Subsequently, the pJFH-1 and the PCR product No. 21 were separately treated with EcoRI and SpeI restriction enzymes, and the fragments each was ligated to obtain pTH/JFH-1. The methods for preparing pTH/JFH-1 (1XFLAG) and pTH/JFH-1 (3XFLAG) using this plasmid are shown in Fig. 14 (A) to (C).

Specifically, to the pTH/JFH-1 cDNA as a template were added 10 µl of 10× buffer, 4 µl of 2 mM dNTPs mixture, and 1 µl each of 10 µM primers TH-F (SEQ ID NO: 35) and TH-1F-R (SEQ ID NO: 36), which were from the Phusion High-Fidelity DNA Polymerase kit (FINNZYMES), then deionized water to bring the total amount to 49.5 µl. Thereafter, 0.5 µl of Phusion DNA Polymerase (FINNZYMES) was added, and PCR was then carried out for 30 cycles where one cycle consists of 98°C for 10 seconds, 55°C for 15 seconds, and 72°C for 30 seconds. The resulting PCR product was designated as PCR product No. 22.

To the pTH/JFH-1 cDNA as a template were added 10 µl of 10× buffer, 4 µl of 2 mM dNTPs mixture, and 1 µl each of 10 µM primers 3189R-IH (SEQ ID NO: 19) and TH-1F-F (SEQ ID NO: 37), which were from the Phusion High-Fidelity DNA Polymerase kit (FINNZYMES), then deionized water to bring the total amount to 49.5 µl. Thereafter, 0.5 µl of Phusion DNA Polymerase (FINNZYMES) was added, and PCR was then carried out. PCR was carried out for 30 cycles where one cycle consists of 98°C for 10 seconds, 55°C for 15 seconds, and 72°C for 1.5 minutes. The resulting PCR product was designated as PCR product No. 23.

Subsequently, to the pTH/JFH1 cDNA as a template were added 10 µl of 10× buffer, 4 µl of 2 mM dNTPs mixture, and 1 µl each of 10 µM primers TH-F (SEQ ID NO: 35) and TH-3F-R (SEQ ID NO: 38), which were from the Phusion High-Fidelity DNA Polymerase kit (FINNZYMES), then deionized water to bring the total amount to 49.5 µl. Thereafter, 0.5 µl of Phusion DNA Polymerase (FINNZYMES) was added, and PCR was then carried out for 30 cycles where one cycle consists of 98°C for 10 seconds, 55°C for 15 seconds, and 72°C for 30 seconds. The resulting PCR product was designated as PCR product No. 24.

Subsequently, to the pTH/JFH-1 cDNA as a template were added 10 µl of 10× buffer, 4 µl of 2 mM dNTPs mixture, and 1 µl each of 10 µM primers 3189R-IH (SEQ ID NO: 19) and TH-3F-F (SEQ ID NO: 39), which were from the Phusion High-Fidelity DNA Polymerase kit (FINNZYMES), then deionized water was added to bring the total amount to 49.5 µl in the end. Thereafter, 0.5 µl of Phusion DNA Polymerase (FINNZYMES) was added, and PCR was then carried out for 30 cycles where one cycle consists of 98°C for 10 seconds, 55°C for 15 seconds, and 72°C for 1.5 minutes. The resulting PCR product was designated as PCR product No. 25.

The PCR products were separately purified and dissolved in 15 µl of H₂O. DNA (1 µ1) of the PCR product No. 22 was mixed with DNA (1 µl) of the PCR product No. 23. To the mixture, which was used as a template, were added 10 µl of 10× buffer, 4 µl of 2 mM dNTPs mixture, and 1 µl each of 10 µM primers TH-F (SEQ ID NO: 35) and 3189R-IH (SEQ ID NO: 19), which were from the Phusion High-Fidelity DNA Polymerase kit (FINNZYMES), then deionized water to bring the total amount to 49.5 µl. Thereafter, 0.5 µl of Phusion DNA Polymerase (FINNZYMES) was added, and PCR was then carried out. PCR was carried out for 30 cycles where one cycle consists of 98°C for 10 seconds, 55°C for 15 seconds, and 72°C for 2 minutes. The resulting PCR product was designated as PCR product No. 26.

Subsequently, DNA (1 µl) of the PCR product No. 24 was mixed with DNA (1 µl) of the PCR product No. 25. To the mixture, which was used as a template, were added 10 µl of 10× buffer, 4 µl of 2 mM dNTPs mixture, and 1 µl each of 10 µM primers TH-F (SEQ ID NO: 35) and 3189R-IH (SEQ ID NO: 19), which were from the Phusion High-Fidelity DNA Polymerase kit (FINNZYMES), then deionized water to bring the total amount to 49.5 µl. Thereafter, 0.5 µl of Phusion DNA Polymerase (FINNZYMES) was added, and PCR was then carried out For 30 cycles where one cycle consists of 98°C for 10 seconds, 55°C for 15 seconds, and 72°C for 2 minutes. The resulting PCR product was designated as PCR product No. 27. The PCR products were purified and dissolved in 30 µl of H₂O.

The pTH/JFH-1 cDNA and the purified PCR product No. 26 were separately digested with the KpnI restriction enzyme, and the HCV cDNA fragments were fractionated by agarose gel electrophoresis, followed by purification. The two DNA fragments were mixed with Ligation Mix (TAKARA BIO INC, Japan) to ligate the two DNA fragments. The vector was designated as pTH/JFH-1 (1XFLAG). The pTH/JFH-1 (1XFLAG) comprises a nucleotide sequence that encodes the gene of the strain TH1 and comprises, in the E2 HVR1 region, a nucleotide sequence that encodes the FLAG peptide sequence.

The pTH/JFH1 cDNA and the purified PCR product No. 27 were separately digested with the KpnI restriction enzyme, and the HCV cDNA fragments were fractionated by agarose gel electrophoresis, followed by purification. The two DNA fragments were mixed with Ligation Mix (TAKARA BIO INC, Japan) to ligate the two DNA fragments. The vector was designated as pTH/JFH-1 (3XFLAG). The pTH/JFH-1 (3XFLAG) comprises a nucleotide sequence that encodes the gene of the strain TH1 and comprises, in the E2 HVR1 region, a nucleotide sequence that comprises 3 repeats of the FLAG peptide sequence.

The nucleotide sequences of pTH/JFH1, pTH/JFH-1 (1XFLAG), and pTH/JFH-1 (3XFLAG) are shown in SEQ ID NO: 40, SEQ ID NO: 41, and SEQ ID NO: 42, respectively, in the Sequence Listing.

All RNAs synthesized from the vectors prepared in the above Examples generated epitope-tagged HCV particles, when introduced into cultured HCV-permissive cells (e.g., Huh7). The culture supernatant containing epitope-tagged HCV particles was allowed to pass through the column filled with a support to which an anti-epitope tagged antibody had been immobilized so as to specifically adsorb the epitope-tagged HCV particles to the supports, and then subjected to elution. Thus, the HCV particles of interest were purified.

### Industrial Applicability

According to the method of producing HCV particles in a cultured cell system and purifying the same of the present invention, HCV particles can be purified more easily at a higher purity than conventional techniques. Since the HCV particles provided by the method of the present invention are of high purity, they are suitable for vaccines for preventing or treating HCV. Further, the epitope-tagged HCV particles of the present invention can be suitably used as tools for inducing antibodies to HCV.

All publications, patents, and patent applications cited herein are incorpora ted herein by reference in their entirety.

### Sequence Listing Free Text

SEQ ID NO: 1: N terminal sequence of E2 protein encoded by J6/JFH-1
SEQ ID NO: 2: N terminal sequence of E2 protein encoded by pJ6/JFH-1 (1XFLAG)
SEQ ID NO: 3: N terminal sequence of E2 protein encoded by pJ6/JFH-1 (3XFLAG)
SEQ ID NO: 4: Primer (E2-F)
SEQ ID NO: 5: Primer (1F-R)
SEQ ID NO: 6: Primer (E2-R)
SEQ ID NO: 7: Primer (1F-F)
SEQ ID NO: 8: Primer (E2-F)
SEQ ID NO: 9: Primer (3F-R)
SEQ ID NO: 10: pJ6/JFH-1
SEQ ID NO: 11: pJ6/JFH-1 (1XFLAG)
SEQ ID NO: 12: pJ6/JFH-1 (3XFLAG)
SEQ ID NO: 13: Primer (S-17)
SEQ ID NO: 14: Primer (R-19)
SEQ ID NO: 15: Probe
SEQ ID NO: 16: Primer (1141S-2a)
SEQ ID NO: 17: Primer (J6E2-K-R)
SEQ ID NO: 18: Primer (J6E2-K-F)
SEQ ID NO: 19: Primer (3189R-1H)
SEQ ID NO: 20: pJ6/JFH-1 (1×FLAG) N→K
SEQ ID NO: 21: pJ6/JFH-1 (3×FLAG) N→K
SEQ ID NO: 22: N terminal sequence of E2 protein encoded by pJFH-1
SEQ ID NO: 23: N terminal sequence of E2 protein encoded by pJFH-1 (1XFLAG)
SEQ ID NO: 24: N terminal sequence of E2 protein encoded by pJFH-1 (3XFLAG)
SEQ ID NO: 25: Primer (JFH1-F)
SEQ ID NO: 26: Primer (JFH1-1F-R)
SEQ ID NO: 27: Primer (JFH1-1F-F)
SEQ ID NO: 28: Primer (JFH1-3F-R)
SEQ ID NO: 29: Primer (JFH1-3F-F)
SEQ ID NO: 30: pJFH-1 (1XFLAG)
SEQ ID NO: 31: pJFH-1 (3XFLAG)
SEQ ID NO: 32: N terminal sequence of E2 protein encoded by pTH/JFH-1
SEQ ID NO: 33: N terminal sequence of E2 protein encoded by pTH/JFH-1 (1XFLAG)
SEQ ID NO: 34: N terminal sequence of E2 protein encoded by pTH/JFH-1 (3XFLAG)
SEQ ID NO: 35: Primer (TH-F)
SEQ ID NO: 36: Primer (TH-1F-R)
SEQ ID NO: 37: Primer (TH-1F-F)
SEQ ID NO: 38: Primer (TH-3F-R)
SEQ ID NO: 39: Primer (TH-3F-F)
SEQ ID NO: 40: pTH/JFH1
SEQ ID NO: 41: pTH/JFH1 (1FLAG)
SEQ ID NO: 42: pTH/JFH1 (3XFLAG)
SEQ ID NO: 43: Primer (JFH1-A)
SEQ ID NO: 44: Primer (JFH1-B)
SEQ ID NO: 45: Primer (TH-C)
SEQ ID NO: 46: Primer (TH-D)
SEQ ID NO: 47: Primer (JFH1-E)
SEQ ID NO: 48: Primer (JFH1-F)
SEQ ID NO: 49: Epitope tag peptide (1XFLAG+Linker)
SEQ ID NO: 50: Epitope tag peptide (3XFLAG+Linker)
SEQ ID NO: 51: Epitope tag peptide (1XFLAG)
SEQ ID NO: 52: Epitope tag peptide (3XFLAG)
SEQ ID NO: 53: Linker
SEQ ID NO: 54: Linker

## Claims

1. A nucleic acid comprising (a), (b), or (c) below and comprising a nucleic acid sequence encoding an epitope tag peptide at hypervariable region 1 (HVR1) in an E2 protein coding sequence of the (a), (b), or (c):
(a) the genome of hepatitis C virus (HCV) JFH-1 strain;
(b) a chimeric hepatitis C virus (HCV) genome comprising: 5'-untranslated region, a Core protein coding sequence, an E1 protein coding sequence, an E2 protein coding sequence, and a p7 protein coding sequence of HCV J6CF strain; and a NS2 protein coding sequence, a NS3 protein coding sequence, a NS4A protein coding sequence, a NS4B protein coding sequence, a NS5A protein coding sequence, a NS5B protein coding sequence, and 3'-untranslated region of the HCV JFH-1 strain; or
(c) a chimeric hepatitis C virus (HCV) genome comprising: 5'-untranslated region of the JFH-1 strain; a Core protein coding sequence, an E1 protein coding sequence, an E2 protein coding sequence, and a p7 protein coding sequence of HCV TH1 strain; and a NS2 protein coding sequence, a NS3 protein coding sequence, a NS4A protein coding sequence, a NS4B protein coding sequence, a NS5A protein coding sequence, a NS5B protein coding sequence, and 3'- untranslated region of the HCV JFH-1 strain.

2. The nucleic acid according to claim 1, wherein the epitope tag peptide comprises the amino acid sequence DYKDDDDK or DYKDHDGDYKDHDIDYKDDDDK.

3. The nucleic acid according to claim 1 or 2, wherein the epitope tag peptide further comprises a linker sequence.

4. The nucleic acid according to claim 3, wherein the linker sequence comprises GGG sequence, GGGGS sequence, or (GGGGS)₃ sequence, or a sequence comprising deletion, substitution or addition of 1-10 amino acids in a part of the sequence GGG, GGGGS or (GGGGS)₃.

5. The nucleic acid according to any one of claims 1 to 4, which is shown in SEQ ID NO: 11, 12, 30, 31, 41, or 42, wherein nucleotide "T" shown in the sequence listing is read as "U" when the nucleic acid is RNA.

6. The nucleic acid according to any one of claims 1 to 5, which, when the amino acid subsequent to the end of the sequence of the hypervariable region 1 (HVR1) is designated as an amino acid at position 1, comprises a mutation which is a substitution of asparagine at position 122 or 124 with lysine.

7. The nucleic acid according to claim 6, which is shown in SEQ ID NO: 20 or 21 wherein nucleotide "T" shown in the Sequence Listing is read as "U" when the nucleic acid is RNA.

8. A vector comprising the nucleic acid according to any one of claims 1 to 7.

9. An epitope-tagged hepatitis C virus (HCV) particle comprising the nucleic acid according to any one of claims 1 to 7 as the genome.

10. A cell comprising the epitope-tagged hepatitis C virus (HCV) particle according to claim 9.

11. The cell according to claim 10, which is Huh7 or a cell line derived therefrom.

12. A method for purifying epitope-tagged hepatitis C virus (HCV) particle comprising:
a step of preparing epitope-tagged HCV particles from the cell according to claim 10 or 11;
a step of bringing a solution containing the epitope-tagged HCV particles into contact with an anti-epitope tag antibody-immobilized support and then adsorbing the epitope-tagged HCV particles to the anti-epitope tag antibody-immobilized support; and
a step of releasing the epitope-tagged HCV particles from the anti-epitope tag antibody-immobilized support to obtain the epitope-tagged HCV particles.

13. The method of purification according to claim 12, wherein the releasing step comprises releasing the epitope-tagged HCV particles from the anti-epitope tag antibody-immobilized support using any of a buffer containing epitope tag peptide, a buffer containing no calcium, 0.1M glycine buffer (pH 2.5-4.0), 1M arginine HCl buffer (pH 4.0-5.0), or 0.1-1 mM HC1.

14. An hepatitis C virus (HCV) vaccine, which is obtained by inactivating the epitope-tagged HCV particle according to claim 9.

15. An anti-hepatitis C virus (HCV) antibody to the epitope-tagged HCV particle according to claim 9.


SEQUENCE LISTING

<110> Japan as Represented by Director General of National
      Institute of Infectious Diseases
      Tokyo Metropolitan Organization for Medical Research
      Toray Industries, Inc.

<120> Production and use of epitope-tagged hepatitis C virus
      particle

<130> PH-3617-PCT

<140> PCT/JP2008/062977
<141> 2008-07-11

<150> JP 2007-184587
<151> 2007-07-13

<160> 54

<170> PatentIn version 3.4

<210> 1
<211> 51
<212> PRT
<213> Artificial

<220>
<223> N terminal sequence of E2 protein encoded in J6/JFH-1

<400> 1

Arg Thr His Thr Val Gly Gly Ser Ala Ala Gln Thr Thr Gly Arg Leu
1               5                   10                  15

Thr Ser Leu Phe Asp Met Gly Pro Arg Gln Lys Ile Gln Leu Val Asn
            20                  25                  30

Thr Asn Gly Ser Trp His Ile Asn Arg Thr Ala Asn Cys Asn Asp Ser
        35                  40                  45

Leu His Thr
    50

<210>  2
<211>  55
<212>  PRT
<213>  Artificial

<220>
<223>  N terminal sequence of E2 protein encoded in pJ6/JFH-1 (1XFLAG)

<400>  2

Arg Thr His Thr Val Gly Gly Ser Ala Ala Asp Tyr Lys Asp Asp Asp
1               5                   10                  15

Asp Lys Gly Gly Gly Ser Leu Phe Asp Met Gly Pro Arg Gln Lys Ile
            20                  25                  30

Gln Leu Val Asn Thr Asn Gly Ser Trp His Ile Asn Arg Thr Ala Asn
        35                  40                  45

Cys Asn Asp Ser Leu His Thr
    50                  55

<210>  3
<211>  69
<212>  PRT
<213>  Artificial

<220>
<223>  N terminal sequence of E2 protein encoded in pJ6/JFH-1 (3XFLAG)

<400>  3

Arg Thr His Thr Val Gly Gly Ser Ala Ala Asp Tyr Lys Asp His Asp
1               5                   10                  15

Gly Asp Tyr Lys Asp His Asp Ile Asp Tyr Lys Asp Asp Asp Asp Lys
            20                  25                  30

Gly Gly Gly Ser Leu Phe Asp Met Gly Pro Arg Gln Lys Ile Gln Leu
        35                  40                  45

Val Asn Thr Asn Gly Ser Trp His Ile Asn Arg Thr Ala Asn Cys Asn
    50                  55                  60

Asp Ser Leu His Thr
65

<210> 4
<211> 20
<212> DNA
<213> Artificial

<220>
<223> primer (E2-F)

<400> 4
cctggtacca tcactggaca                                                20

<210> 5
<211> 44
<212> DNA
<213> Artificial

<220>
<223> primer (1F-R)

<400> 5
cccttgtcat cgtcatcctt gtaatccgcg gcagaacccc caac                    44

<210> 6
<211> 20
<212> DNA
<213> Artificial

<220>
<223> primer (E2-R)

<400> 6
tgggtggtac ccactcctga 20


<210> 7
<211> 44
<212> DNA
<213> Artificial

<220>
<223> primer (1F-F)

<400> 7
aggatgacga tgacaaggga ggcggtagct tatttgacat gggc 44


<210> 8
<211> 65
<212> DNA
<213> Artificial

<220>
<223> primer (3F-R)

<400> 8
taatcgatgt catgatcttt ataatcaccg tcatggtctt tgtagtccgc ggcagaaccc 60

ccaac 65


<210> 9
<211> 65

<212>  DNA
<213>  Artificial

<220>
<223>  primer (3F-F)

<400>  9
aagatcatga catcgattac aaggatgacg atgacaaggg aggcggtagc ttatttgaca      60

tgggc                                                                  65


<210>  10
<211>  9683
<212>  DNA
<213>  Artificial

<220>
<223>  pJ6/JFH-1

<400>  10
acccgcccct aataggggcg acactccgcc atgaatcact cccctgtgag gaactactgt      60

cttcacgcag aaagcgtcta gccatggcgt tagtatgagt gtcgtacagc ctccaggccc     120

ccccctcccg ggagagccat agtggtctgc ggaaccggtg agtacaccgg aattgccggg     180

aagactgggt cctttcttgg ataaacccac tctatgcccg gccatttggg cgtgcccccg     240

caagactgct agccgagtag cgttgggttg cgaaaggcct tgtggtactg cctgataggg     300

tgcttgcgag tgccccggga ggtctcgtag accgtgcacc atgagcacaa atcctaaacc     360

tcaaagaaaa accaaaagaa acaccaaccg tcgcccacaa gacgttaagt ttccgggcgg     420

cggccagatc gttggcggag tatacttgtt gccgcgcagg ggccccaggt tgggtgtgcg     480

cgcgacaagg aagacttcgg agcggtccca gccacgtgga aggcgccagc ccatccctaa     540

agatcggcgc tccactggca aatcctgggg aaaaccagga tacccctggc ccctatacgg     600

gaatgaggga ctcggctggg caggatggct cctgtccccc cgaggttccc gtccctcttg     660

gggccccaat gacccccggc ataggtcgcg caacgtgggt aaggtcatcg ataccctaac 720

gtgcggcttt gccgacctca tggggtacat ccctgtcgtg ggcgccccgc tcggcggcgt 780

cgccagagct ctcgcgcatg gcgtgagagt cctggaggac ggggttaatt ttgcaacagg 840

gaacttaccc ggttgctcct tttctatctt cttgctggcc ctgctgtcct gcatcaccac 900

cccggtctcc gctgccgaag tgaagaacat cagtaccggc tacatggtga ctaacgactg 960

caccaatgac agcattacct ggcagctcca ggctgctgtc ctccacgtcc ccgggtgcgt 1020

cccgtgcgag aaagtgggga atgcatctca gtgctggata ccggtctcac cgaatgtggc 1080

cgtgcagcgg cccggcgccc tcacgcaggg cttgcggacg cacatcgaca tggttgtgat 1140

gtccgccacg ctctgctctg ccctctacgt gggggacctc tgcggtgggg tgatgctcgc 1200

agcccaaatg ttcattgtct cgccgcagca ccactggttt gtccaagact gcaattgctc 1260

catctaccct ggtaccatca ctggacaccg catggcatgg gacatgatga tgaactggtc 1320

gcccacggct accatgatct tggcgtacgc gatgcgtgtc cccgaggtca ttatagacat 1380

cattagcggg gctcattggg gcgtcatgtt cggcttggcc tacttctcta tgcagggagc 1440

gtgggcgaaa gtcgttgtca tccttctgtt ggccgccggg gtggacgcgc gcacccatac 1500

tgttgggggt tctgccgcgc agaccaccgg gcgcctcacc agcttatttg acatgggccc 1560

caggcagaaa atccagctcg ttaacaccaa tggcagctgg cacatcaacc gcaccgccct 1620

gaactgcaat gactccttgc acaccggctt tatcgcgtct ctgttctaca cccacagctt 1680

caactcgtca ggatgtcccg aacgcatgtc cgcctgccgc agtatcgagg ccttccgggt 1740

gggatggggc gccttgcaat atgaggataa tgtcaccaat ccagaggata tgagacccta 1800

ttgctggcac tacccaccaa ggcagtgtgg cgtggtctcc gcgaagactg tgtgtggccc 1860

agtgtactgt ttcaccccca gcccagtggt agtgggcacg accgacaggc ttggagcgcc 1920

cacttacacg tggggggaga atgagacaga tgtcttccta ttgaacagca ctcgaccacc 1980

gctggggtca tggttcggct gcacgtggat gaactcttct ggctacacca agacttgcgg 2040

cgcaccaccc tgccgtacta gagctgactt caacgccagc acggacctgt tgtgccccac 2100

ggactgtttt aggaagcatc ctgataccac ttacctcaaa tgcggctctg ggccctggct 2160

cacgccaagg tgcctgatcg actaccccta caggctctgg cattacccct gcacagttaa 2220

ctataccatc ttcaaaataa ggatgtatgt gggaggggtt gagcacaggc tcacggctgc 2280

atgcaatttc actcgtgggg atcgttgcaa cttggaggac agagacagaa gtcaactgtc 2340

tcctttgttg cactccacca cggaatgggc cattttacct tgctcttact cggacctgcc 2400

cgccttgtcg actggtcttc tccacctcca ccaaaacatc gtggacgtac aattcatgta 2460

tggcctatca cctgccctca caaaatacat cgtccgatgg gagtgggtaa tactcttatt 2520

cctgctctta gcggacgcca gggtttgcgc ctgcttatgg atgctcatct tgttgggcca 2580

ggccgaagca gcactagaga agctggtcat cttgcacgct gcgagcgcag ctagctgcaa 2640

tggcttccta tattttgtca tctttttcgt ggctgcttgg tacatcaagg gtcgggtagt 2700

ccccttagct acctattccc tcactggcct gtggtccttt agcctactgc tcctagcatt 2760

gccccaacag gcttatgctt atgacgcatc tgtgcatggc cagataggag cggctctgct 2820

ggtaatgatc accctcttca cactcacccc ggggtataag accctcctcg gccagtgtct 2880

gtggtggttg tgctatctcc tgaccctggg ggaagccatg attcaggagt gggtaccacc 2940

catgcaggtg cgcggcggcc gcgatggcat cgcgtgggcc gtcactatat tctgcccggg 3000

tgtggtgttt gacattacca aatggctttt ggcgttgctt gggcctgctt acctcttaag 3060

ggccgctttg acacatgtgc cgtacttcgt cagagctcac gctctgataa gggtatgcgc 3120

tttggtgaag cagctcgcgg ggggtaggta tgttcaggtg gcgctattgg cccttggcag 3180

gtggactggc acctacatct atgaccacct cacacctatg tcggactggg ccgctagcgg 3240

cctgcgcgac ttagcggtcg ccgtggaacc catcatcttc agtccgatgg agaagaaggt 3300

catcgtctgg ggagcggaga cggctgcatg tggggacatt ctacatggac ttcccgtgtc 3360

cgcccgactc ggccaggaga tcctcctcgg cccagctgat ggctacacct ccaaggggtg 3420

gaagctcctt gctcccatca ctgcttatgc ccagcaaaca cgaggcctcc tgggcgccat 3480

agtggtgagt atgacggggc gtgacaggac agaacaggcc ggggaagtcc aaatcctgtc 3540

cacagtctct cagtccttcc tcggaacaac catctcgggg gttttgtgga ctgtttacca 3600

cggagctggc aacaagactc tagccggctt acggggtccg gtcacgcaga tgtactcgag 3660

tgctgagggg gacttggtag gctggcccag cccccctggg accaagtctt tggagccgtg 3720

caagtgtgga gccgtcgacc tatatctggt cacgcggaac gctgatgtca tcccggctcg 3780

gagacgcggg gacaagcggg gagcattgct ctccccgaga cccatttcga ccttgaaggg 3840

gtcctcgggg gggccggtgc tctgccctag gggccacgtc gttgggctct tccgagcagc 3900

tgtgtgctct cggggcgtgg ccaaatccat cgatttcatc cccgttgaga cactcgacgt 3960

tgttacaagg tctcccactt tcagtgacaa cagcacgcca ccggctgtgc cccagaccta 4020

tcaggtcggg tacttgcatg ctccaactgg cagtggaaag agcaccaagg tccctgtcgc 4080

gtatgccgcc caggggtaca aagtactagt gcttaacccc tcggtagctg ccaccctggg 4140

gtttggggcg tacctatcca aggcacatgg catcaatccc aacattagga ctggagtcag 4200

gaccgtgatg accggggagg ccatcacgta ctccacatat ggcaaatttc tcgccgatgg 4260

gggctgcgct agcggcgcct atgacatcat catatgcgat gaatgccacg ctgtggatgc 4320

tacctccatt ctcggcatcg gaacggtcct tgatcaagca gagacagccg gggtcagact 4380

aactgtgctg gctacggcca caccccccgg gtcagtgaca accccccatc ccgatataga 4440

agaggtaggc ctcgggcggg agggtgagat ccccttctat gggagggcga ttcccctatc 4500

ctgcatcaag ggagggagac acctgatttt ctgccactca aagaaaaagt gtgacgagct 4560

cgcggcggcc cttcggggca tgggcttgaa tgccgtggca tactatagag ggttggacgt 4620

ctccataata ccagctcagg gagatgtggt ggtcgtcgcc accgacgccc tcatgacggg 4680

gtacactgga gactttgact ccgtgatcga ctgcaatgta gcggtcaccc aagctgtcga 4740

cttcagcctg gaccccacct tcactataac cacacagact gtcccacaag acgctgtctc 4800

acgcagtcag cgccgcgggc gcacaggtag aggaagacag ggcacttata ggtatgtttc 4860

cactggtgaa cgagcctcag gaatgtttga cagtgtagtg ctttgtgagt gctacgacgc 4920

aggggctgcg tggtacgatc tcacaccagc ggagaccacc gtcaggctta gagcgtattt 4980

caacacgccc ggcctacccg tgtgtcaaga ccatcttgaa ttttgggagg cagttttcac 5040

cggcctcaca cacatagacg cccacttcct ctcccaaaca aagcaagcgg gggagaactt 5100

cgcgtaccta gtagcctacc aagctacggt gtgcgccaga gccaaggccc ctcccccgtc 5160

ctgggacgcc atgtggaagt gcctggcccg actcaagcct acgcttgcgg gccccacacc 5220

tctcctgtac cgtttgggcc ctattaccaa tgaggtcacc ctcacacacc ctgggacgaa 5280

gtacatcgcc acatgcatgc aagctgacct tgaggtcatg accagcacgt gggtcctagc 5340

tggaggagtc ctggcagccg tcgccgcata ttgcctggcg actggatgcg tttccatcat 5400

cggccgcttg cacgtcaacc agcgagtcgt cgttgcgccg gataaggagg tcctgtatga 5460

ggcttttgat gagatggagg aatgcgcctc tagggcggct ctcatcgaag aggggcagcg 5520

gatagccgag atgttgaagt ccaagatcca aggcttgctg cagcaggcct ctaagcaggc 5580

ccaggacata caacccgcta tgcaggcttc atggcccaaa gtggaacaat tttgggccag 5640

acacatgtgg aacttcatta gcggcatcca atacctcgca ggattgtcaa cactgccagg 5700

gaaccccgcg gtggcttcca tgatggcatt cagtgccgcc ctcaccagtc cgttgtcgac 5760

cagtaccacc atccttctca acatcatggg aggctggtta gcgtcccaga tcgcaccacc 5820

cgcgggggcc accggctttg tcgtcagtgg cctggtgggg gctgccgtgg gcagcatagg 5880

cctgggtaag gtgctggtgg acatcctggc aggatatggt gcgggcattt cgggggccct 5940

cgtcgcattc aagatcatgt ctggcgagaa gccctctatg gaagatgtca tcaatctact 6000

gcctgggatc ctgtctccgg gagccctggt ggtgggggtc atctgcgcgg ccattctgcg 6060

ccgccacgtg ggaccggggg agggcgcggt ccaatggatg aacaggctta ttgcctttgc 6120

ttccagagga aaccacgtcg cccctactca ctacgtgacg gagtcggatg cgtcgcagcg 6180

tgtgacccaa ctacttggct ctcttactat aaccagccta ctcagaagac tccacaattg 6240

gataactgag gactgcccca tcccatgctc cggatcctgg ctccgcgacg tgtgggactg 6300

ggtttgcacc atcttgacag acttcaaaaa ttggctgacc tctaaattgt tccccaagct 6360

gcccggcctc cccttcatct cttgtcaaaa ggggtacaag ggtgtgtggg ccggcactgg 6420

catcatgacc acgcgctgcc cttgcggcgc caacatctct ggcaatgtcc gcctgggctc 6480

tatgaggatc acagggccta aaacctgcat gaacacctgg caggggacct ttcctatcaa 6540

ttgctacacg gagggccagt gcgcgccgaa accccccacg aactacaaga ccgccatctg 6600

gagggtggcg gcctcggagt acgcggaggt gacgcagcat gggtcgtact cctatgtaac 6660

aggactgacc actgacaatc tgaaaattcc ttgccaacta ccttctccag agtttttctc 6720

ctgggtggac ggtgtgcaga tccataggtt tgcacccaca ccaaagccgt ttttccggga 6780

tgaggtctcg ttctgcgttg ggcttaattc ctatgctgtc gggtcccagc ttccctgtga 6840

acctgagccc gacgcagacg tattgaggtc catgctaaca gatccgcccc acatcacggc 6900

ggagactgcg gcgcggcgct tggcacgggg atcacctcca tctgaggcga gctcctcagt 6960

gagccagcta tcagcaccgt cgctgcgggc cacctgcacc acccacagca acacctatga 7020

cgtggacatg gtcgatgcca acctgctcat ggagggcggt gtggctcaga cagagcctga 7080

gtccagggtg cccgttctgg actttctcga gccaatggcc gaggaagaga gcgaccttga 7140

gccctcaata ccatcggagt gcatgctccc caggagcggg tttccacggg ccttaccggc 7200

ttgggcacgg cctgactaca acccgccgct cgtggaatcg tggaggaggc cagattacca 7260

accgcccacc gttgctggtt gtgctctccc cccccccaag aaggccccga cgcctccccc 7320

aaggagacgc cggacagtgg gtctgagcga gagcaccata tcagaagccc tccagcaact 7380

ggccatcaag acctttggcc agcccccctc gagcggtgat gcaggctcgt ccacgggggc 7440

gggcgccgcc gaatccggcg gtccgacgtc ccctggtgag ccggccccct cagagacagg 7500

ttccgcctcc tctatgcccc ccctcgaggg ggagcctgga gatccggacc tggagtctga 7560

tcaggtagag cttcaacctc ccccccaggg ggggggggta gctcccggtt cgggctcggg 7620

gtcttggtct acttgctccg aggaggacga taccaccgtg tgctgctcca tgtcatactc 7680

ctggaccggg gctctaataa ctccctgtag ccccgaagag gaaaagttgc caatcaaccc 7740

tttgagtaac tcgctgttgc gataccataa caaggtgtac tgtacaacat caaagagcgc 7800

ctcacagagg gctaaaaagg taacttttga caggacgcaa gtgctcgacg cccattatga 7860

ctcagtctta aaggacatca agctagcggc ttccaaggtc agcgcaaggc tcctcacctt 7920

ggaggaggcg tgccagttga ctccacccca ttctgcaaga tccaagtatg gattcggggc 7980

caaggaggtc cgcagcttgt ccgggagggc cgttaaccac atcaagtccg tgtggaagga 8040

cctcctggaa gacccacaaa caccaattcc cacaaccatc atggccaaaa atgaggtgtt 8100

ctgcgtggac cccgccaagg ggggtaagaa accagctcgc ctcatcgttt accctgacct 8160

cggcgtccgg gtctgcgaga aaatggccct ctatgacatt acacaaaagc ttcctcaggc 8220

ggtaatggga gcttcctatg gcttccagta ctcccctgcc caacgggtgg agtatctctt 8280

gaaagcatgg gcggaaaaga aggaccccat gggtttttcg tatgataccc gatgcttcga 8340

ctcaaccgtc actgagagag acatcaggac cgaggagtcc atataccagg cctgctccct 8400

gcccgaggag gcccgcactg ccatacactc gctgactgag agactttacg taggagggcc 8460

catgttcaac agcaagggtc aaacctgcgg ttacagacgt tgccgcgcca gcggggtgct 8520

aaccactagc atgggtaaca ccatcacatg ctatgtgaaa gccctagcgg cctgcaaggc 8580

tgcggggata gttgcgccca caatgctggt atgcggcgat gacctagtag tcatctcaga 8640

aagccagggg actgaggagg acgagcggaa cctgagagcc ttcacggagg ccatgaccag 8700

gtactctgcc cctcctggtg atccccccag accggaatat gacctggagc taataacatc 8760

ctgttcctca aatgtgtctg tggcgttggg cccgcggggc cgccgcagat actacctgac 8820

cagagaccca accactccac tcgcccgggc tgcctgggaa acagttagac actcccctat 8880

caattcatgg ctgggaaaca tcatccagta tgctccaacc atatgggttc gcatggtcct 8940

aatgacacac ttcttctcca ttctcatggt ccaagacacc ctggaccaga acctcaactt 9000

tgagatgtat ggatcagtat actccgtgaa tcctttggac cttccagcca taattgagag 9060

gttacacggg cttgacgcct tttctatgca cacatactct caccacgaac tgacgcgggt 9120

ggcttcagcc ctcagaaaac ttggggcgcc acccctcagg gtgtggaaga gtcgggctcg 9180

cgcagtcagg gcgtccctca tctcccgtgg agggaaagcg gccgtttgcg gccgatatct 9240

cttcaattgg gcggtgaaga ccaagctcaa actcactcca ttgccggagg cgcgcctact 9300

ggacttatcc agttggttca ccgtcggcgc cggcgggggc gacatttttc acagcgtgtc 9360

gcgcgcccga ccccgctcat tactcttcgg cctactccta cttttcgtag gggtaggcct 9420

cttcctactc cccgctcggt agagcggcac acactaggta cactccatag ctaactgttc 9480

cttttttttt tttttttttt tttttttttt tttttttttt ttttcttttt tttttttttc 9540

cctctttctt cccttctcat cttattctac tttctttctt ggtggctcca tcttagccct 9600

agtcacggct agctgtgaaa ggtccgtgag ccgcatgact gcagagagtg ccgtaactgg 9660

tctctctgca gatcatgtct aga 9683

<210> 11
<211> 9695
<212> DNA
<213> Artificial

<220>
<223> pJ6/JFH-1 (1XFLAG)

<400> 11
acccgcccct aataggggcg acactccgcc atgaatcact cccctgtgag gaactactgt 60

cttcacgcag aaagcgtcta gccatggcgt tagtatgagt gtcgtacagc ctccaggccc 120

ccccctcccg ggagagccat agtggtctgc ggaaccggtg agtacaccgg aattgccggg 180

aagactgggt cctttcttgg ataaacccac tctatgcccg gccatttggg cgtgcccccg 240

caagactgct agccgagtag cgttgggttg cgaaaggcct tgtggtactg cctgataggg 300

tgcttgcgag tgccccggga ggtctcgtag accgtgcacc atgagcacaa atcctaaacc 360

tcaaagaaaa accaaaagaa acaccaaccg tcgcccacaa gacgttaagt ttccgggcgg 420

cggccagatc gttggcggag tatacttgtt gccgcgcagg ggccccaggt tgggtgtgcg 480

cgcgacaagg aagacttcgg agcggtccca gccacgtgga aggcgccagc ccatccctaa 540

agatcggcgc tccactggca aatcctgggg aaaaccagga tacccctggc ccctatacgg 600

gaatgaggga ctcggctggg caggatggct cctgtccccc cgaggttccc gtccctcttg 660

gggccccaat gacccccggc ataggtcgcg caacgtgggt aaggtcatcg ataccctaac 720

gtgcggcttt gccgacctca tggggtacat ccctgtcgtg ggcgccccgc tcggcggcgt 780

cgccagagct ctcgcgcatg gcgtgagagt cctggaggac ggggttaatt ttgcaacagg 840

gaacttaccc ggttgctcct tttctatctt cttgctggcc ctgctgtcct gcatcaccac 900

cccggtctcc gctgccgaag tgaagaacat cagtaccggc tacatggtga ctaacgactg 960

caccaatgac agcattacct ggcagctcca ggctgctgtc ctccacgtcc ccgggtgcgt 1020

cccgtgcgag aaagtgggga atgcatctca gtgctggata ccggtctcac cgaatgtggc 1080

cgtgcagcgg cccggcgccc tcacgcaggg cttgcggacg cacatcgaca tggttgtgat 1140

gtccgccacg ctctgctctg ccctctacgt gggggacctc tgcggtgggg tgatgctcgc 1200

agcccaaatg ttcattgtct cgccgcagca ccactggttt gtccaagact gcaattgctc 1260

catctaccct ggtaccatca ctggacaccg catggcatgg gacatgatga tgaactggtc 1320

gcccacggct accatgatct tggcgtacgc gatgcgtgtc cccgaggtca ttatagacat 1380

cattagcggg gctcattggg gcgtcatgtt cggcttggcc tacttctcta tgcagggagc 1440

gtgggcgaaa gtcgttgtca tccttctgtt ggccgccggg gtggacgcgc gcacccatac 1500

tgttgggggt tctgccgcgg attacaagga tgacgatgac aagggaggcg gtagcttatt 1560

tgacatgggc cccaggcaga aaatccagct cgttaacacc aatggcagct ggcacatcaa 1620

ccgcaccgcc ctgaactgca atgactcctt gcacaccggc tttatcgcgt ctctgttcta 1680

cacccacagc ttcaactcgt caggatgtcc cgaacgcatg tccgcctgcc gcagtatcga 1740

ggccttccgg gtgggatggg gcgccttgca atatgaggat aatgtcacca atccagagga 1800

tatgagaccc tattgctggc actacccacc aaggcagtgt ggcgtggtct ccgcgaagac 1860

tgtgtgtggc ccagtgtact gtttcacccc cagcccagtg gtagtgggca cgaccgacag 1920

gcttggagcg cccacttaca cgtgggggga gaatgagaca gatgtcttcc tattgaacag 1980

cactcgacca ccgctggggt catggttcgg ctgcacgtgg atgaactctt ctggctacac 2040

caagacttgc ggcgcaccac cctgccgtac tagagctgac ttcaacgcca gcacggacct 2100

gttgtgcccc acggactgtt ttaggaagca tcctgatacc acttacctca aatgcggctc 2160

tgggccctgg ctcacgccaa ggtgcctgat cgactacccc tacaggctct ggcattaccc 2220

ctgcacagtt aactatacca tcttcaaaat aaggatgtat gtgggagggg ttgagcacag 2280

gctcacggct gcatgcaatt tcactcgtgg ggatcgttgc aacttggagg acagagacag 2340

aagtcaactg tctcctttgt tgcactccac cacggaatgg gccattttac cttgctctta 2400

ctcggacctg cccgccttgt cgactggtct tctccacctc caccaaaaca tcgtggacgt 2460

acaattcatg tatggcctat cacctgccct cacaaaatac atcgtccgat gggagtgggt 2520

aatactctta ttcctgctct tagcggacgc cagggtttgc gcctgcttat ggatgctcat 2580

cttgttgggc caggccgaag cagcactaga gaagctggtc atcttgcacg ctgcgagcgc 2640

agctagctgc aatggcttcc tatattttgt catctttttc gtggctgctt ggtacatcaa 2700

gggtcgggta gtccccttag ctacctattc cctcactggc ctgtggtcct ttagcctact 2760

gctcctagca ttgccccaac aggcttatgc ttatgacgca tctgtgcatg gccagatagg 2820

agcggctctg ctggtaatga tcaccctctt cacactcacc ccggggtata agaccctcct 2880

cggccagtgt ctgtggtggt tgtgctatct cctgaccctg ggggaagcca tgattcagga 2940

gtgggtacca cccatgcagg tgcgcggcgg ccgcgatggc atcgcgtggg ccgtcactat 3000

attctgcccg ggtgtggtgt ttgacattac caaatggctt ttggcgttgc ttgggcctgc 3060

ttacctctta agggccgctt tgacacatgt gccgtacttc gtcagagctc acgctctgat 3120

aagggtatgc gctttggtga agcagctcgc ggggggtagg tatgttcagg tggcgctatt 3180

ggcccttggc aggtggactg gcacctacat ctatgaccac ctcacaccta tgtcggactg 3240

ggccgctagc ggcctgcgcg acttagcggt cgccgtggaa cccatcatct tcagtccgat 3300

ggagaagaag gtcatcgtct ggggagcgga gacggctgca tgtggggaca ttctacatgg 3360

acttcccgtg tccgcccgac tcggccagga gatcctcctc ggcccagctg atggctacac 3420

ctccaagggg tggaagctcc ttgctcccat cactgcttat gcccagcaaa cacgaggcct 3480

cctgggcgcc atagtggtga gtatgacggg gcgtgacagg acagaacagg ccggggaagt 3540

ccaaatcctg tccacagtct ctcagtcctt cctcggaaca accatctcgg gggttttgtg 3600

gactgtttac cacggagctg gcaacaagac tctagccggc ttacggggtc cggtcacgca 3660

gatgtactcg agtgctgagg gggacttggt aggctggccc agcccccctg ggaccaagtc 3720

tttggagccg tgcaagtgtg gagccgtcga cctatatctg gtcacgcgga acgctgatgt 3780

catcccggct cggagacgcg gggacaagcg gggagcattg ctctccccga gacccatttc 3840

gaccttgaag gggtcctcgg gggggccggt gctctgccct aggggccacg tcgttgggct 3900

cttccgagca gctgtgtgct ctcggggcgt ggccaaatcc atcgatttca tccccgttga 3960

gacactcgac gttgttacaa ggtctcccac tttcagtgac aacagcacgc caccggctgt 4020

gccccagacc tatcaggtcg ggtacttgca tgctccaact ggcagtggaa agagcaccaa 4080

ggtccctgtc gcgtatgccg cccaggggta caaagtacta gtgcttaacc cctcggtagc 4140

tgccaccctg ggtttggggg cgtacctatc caaggcacat ggcatcaatc ccaacattag 4200

gactggagtc aggaccgtga tgaccgggga ggccatcacg tactccacat atggcaaatt 4260

tctcgccgat gggggctgcg ctagcggcgc ctatgacatc atcatatgcg atgaatgcca 4320

cgctgtggat gctacctcca ttctcggcat cggaacggtc cttgatcaag cagagacagc 4380

cggggtcaga ctaactgtgc tggctacggc cacacccccc gggtcagtga caacccccca 4440

tcccgatata gaagaggtag gcctcgggcg ggagggtgag atccccttct atgggagggc 4500

gattccccta tcctgcatca agggagggag acacctgatt ttctgccact caaagaaaaa 4560

gtgtgacgag ctcgcggcgg cccttcgggg catgggcttg aatgccgtgg catactatag 4620

agggttggac gtctccataa taccagctca gggagatgtg gtggtcgtcg ccaccgacgc 4680

cctcatgacg gggtacactg gagactttga ctccgtgatc gactgcaatg tagcggtcac 4740

ccaagctgtc gacttcagcc tggaccccac cttcactata accacacaga ctgtcccaca 4800

agacgctgtc tcacgcagtc agcgccgcgg gcgcacaggt agaggaagac agggcactta 4860

taggtatgtt tccactggtg aacgagcctc aggaatgttt gacagtgtag tgctttgtga 4920

gtgctacgac gcaggggctg cgtggtacga tctcacacca gcggagacca ccgtcaggct 4980

tagagcgtat ttcaacacgc ccggcctacc cgtgtgtcaa gaccatcttg aattttggga 5040

ggcagttttc accggcctca cacacataga cgcccacttc ctctcccaaa caaagcaagc 5100

gggggagaac ttcgcgtacc tagtagccta ccaagctacg gtgtgcgcca gagccaaggc 5160

ccctcccccg tcctgggacg ccatgtggaa gtgcctggcc cgactcaagc ctacgcttgc 5220

gggccccaca cctctcctgt accgtttggg ccctattacc aatgaggtca ccctcacaca 5280

ccctgggacg aagtacatcg ccacatgcat gcaagctgac cttgaggtca tgaccagcac 5340

gtgggtccta gctggaggag tcctggcagc cgtcgccgca tattgcctgg cgactggatg 5400

cgtttccatc atcggccgct tgcacgtcaa ccagcgagtc gtcgttgcgc cggataagga 5460

ggtcctgtat gaggcttttg atgagatgga ggaatgcgcc tctagggcgg ctctcatcga 5520

agaggggcag cggatagccg agatgttgaa gtccaagatc caaggcttgc tgcagcaggc 5580

ctctaagcag gcccaggaca tacaacccgc tatgcaggct tcatggccca aagtggaaca 5640

attttgggcc agacacatgt ggaacttcat tagcggcatc caatacctcg caggattgtc 5700

aacactgcca gggaaccccg cggtggcttc catgatggca ttcagtgccg ccctcaccag 5760

tccgttgtcg accagtacca ccatccttct caacatcatg ggaggctggt tagcgtccca 5820

gatcgcacca cccgcggggg ccaccggctt tgtcgtcagt ggcctggtgg gggctgccgt 5880

gggcagcata ggcctgggta aggtgctggt ggacatcctg gcaggatatg gtgcgggcat 5940

ttcgggggcc ctcgtcgcat tcaagatcat gtctggcgag aagccctcta tggaagatgt 6000

catcaatcta ctgcctggga tcctgtctcc gggagccctg gtggtggggg tcatctgcgc 6060

ggccattctg cgccgccacg tgggaccggg ggagggcgcg gtccaatgga tgaacaggct 6120

tattgccttt gcttccagag gaaaccacgt cgcccctact cactacgtga cggagtcgga 6180

tgcgtcgcag cgtgtgaccc aactacttgg ctctcttact ataaccagcc tactcagaag 6240

actccacaat tggataactg aggactgccc catcccatgc tccggatcct ggctccgcga 6300

cgtgtgggac tgggtttgca ccatcttgac agacttcaaa aattggctga cctctaaatt 6360

gttccccaag ctgcccggcc tccccttcat ctcttgtcaa aaggggtaca agggtgtgtg 6420

ggccggcact ggcatcatga ccacgcgctg cccttgcggc gccaacatct ctggcaatgt 6480

ccgcctgggc tctatgagga tcacagggcc taaaacctgc atgaacacct ggcaggggac 6540

ctttcctatc aattgctaca cggagggcca gtgcgcgccg aaacccccca cgaactacaa 6600

gaccgccatc tggagggtgg cggcctcgga gtacgcggag gtgacgcagc atgggtcgta 6660

ctcctatgta acaggactga ccactgacaa tctgaaaatt ccttgccaac taccttctcc 6720

agagtttttc tcctgggtgg acggtgtgca gatccatagg tttgcaccca caccaaagcc 6780

gtttttccgg gatgaggtct cgttctgcgt tgggcttaat tcctatgctg tcgggtccca 6840

gcttccctgt gaacctgagc ccgacgcaga cgtattgagg tccatgctaa cagatccgcc 6900

ccacatcacg gcggagactg cggcgcggcg cttggcacgg ggatcacctc catctgaggc 6960

gagctcctca gtgagccagc tatcagcacc gtcgctgcgg gccacctgca ccacccacag 7020

caacacctat gacgtggaca tggtcgatgc caacctgctc atggagggcg gtgtggctca 7080

gacagagcct gagtccaggg tgcccgttct ggactttctc gagccaatgg ccgaggaaga 7140

gagcgacctt gagccctcaa taccatcgga gtgcatgctc cccaggagcg ggtttccacg 7200

ggccttaccg gcttgggcac ggcctgacta caacccgccg ctcgtggaat cgtggaggag 7260

gccagattac caaccgccca ccgttgctgg ttgtgctctc ccccccccca agaaggcccc 7320

gacgcctccc ccaaggagac gccggacagt gggtctgagc gagagcacca tatcagaagc 7380

cctccagcaa ctggccatca agacctttgg ccagcccccc tcgagcggtg atgcaggctc 7440

gtccacgggg gcgggcgccg ccgaatccgg cggtccgacg tcccctggtg agccggcccc 7500

ctcagagaca ggttccgcct cctctatgcc ccccctcgag ggggagcctg gagatccgga 7560

cctggagtct gatcaggtag agcttcaacc tcccccccag gggggggggg tagctcccgg 7620

ttcgggctcg gggtcttggt ctacttgctc cgaggaggac gataccaccg tgtgctgctc 7680

catgtcatac tcctggaccg gggctctaat aactccctgt agccccgaag aggaaaagtt 7740

gccaatcaac cctttgagta actcgctgtt gcgataccat aacaaggtgt actgtacaac 7800

atcaaagagc gcctcacaga gggctaaaaa ggtaactttt gacaggacgc aagtgctcga 7860

cgcccattat gactcagtct taaaggacat caagctagcg gcttccaagg tcagcgcaag 7920

gctcctcacc ttggaggagg cgtgccagtt gactccaccc cattctgcaa gatccaagta 7980

tggattcggg gccaaggagg tccgcagctt gtccgggagg gccgttaacc acatcaagtc 8040

cgtgtggaag gacctcctgg aagacccaca aacaccaatt cccacaacca tcatggccaa 8100

aaatgaggtg ttctgcgtgg accccgccaa ggggggtaag aaaccagctc gcctcatcgt 8160

ttaccctgac ctcggcgtcc gggtctgcga gaaaatggcc ctctatgaca ttacacaaaa 8220

gcttcctcag gcggtaatgg gagcttccta tggcttccag tactcccctg cccaacgggt 8280

ggagtatctc ttgaaagcat gggcggaaaa gaaggacccc atgggttttt cgtatgatac 8340

ccgatgcttc gactcaaccg tcactgagag agacatcagg accgaggagt ccatatacca 8400

ggcctgctcc ctgcccgagg aggcccgcac tgccatacac tcgctgactg agagacttta 8460

cgtaggaggg cccatgttca acagcaaggg tcaaacctgc ggttacagac gttgccgcgc 8520

cagcggggtg ctaaccacta gcatgggtaa caccatcaca tgctatgtga aagccctagc 8580

ggcctgcaag gctgcgggga tagttgcgcc cacaatgctg gtatgcggcg atgacctagt 8640

agtcatctca gaaagccagg ggactgagga ggacgagcgg aacctgagag ccttcacgga 8700

ggccatgacc aggtactctg cccctcctgg tgatcccccc agaccggaat atgacctgga 8760

gctaataaca tcctgttcct caaatgtgtc tgtggcgttg ggcccgcggg gccgccgcag 8820

atactacctg accagagacc caaccactcc actcgcccgg gctgcctggg aaacagttag 8880

acactcccct atcaattcat ggctgggaaa catcatccag tatgctccaa ccatatgggt 8940

tcgcatggtc ctaatgacac acttcttctc cattctcatg gtccaagaca ccctggacca 9000

gaacctcaac tttgagatgt atggatcagt atactccgtg aatcctttgg accttccagc 9060

cataattgag aggttacacg ggcttgacgc cttttctatg cacacatact ctcaccacga 9120

actgacgcgg gtggcttcag ccctcagaaa acttggggcg ccacccctca gggtgtggaa 9180

gagtcgggct cgcgcagtca gggcgtccct catctcccgt ggagggaaag cggccgtttg 9240

cggccgatat ctcttcaatt gggcggtgaa gaccaagctc aaactcactc cattgccgga 9300

ggcgcgccta ctggacttat ccagttggtt caccgtcggc gccggcgggg gcgacatttt 9360

tcacagcgtg tcgcgcgccc gaccccgctc attactcttc ggcctactcc tacttttcgt 9420

aggggtaggc ctcttcctac tccccgctcg gtagagcggc acacactagg tacactccat 9480

agctaactgt tccttttttt tttttttttt tttttttttt tttttttttt ttttttcttt 9540

tttttttttt tccctctttc ttcccttctc atcttattct actttctttc ttggtggctc 9600

catcttagcc ctagtcacgg ctagctgtga aaggtccgtg agccgcatga ctgcagagag 9660

tgccgtaact ggtctctctg cagatcatgt ctaga 9695

<210> 12
<211> 9737
<212> DNA
<213> Artificial

<220>
<223> pJ6/JFH-1 (3XFLAG)

<400> 12
acccgcccct aataggggcg acactccgcc atgaatcact cccctgtgag gaactactgt 60

cttcacgcag aaagcgtcta gccatggcgt tagtatgagt gtcgtacagc ctccaggccc 120

ccccctcccg ggagagccat agtggtctgc ggaaccggtg agtacaccgg aattgccggg 180

aagactgggt cctttcttgg ataaacccac tctatgcccg gccatttggg cgtgcccccg 240

caagactgct agccgagtag cgttgggttg cgaaaggcct tgtggtactg cctgataggg 300

tgcttgcgag tgccccggga ggtctcgtag accgtgcacc atgagcacaa atcctaaacc 360

tcaaagaaaa accaaaagaa acaccaaccg tcgcccacaa gacgttaagt ttccgggcgg 420

cggccagatc gttggcggag tatacttgtt gccgcgcagg ggccccaggt tgggtgtgcg 480

cgcgacaagg aagacttcgg agcggtccca gccacgtgga aggcgccagc ccatccctaa 540

agatcggcgc tccactggca aatcctgggg aaaaccagga tacccctggc ccctatacgg 600

gaatgaggga ctcggctggg caggatggct cctgtccccc cgaggttccc gtccctcttg 660

gggccccaat gacccccggc ataggtcgcg caacgtgggt aaggtcatcg ataccctaac 720

gtgcggcttt gccgacctca tggggtacat ccctgtcgtg ggcgccccgc tcggcggcgt 780

cgccagagct ctcgcgcatg gcgtgagagt cctggaggac ggggttaatt ttgcaacagg 840

gaacttaccc ggttgctcct tttctatctt cttgctggcc ctgctgtcct gcatcaccac 900

cccggtctcc gctgccgaag tgaagaacat cagtaccggc tacatggtga ctaacgactg 960

caccaatgac agcattacct ggcagctcca ggctgctgtc ctccacgtcc ccgggtgcgt 1020

cccgtgcgag aaagtgggga atgcatctca gtgctggata ccggtctcac cgaatgtggc 1080

cgtgcagcgg cccggcgccc tcacgcaggg cttgcggacg cacatcgaca tggttgtgat 1140

gtccgccacg ctctgctctg ccctctacgt gggggacctc tgcggtgggg tgatgctcgc 1200

agcccaaatg ttcattgtct cgccgcagca ccactggttt gtccaagact gcaattgctc 1260

catctaccct ggtaccatca ctggacaccg catggcatgg gacatgatga tgaactggtc 1320

gcccacggct accatgatct tggcgtacgc gatgcgtgtc cccgaggtca ttatagacat 1380

cattagcggg gctcattggg gcgtcatgtt cggcttggcc tacttctcta tgcagggagc 1440

gtgggcgaaa gtcgttgtca tccttctgtt ggccgccggg gtggacgcgc gcacccatac 1500

tgttgggggt tctgccgcgg actacaaaga ccatgacggt gattataaag atcatgacat 1560

cgattacaag gatgacgatg acaagggagg cggtagctta tttgacatgg gccccaggca 1620

gaaaatccag ctcgttaaca ccaatggcag ctggcacatc aaccgcaccg ccctgaactg 1680

caatgactcc ttgcacaccg gctttatcgc gtctctgttc tacacccaca gcttcaactc 1740

gtcaggatgt cccgaacgca tgtccgcctg ccgcagtatc gaggccttcc gggtgggatg 1800

gggcgccttg caatatgagg ataatgtcac caatccagag gatatgagac cctattgctg 1860

gcactaccca ccaaggcagt gtggcgtggt ctccgcgaag actgtgtgtg gcccagtgta 1920

ctgtttcacc cccagcccag tggtagtggg cacgaccgac aggcttggag cgcccactta 1980

cacgtggggg gagaatgaga cagatgtctt cctattgaac agcactcgac caccgctggg 2040

gtcatggttc ggctgcacgt ggatgaactc ttctggctac accaagactt gcggcgcacc 2100

accctgccgt actagagctg acttcaacgc cagcacggac ctgttgtgcc ccacggactg 2160

ttttaggaag catcctgata ccacttacct caaatgcggc tctgggccct ggctcacgcc 2220

aaggtgcctg atcgactacc cctacaggct ctggcattac ccctgcacag ttaactatac 2280

catcttcaaa ataaggatgt atgtgggagg ggttgagcac aggctcacgg ctgcatgcaa 2340

tttcactcgt ggggatcgtt gcaacttgga ggacagagac agaagtcaac tgtctccttt 2400

gttgcactcc accacggaat gggccatttt accttgctct tactcggacc tgcccgcctt 2460

gtcgactggt cttctccacc tccaccaaaa catcgtggac gtacaattca tgtatggcct 2520

atcacctgcc ctcacaaaat acatcgtccg atgggagtgg gtaatactct tattcctgct 2580

cttagcggac gccagggttt gcgcctgctt atggatgctc atcttgttgg gccaggccga 2640

agcagcacta gagaagctgg tcatcttgca cgctgcgagc gcagctagct gcaatggctt 2700

cctatatttt gtcatctttt tcgtggctgc ttggtacatc aagggtcggg tagtcccctt 2760

agctacctat tccctcactg gcctgtggtc ctttagccta ctgctcctag cattgcccca 2820

acaggcttat gcttatgacg catctgtgca tggccagata ggagcggctc tgctggtaat 2880

gatcaccctc ttcacactca ccccggggta taagaccctc ctcggccagt gtctgtggtg 2940

gttgtgctat ctcctgaccc tgggggaagc catgattcag gagtgggtac cacccatgca 3000

ggtgcgcggc ggccgcgatg gcatcgcgtg ggccgtcact atattctgcc cgggtgtggt 3060

gtttgacatt accaaatggc ttttggcgtt gcttgggcct gcttacctct taagggccgc 3120

tttgacacat gtgccgtact tcgtcagagc tcacgctctg ataagggtat gcgctttggt 3180

gaagcagctc gcggggggta ggtatgttca ggtggcgcta ttggcccttg gcaggtggac 3240

tggcacctac atctatgacc acctcacacc tatgtcggac tgggccgcta gcggcctgcg 3300

cgacttagcg gtcgccgtgg aacccatcat cttcagtccg atggagaaga aggtcatcgt 3360

ctggggagcg gagacggctg catgtgggga cattctacat ggacttcccg tgtccgcccg 3420

actcggccag gagatcctcc tcggcccagc tgatggctac acctccaagg ggtggaagct 3480

ccttgctccc atcactgctt atgcccagca aacacgaggc ctcctgggcg ccatagtggt 3540

gagtatgacg gggcgtgaca ggacagaaca ggccggggaa gtccaaatcc tgtccacagt 3600

ctctcagtcc ttcctcggaa caaccatctc ggggggtttg tggactgttt accacggagc 3660

tggcaacaag actctagccg gcttacgggg tccggtcacg cagatgtact cgagtgctga 3720

gggggacttg gtaggctggc ccagcccccc tgggaccaag tctttggagc cgtgcaagtg 3780

tggagccgtc gacctatatc tggtcacgcg gaacgctgat gtcatcccgg ctcggagacg 3840

cggggacaag cggggagcat tgctctcccc gagacccatt tcgaccttga aggggtcctc 3900

gggggggccg gtgctctgcc ctaggggcca cgtcgttggg ctcttccgag cagctgtgtg 3960

ctctcggggc gtggccaaat ccatcgattt catccccgtt gagacactcg acgttgttac 4020

aaggtctccc actttcagtg acaacagcac gccaccggct gtgccccaga cctatcaggt 4080

cgggtacttg catgctccaa ctggcagtgg aaagagcacc aaggtccctg tcgcgtatgc 4140

cgcccagggg tacaaagtac tagtgcttaa cccctcggta gctgccaccc tggggtttgg 4200

ggcgtaccta tccaaggcac atggcatcaa tcccaacatt aggactggag tcaggaccgt 4260

gatgaccggg gaggccatca cgtactccac atatggcaaa tttctcgccg atgggggctg 4320

cgctagcggc gcctatgaca tcatcatatg cgatgaatgc cacgctgtgg atgctacctc 4380

cattctcggc atcggaacgg tccttgatca agcagagaca gccggggtca gactaactgt 4440

gctggctacg gccacacccc ccgggtcagt gacaaccccc catcccgata tagaagaggt 4500

aggcctcggg cgggagggtg agatcccctt ctatgggagg gcgattcccc tatcctgcat 4560

caagggaggg agacacctga ttttctgcca ctcaaagaaa aagtgtgacg agctcgcggc 4620

ggcccttcgg ggcatgggct tgaatgccgt ggcatactat agagggttgg acgtctccat 4680

aataccagct cagggagatg tggtggtcgt cgccaccgac gccctcatga cggggtacac 4740

tggagacttt gactccgtga tcgactgcaa tgtagcggtc acccaagctg tcgacttcag 4800

cctggacccc accttcacta taaccacaca gactgtccca caagacgctg tctcacgcag 4860

tcagcgccgc gggcgcacag gtagaggaag acagggcact tataggtatg tttccactgg 4920

tgaacgagcc tcaggaatgt ttgacagtgt agtgctttgt gagtgctacg acgcaggggc 4980

tgcgtggtac gatctcacac cagcggagac caccgtcagg cttagagcgt atttcaacac 5040

gcccggccta cccgtgtgtc aagaccatct tgaattttgg gaggcagttt tcaccggcct 5100

cacacacata gacgcccact tcctctccca aacaaagcaa gcgggggaga acttcgcgta 5160

cctagtagcc taccaagcta cggtgtgcgc cagagccaag gcccctcccc cgtcctggga 5220

cgccatgtgg aagtgcctgg cccgactcaa gcctacgctt gcgggcccca cacctctcct 5280

gtaccgtttg ggccctatta ccaatgaggt caccctcaca caccctggga cgaagtacat 5340

cgccacatgc atgcaagctg accttgaggt catgaccagc acgtgggtcc tagctggagg 5400

agtcctggca gccgtcgccg catattgcct ggcgactgga tgcgtttcca tcatcggccg 5460

cttgcacgtc aaccagcgag tcgtcgttgc gccggataag gaggtcctgt atgaggcttt 5520

tgatgagatg gaggaatgcg cctctagggc ggctctcatc gaagaggggc agcggatagc 5580

cgagatgttg aagtccaaga tccaaggctt gctgcagcag gcctctaagc aggcccagga 5640

catacaaccc gctatgcagg cttcatggcc caaagtggaa caattttggg ccagacacat 5700

gtggaacttc attagcggca tccaatacct cgcaggattg tcaacactgc cagggaaccc 5760

cgcggtggct tccatgatgg cattcagtgc cgccctcacc agtccgttgt cgaccagtac 5820

caccatcctt ctcaacatca tgggaggctg gttagcgtcc cagatcgcac cacccgcggg 5880

ggccaccggc tttgtcgtca gtggcctggt gggggctgcc gtgggcagca taggcctggg 5940

taaggtgctg gtggacatcc tggcaggata tggtgcgggc atttcggggg ccctcgtcgc 6000

attcaagatc atgtctggcg agaagccctc tatggaagat gtcatcaatc tactgcctgg 6060

gatcctgtct ccgggagccc tggtggtggg ggtcatctgc gcggccattc tgcgccgcca 6120

cgtgggaccg ggggagggcg cggtccaatg gatgaacagg cttattgcct ttgcttccag 6180

aggaaaccac gtcgccccta ctcactacgt gacggagtcg gatgcgtcgc agcgtgtgac 6240

ccaactactt ggctctctta ctataaccag cctactcaga agactccaca attggataac 6300

tgaggactgc cccatcccat gctccggatc ctggctccgc gacgtgtggg actgggtttg 6360

caccatcttg acagacttca aaaattggct gacctctaaa ttgttcccca agctgcccgg 6420

cctccccttc atctcttgtc aaaaggggta caagggtgtg tgggccggca ctggcatcat 6480

gaccacgcgc tgcccttgcg gcgccaacat ctctggcaat gtccgcctgg gctctatgag 6540

gatcacaggg cctaaaacct gcatgaacac ctggcagggg acctttccta tcaattgcta 6600

cacggagggc cagtgcgcgc cgaaaccccc cacgaactac aagaccgcca tctggagggt 6660

ggcggcctcg gagtacgcgg aggtgacgca gcatgggtcg tactcctatg taacaggact 6720

gaccactgac aatctgaaaa ttccttgcca actaccttct ccagagtttt tctcctgggt 6780

ggacggtgtg cagatccata ggtttgcacc cacaccaaag ccgtttttcc gggatgaggt 6840

ctcgttctgc gttgggctta attcctatgc tgtcgggtcc cagcttccct gtgaacctga 6900

gcccgacgca gacgtattga ggtccatgct aacagatccg ccccacatca cggcggagac 6960

tgcggcgcgg cgcttggcac ggggatcacc tccatctgag gcgagctcct cagtgagcca 7020

gctatcagca ccgtcgctgc gggccacctg caccacccac agcaacacct atgacgtgga 7080

catggtcgat gccaacctgc tcatggaggg cggtgtggct cagacagagc ctgagtccag 7140

ggtgcccgtt ctggactttc tcgagccaat ggccgaggaa gagagcgacc ttgagccctc 7200

aataccatcg gagtgcatgc tccccaggag cgggtttcca cgggccttac cggcttgggc 7260

acggcctgac tacaacccgc cgctcgtgga atcgtggagg aggccagatt accaaccgcc 7320

caccgttgct ggttgtgctc tccccccccc caagaaggcc ccgacgcctc ccccaaggag 7380

acgccggaca gtgggtctga gcgagagcac catatcagaa gccctccagc aactggccat 7440

caagaccttt ggccagcccc cctcgagcgg tgatgcaggc tcgtccacgg gggcgggcgc 7500

cgccgaatcc ggcggtccga cgtcccctgg tgagccggcc ccctcagaga caggttccgc 7560

ctcctctatg ccccccctcg agggggagcc tggagatccg gacctggagt ctgatcaggt 7620

agagcttcaa cctccccccc aggggggggg ggtagctccc ggttcgggct cggggtcttg 7680

gtctacttgc tccgaggagg acgataccac cgtgtgctgc tccatgtcat actcctggac 7740

cggggctcta ataactccct gtagccccga agaggaaaag ttgccaatca accctttgag 7800

taactcgctg ttgcgatacc ataacaaggt gtactgtaca acatcaaaga gcgcctcaca 7860

gagggctaaa aaggtaactt ttgacaggac gcaagtgctc gacgcccatt atgactcagt 7920

cttaaaggac atcaagctag cggcttccaa ggtcagcgca aggctcctca ccttggagga 7980

ggcgtgccag ttgactccac cccattctgc aagatccaag tatggattcg gggccaagga 8040

ggtccgcagc ttgtccggga gggccgttaa ccacatcaag tccgtgtgga aggacctcct 8100

ggaagaccca caaacaccaa ttcccacaac catcatggcc aaaaatgagg tgttctgcgt 8160

ggaccccgcc aaggggggta agaaaccagc tcgcctcatc gtttaccctg acctcggcgt 8220

ccgggtctgc gagaaaatgg ccctctatga cattacacaa aagcttcctc aggcggtaat 8280

gggagcttcc tatggcttcc agtactcccc tgcccaacgg gtggagtatc tcttgaaagc 8340

atgggcggaa aagaaggacc ccatgggttt ttcgtatgat acccgatgct tcgactcaac 8400

cgtcactgag agagacatca ggaccgagga gtccatatac caggcctgct ccctgcccga 8460

ggaggcccgc actgccatac actcgctgac tgagagactt tacgtaggag ggcccatgtt 8520

caacagcaag ggtcaaacct gcggttacag acgttgccgc gccagcgggg tgctaaccac 8580

tagcatgggt aacaccatca catgctatgt gaaagcccta gcggcctgca aggctgcggg 8640

gatagttgcg cccacaatgc tggtatgcgg cgatgaccta gtagtcatct cagaaagcca 8700

ggggactgag gaggacgagc ggaacctgag agccttcacg gaggccatga ccaggtactc 8760

tgcccctcct ggtgatcccc ccagaccgga atatgacctg gagctaataa catcctgttc 8820

ctcaaatgtg tctgtggcgt tgggcccgcg gggccgccgc agatactacc tgaccagaga 8880

cccaaccact ccactcgccc gggctgcctg ggaaacagtt agacactccc ctatcaattc 8940

atggctggga aacatcatcc agtatgctcc aaccatatgg gttcgcatgg tcctaatgac 9000

acacttcttc tccattctca tggtccaaga caccctggac cagaacctca actttgagat 9060

gtatggatca gtatactccg tgaatccttt ggaccttcca gccataattg agaggttaca 9120

cgggcttgac gccttttcta tgcacacata ctctcaccac gaactgacgc gggtggcttc 9180

agccctcaga aaacttgggg cgccacccct cagggtgtgg aagagtcggg ctcgcgcagt 9240

cagggcgtcc ctcatctccc gtggagggaa agcggccgtt tgcggccgat atctcttcaa 9300

ttgggcggtg aagaccaagc tcaaactcac tccattgccg gaggcgcgcc tactggactt 9360

atccagttgg ttcaccgtcg gcgccggcgg gggcgacatt tttcacagcg tgtcgcgcgc 9420

ccgaccccgc tcattactct tcggcctact cctacttttc gtaggggtag gcctcttcct 9480

actccccgct cggtagagcg gcacacacta ggtacactcc atagctaact gttccttttt 9540

tttttttttt tttttttttt tttttttttt ttttttttct tttttttttt tttccctctt 9600

tcttcccttc tcatcttatt ctactttctt tcttggtggc tccatcttag ccctagtcac 9660

ggctagctgt gaaaggtccg tgagccgcat gactgcagag agtgccgtaa ctggtctctc 9720

tgcagatcat gtctaga 9737

<210> 13
<211> 17
<212> DNA
<213> Artificial

<220>
<223> primer (S-17)

<400> 13
cgggagagcc atagtgg 17

<210> 14
<211> 19
<212> DNA
<213> Artificial

<220>
<223> primer (R-19)

<400> 14
agtaccacaa ggcctttcg 19

<210> 15
<211> 20
<212> DNA
<213> Artificial

<220>
<223> probe

<400> 15
ctgcggaacc gtgagtacac 20

<210> 16
<211> 18
<212> DNA
<213> Artificial

<220>
<223> primer (1141S-2a)

<400> 16
tgtccgccac gctctgct 18

<210> 17
<211> 21
<212> DNA
<213> Artificial

<220>
<223> primer (J6E2-K-R)

<400> 17
atctgtctct ttctcccccc a 21

<210> 18
<211> 21
<212> DNA
<213> Artificial

<220>
<223> primer (J6E2-K-F)

<400> 18
tggggggaga aagagacaga t 21

<210> 19
<211> 18
<212> DNA
<213> Artificial

<220>
<223> primer (3189R-1H)

<400> 19
ccagtccacc tgccaagg 18

<210> 20
<211> 9695
<212> DNA
<213> Artificial

<220>
<223> pJ6/JFH-1 (1XFLAG) N->K

<400> 20
   acccgcccct aataggggcg acactccgcc atgaatcact cccctgtgag gaactactgt 60
   cttcacgcag aaagcgtcta gccatggcgt tagtatgagt gtcgtacagc ctccaggccc 120
   ccccctcccg ggagagccat agtggtctgc ggaaccggtg agtacaccgg aattgccggg 180
   aagactgggt cctttcttgg ataaacccac tctatgcccg gccatttggg cgtgcccccg 240
   caagactgct agccgagtag cgttgggttg cgaaaggcct tgtggtactg cctgataggg 300
   tgcttgcgag tgccccggga ggtctcgtag accgtgcacc atgagcacaa atcctaaacc 360
   tcaaagaaaa accaaaagaa acaccaaccg tcgcccacaa gacgttaagt ttccgggcgg 420
   cggccagatc gttggcggag tatacttgtt gccgcgcagg ggccccaggt tgggtgtgcg 480
   cgcgacaagg aagacttcgg agcggtccca gccacgtgga aggcgccagc ccatccctaa 540
   agatcggcgc tccactggca aatcctgggg aaaaccagga tacccctggc ccctatacgg 600
   gaatgaggga ctcggctggg caggatggct cctgtccccc cgaggttccc gtccctcttg 660
   gggccccaat gacccccggc ataggtcgcg caacgtgggt aaggtcatcg ataccctaac 720
   gtgcggcttt gccgacctca tggggtacat ccctgtcgtg ggcgccccgc tcggcggcgt 780
   cgccagagct ctcgcgcatg gcgtgagagt cctggaggac ggggttaatt ttgcaacagg 840
   gaacttaccc ggttgctcct tttctatctt cttgctggcc ctgctgtcct gcatcaccac 900
   cccggtctcc gctgccgaag tgaagaacat cagtaccggc tacatggtga ctaacgactg 960
   caccaatgac agcattacct ggcagctcca ggctgctgtc ctccacgtcc ccgggtgcgt 1020
   cccgtgcgag aaagtgggga atgcatctca gtgctggata ccggtctcac cgaatgtggc 1080
   cgtgcagcgg cccggcgccc tcacgcaggg cttgcggacg cacatcgaca tggttgtgat 1140
   gtccgccacg ctctgctctg ccctctacgt gggggacctc tgcggtgggg tgatgctcgc 1200
   agcccaaatg ttcattgtct cgccgcagca ccactggttt gtccaagact gcaattgctc 1260
   catctaccct ggtaccatca ctggacaccg catggcatgg gacatgatga tgaactggtc 1320
   gcccacggct accatgatct tggcgtacgc gatgcgtgtc cccgaggtca ttatagacat 1380
   cattagcggg gctcattggg gcgtcatgtt cggcttggcc tacttctcta tgcagggagc 1440
   gtgggcgaaa gtcgttgtca tccttctgtt ggccgccggg gtggacgcgc gcacccatac 1500
   tgttgggggt tctgccgcgg attacaagga tgacgatgac aagggaggcg gtagcttatt 1560
   tgacatgggc cccaggcaga aaatccagct cgttaacacc aatggcagct ggcacatcaa 1620
   ccgcaccgcc ctgaactgca atgactcctt gcacaccggc tttatcgcgt ctctgttcta 1680
   cacccacagc ttcaactcgt caggatgtcc cgaacgcatg tccgcctgcc gcagtatcga 1740
   ggccttccgg gtgggatggg gcgccttgca atatgaggat aatgtcacca atccagagga 1800
   tatgagaccc tattgctggc actacccacc aaggcagtgt ggcgtggtct ccgcgaagac 1860
   tgtgtgtggc ccagtgtact gtttcacccc cagcccagtg gtagtgggca cgaccgacag 1920
   gcttggagcg cccacttaca cgtgggggga gaaagagaca gatgtcttcc tattgaacag 1980
   cactcgacca ccgctggggt catggttcgg ctgcacgtgg atgaactctt ctggctacac 2040
   caagacttgc ggcgcaccac cctgccgtac tagagctgac ttcaacgcca gcacggacct 2100
   gttgtgcccc acggactgtt ttaggaagca tcctgatacc acttacctca aatgcggctc 2160
   tgggccctgg ctcacgccaa ggtgcctgat cgactacccc tacaggctct ggcattaccc 2220
   ctgcacagtt aactatacca tcttcaaaat aaggatgtat gtgggagggg ttgagcacag 2280
   gctcacggct gcatgcaatt tcactcgtgg ggatcgttgc aacttggagg acagagacag 2340
   aagtcaactg tctcctttgt tgcactccac cacggaatgg gccattttac cttgctctta 2400
   ctcggacctg cccgccttgt cgactggtct tctccacctc caccaaaaca tcgtggacgt 2460
   acaattcatg tatggcctat cacctgccct cacaaaatac atcgtccgat gggagtgggt 2520
   aatactctta ttcctgctct tagcggacgc cagggtttgc gcctgcttat ggatgctcat 2580
   cttgttgggc caggccgaag cagcactaga gaagctggtc atcttgcacg ctgcgagcgc 2640
   agctagctgc aatggcttcc tatattttgt catctttttc gtggctgctt ggtacatcaa 2700
   gggtcgggta gtccccttag ctacctattc cctcactggc ctgtggtcct ttagcctact 2760
   gctcctagca ttgccccaac aggcttatgc ttatgacgca tctgtgcatg gccagatagg 2820
   agcggctctg ctggtaatga tcaccctctt cacactcacc ccggggtata agaccctcct 2880
   cggccagtgt ctgtggtggt tgtgctatct cctgaccctg ggggaagcca tgattcagga 2940
   gtgggtacca cccatgcagg tgcgcggcgg ccgcgatggc atcgcgtggg ccgtcactat 3000
   attctgcccg ggtgtggtgt ttgacattac caaatggctt ttggcgttgc ttgggcctgc 3060
   ttacctctta agggccgctt tgacacatgt gccgtacttc gtcagagctc acgctctgat 3120
   aagggtatgc gctttggtga agcagctcgc ggggggtagg tatgttcagg tggcgctatt 3180
   ggcccttggc aggtggactg gcacctacat ctatgaccac ctcacaccta tgtcggactg 3240
   ggccgctagc ggcctgcgcg acttagcggt cgccgtggaa cccatcatct tcagtccgat 3300
   ggagaagaag gtcatcgtct ggggagcgga gacggctgca tgtggggaca ttctacatgg 3360
   acttcccgtg tccgcccgac tcggccagga gatcctcctc ggcccagctg atggctacac 3420
   ctccaagggg tggaagctcc ttgctcccat cactgcttat gcccagcaaa cacgaggcct 3480
   cctgggcgcc atagtggtga gtatgacggg gcgtgacagg acagaacagg ccggggaagt 3540
   ccaaatcctg tccacagtct ctcagtcctt cctcggaaca accatctcgg gggttttgtg 3600
   gactgtttac cacggagctg gcaacaagac tctagccggc ttacggggtc cggtcacgca 3660
   gatgtactcg agtgctgagg gggacttggt aggctggccc agcccccctg ggaccaagtc 3720
   tttggagccg tgcaagtgtg gagccgtcga cctatatctg gtcacgcgga acgctgatgt 3780
   catcccggct cggagacgcg gggacaagcg gggagcattg ctctccccga gacccatttc 3840
   gaccttgaag ggtcctcggg gggggccggt gctctgccct aggggccacg tcgttgggct 3900
   cttccgagca gctgtgtgct ctcggggcgt ggccaaatcc atcgatttca tccccgttga 3960
   gacactcgac gttgttacaa ggtctcccac tttcagtgac aacagcacgc caccggctgt 4020
   gccccagacc tatcaggtcg ggtacttgca tgctccaact ggcagtggaa agagcaccaa 4080
   ggtccctgtc gcgtatgccg cccaggggta caaagtacta gtgcttaacc cctcggtagc 4140
   tgccaccctg gggtttgggg cgtacctatc caaggcacat ggcatcaatc ccaacattag 4200
   gactggagtc aggaccgtga tgaccgggga ggccatcacg tactccacat atggcaaatt 4260
   tctcgccgat gggggctgcg ctagcggcgc ctatgacatc atcatatgcg atgaatgcca 4320
   cgctgtggat gctacctcca ttctcggcat cggaacggtc cttgatcaag cagagacagc 4380
   cggggtcaga ctaactgtgc tggctacggc cacacccccc gggtcagtga caacccccca 4440
   tcccgatata gaagaggtag gcctcgggcg ggagggtgag atccccttct atgggagggc 4500
   gattccccta tcctgcatca agggagggag acacctgatt ttctgccact caaagaaaaa 4560
   gtgtgacgag ctcgcggcgg cccttcgggg catgggcttg aatgccgtgg catactatag 4620
   agggttggac gtctccataa taccagctca gggagatgtg gtggtcgtcg ccaccgacgc 4680
   cctcatgacg gggtacactg gagactttga ctccgtgatc gactgcaatg tagcggtcac 4740
   ccaagctgtc gacttcagcc tggaccccac cttcactata accacacaga ctgtcccaca 4800
   agacgctgtc tcacgcagtc agcgccgcgg gcgcacaggt agaggaagac agggcactta 4860
   taggtatgtt tccactggtg aacgagcctc aggaatgttt gacagtgtag tgctttgtga 4920
   gtgctacgac gcaggggctg cgtggtacga tctcacacca gcggagacca ccgtcaggct 4980
   tagagcgtat ttcaacacgc ccggcctacc cgtgtgtcaa gaccatcttg aattttggga 5040
   ggcagttttc accggcctca cacacataga cgcccacttc ctctcccaaa caaagcaagc 5100
   gggggagaac ttcgcgtacc tagtagccta ccaagctacg gtgtgcgcca gagccaaggc 5160
   ccctcccccg tcctgggacg ccatgtggaa gtgcctggcc cgactcaagc ctacgcttgc 5220
   gggccccaca cctctcctgt accgtttggg ccctattacc aatgaggtca ccctcacaca 5280
   ccctgggacg aagtacatcg ccacatgcat gcaagctgac cttgaggtca tgaccagcac 5340
   gtgggtccta gctggaggag tcctggcagc cgtcgccgca tattgcctgg cgactggatg 5400
   cgtttccatc atcggccgct tgcacgtcaa ccagcgagtc gtcgttgcgc cggataagga 5460
   ggtcctgtat gaggcttttg atgagatgga ggaatgcgcc tctagggcgg ctctcatcga 5520
   agaggggcag cggatagccg agatgttgaa gtccaagatc caaggcttgc tgcagcaggc 5580
   ctctaagcag gcccaggaca tacaacccgc tatgcaggct tcatggccca aagtggaaca 5640
   attttgggcc agacacatgt ggaacttcat tagcggcatc caatacctcg caggattgtc 5700
   aacactgcca gggaaccccg cggtggcttc catgatggca ttcagtgccg ccctcaccag 5760
   tccgttgtcg accagtacca ccatccttct caacatcatg ggaggctggt tagcgtccca 5820
   gatcgcacca cccgcggggg ccaccggctt tgtcgtcagt ggcctggtgg gggctgccgt 5880
   gggcagcata ggcctgggta aggtgctggt ggacatcctg gcaggatatg gtgcgggcat 5940
   ttcgggggcc ctcgtcgcat tcaagatcat gtctggcgag aagccctcta tggaagatgt 6000
   catcaatcta ctgcctggga tcctgtctcc gggagccctg gtggtggggg tcatctgcgc 6060
   ggccattctg cgccgccacg tgggaccggg ggagggcgcg gtccaatgga tgaacaggct 6120
   tattgccttt gcttccagag gaaaccacgt cgcccctact cactacgtga cggagtcgga 6180
   tgcgtcgcag cgtgtgaccc aactacttgg ctctcttact ataaccagcc tactcagaag 6240
   actccacaat tggataactg aggactgccc catcccatgc tccggatcct ggctccgcga 6300
   cgtgtgggac tgggtttgca ccatcttgac agacttcaaa aattggctga cctctaaatt 6360
   gttccccaag ctgcccggcc tccccttcat ctcttgtcaa aaggggtaca agggtgtgtg 6420
   ggccggcact ggcatcatga ccacgcgctg cccttgcggc gccaacatct ctggcaatgt 6480
   ccgcctgggc tctatgagga tcacagggcc taaaacctgc atgaacacct ggcaggggac 6540
   ctttcctatc aattgctaca cggagggcca gtgcgcgccg aaacccccca cgaactacaa 6600
   gaccgccatc tggagggtgg cggcctcgga gtacgcggag gtgacgcagc atgggtcgta 6660
   ctcctatgta acaggactga ccactgacaa tctgaaaatt ccttgccaac taccttctcc 6720
   agagtttttc tcctgggtgg acggtgtgca gatccatagg tttgcaccca caccaaagcc 6780
   gtttttccgg gatgaggtct cgttctgcgt tgggcttaat tcctatgctg tcgggtccca 6840
   gcttccctgt gaacctgagc ccgacgcaga cgtattgagg tccatgctaa cagatccgcc 6900
   ccacatcacg gcggagactg cggcgcggcg cttggcacgg ggatcacctc catctgaggc 6960
   gagctcctca gtgagccagc tatcagcacc gtcgctgcgg gccacctgca ccacccacag 7020
   caacacctat gacgtggaca tggtcgatgc caacctgctc atggagggcg gtgtggctca 7080
   gacagagcct gagtccaggg tgcccgttct ggactttctc gagccaatgg ccgaggaaga 7140
   gagcgacctt gagccctcaa taccatcgga gtgcatgctc cccaggagcg ggtttccacg 7200
   ggccttaccg gcttgggcac ggcctgacta caacccgccg ctcgtggaat cgtggaggag 7260
   gccagattac caaccgccca ccgttgctgg ttgtgctctc ccccccccca agaaggcccc 7320
   gacgcctccc ccaaggagac gccggacagt gggtctgagc gagagcacca tatcagaagc 7380
   cctccagcaa ctggccatca agacctttgg ccagcccccc tcgagcggtg atgcaggctc 7440
   gtccacgggg gcgggcgccg ccgaatccgg cggtccgacg tcccctggtg agccggcccc 7500
   ctcagagaca ggttccgcct cctctatgcc ccccctcgag ggggagcctg gagatccgga 7560
   cctggagtct gatcaggtag agcttcaacc tcccccccag gggggggggg tagctcccgg 7620
   ttcgggctcg gggtcttggt ctacttgctc cgaggaggac gataccaccg tgtgctgctc 7680
   catgtcatac tcctggaccg gggctctaat aactccctgt agccccgaag aggaaaagtt 7740
   gccaatcaac cctttgagta actcgctgtt gcgataccat aacaaggtgt actgtacaac 7800
   atcaaagagc gcctcacaga gggctaaaaa ggtaactttt gacaggacgc aagtgctcga 7860
   cgcccattat gactcagtct taaaggacat caagctagcg gcttccaagg tcagcgcaag 7920
   gctcctcacc ttggaggagg cgtgccagtt gactccaccc cattctgcaa gatccaagta 7980
   tggattcggg gccaaggagg tccgcagctt gtccgggagg gccgttaacc acatcaagtc 8040
   cgtgtggaag gacctcctgg aagacccaca aacaccaatt cccacaacca tcatggccaa 8100
   aaatgaggtg ttctgcgtgg accccgccaa ggggggtaag aaaccagctc gcctcatcgt 8160
   ttaccctgac ctcggcgtcc gggtctgcga gaaaatggcc ctctatgaca ttacacaaaa 8220
   gcttcctcag gcggtaatgg gagcttccta tggcttccag tactcccctg cccaacgggt 8280
   ggagtatctc ttgaaagcat gggcggaaaa gaaggacccc atgggttttt cgtatgatac 8340
   ccgatgcttc gactcaaccg tcactgagag agacatcagg accgaggagt ccatatacca 8400
   ggcctgctcc ctgcccgagg aggcccgcac tgccatacac tcgctgactg agagacttta 8460
   cgtaggaggg cccatgttca acagcaaggg tcaaacctgc ggttacagac gttgccgcgc 8520
   cagcggggtg ctaaccacta gcatgggtaa caccatcaca tgctatgtga aagccctagc 8580
   ggcctgcaag gctgcgggga tagttgcgcc cacaatgctg gtatgcggcg atgacctagt 8640
   agtcatctca gaaagccagg ggactgagga ggacgagcgg aacctgagag ccttcacgga 8700
   ggccatgacc aggtactctg cccctcctgg tgatcccccc agaccggaat atgacctgga 8760
   gctaataaca tcctgttcct caaatgtgtc tgtggcgttg ggcccgcggg gccgccgcag 8820
   atactacctg accagagacc caaccactcc actcgcccgg gctgcctggg aaacagttag 8880
   acactcccct atcaattcat ggctgggaaa catcatccag tatgctccaa ccatatgggt 8940
   tcgcatggtc ctaatgacac acttcttctc cattctcatg gtccaagaca ccctggacca 9000
   gaacctcaac tttgagatgt atggatcagt atactccgtg aatcctttgg accttccagc 9060
   cataattgag aggttacacg ggcttgacgc cttttctatg cacacatact ctcaccacga 9120
   actgacgcgg gtggcttcag ccctcagaaa acttggggcg ccacccctca gggtgtggaa 9180
   gagtcgggct cgcgcagtca gggcgtccct catctcccgt ggagggaaag cggccgtttg 9240
   cggccgatat ctcttcaatt gggcggtgaa gaccaagctc aaactcactc cattgccgga 9300
   ggcgcgccta ctggacttat ccagttggtt caccgtcggc gccggcgggg gcgacatttt 9360
   tcacagcgtg tcgcgcgccc gaccccgctc attactcttc ggcctactcc tacttttcgt 9420
   aggggtaggc ctcttcctac tccccgctcg gtagagcggc acacactagg tacactccat 9480
   agctaactgt tccttttttt tttttttttt tttttttttt tttttttttt tttttctttt 9540
   tttttttttt tccctctttc ttcccttctc atcttattct actttctttc ttggtggctc 9600
   catcttagcc ctagtcacgg ctagctgtga aaggtccgtg agccgcatga ctgcagagag 9660
   tgccgtaact ggtctctctg cagatcatgt ctaga 9695

<210> 21
<211> 9737
<212> DNA
<213> Artificial

<220>
<223> pJ6/JFH-1 (3XFLAG) N->K

<400> 21
acccgcccct aataggggcg acactccgcc atgaatcact cccctgtgag gaactactgt 60

cttcacgcag aaagcgtcta gccatggcgt tagtatgagt gtcgtacagc ctccaggccc 120

ccccctcccg ggagagccat agtggtctgc ggaaccggtg agtacaccgg aattgccggg 180

aagactgggt cctttcttgg ataaacccac tctatgcccg gccatttggg cgtgcccccg 240

caagactgct agccgagtag cgttgggttg cgaaaggcct tgtggtactg cctgataggg 300

tgcttgcgag tgccccggga ggtctcgtag accgtgcacc atgagcacaa atcctaaacc 360

tcaaagaaaa accaaaagaa acaccaaccg tcgcccacaa gacgttaagt ttccgggcgg 420

cggccagatc gttggcggag tatacttgtt gccgcgcagg ggccccaggt tgggtgtgcg 480

cgcgacaagg aagacttcgg agcggtccca gccacgtgga aggcgccagc ccatccctaa 540

agatcggcgc tccactggca aatcctgggg aaaaccagga tacccctggc ccctatacgg 600

gaatgaggga ctcggctggg caggatggct cctgtccccc cgaggttccc gtccctcttg 660

gggccccaat gacccccggc ataggtcgcg caacgtgggt aaggtcatcg ataccctaac 720

gtgcggcttt gccgacctca tggggtacat ccctgtcgtg ggcgccccgc tcggcggcgt 780

cgccagagct ctcgcgcatg gcgtgagagt cctggaggac ggggttaatt ttgcaacagg 840

gaacttaccc ggttgctcct tttctatctt cttgctggcc ctgctgtcct gcatcaccac 900

cccggtctcc gctgccgaag tgaagaacat cagtaccggc tacatggtga ctaacgactg 960

caccaatgac agcattacct ggcagctcca ggctgctgtc ctccacgtcc ccgggtgcgt 1020

cccgtgcgag aaagtgggga atgcatctca gtgctggata ccggtctcac cgaatgtggc 1080

cgtgcagcgg cccggcgccc tcacgcaggg cttgcggacg cacatcgaca tggttgtgat 1140

gtccgccacg ctctgctctg ccctctacgt gggggacctc tgcggtgggg tgatgctcgc 1200

agcccaaatg ttcattgtct cgccgcagca ccactggttt gtccaagact gcaattgctc 1260

catctaccct ggtaccatca ctggacaccg catggcatgg gacatgatga tgaactggtc 1320

gcccacggct accatgatct tggcgtacgc gatgcgtgtc cccgaggtca ttatagacat 1380

cattagcggg gctcattggg gcgtcatgtt cggcttggcc tacttctcta tgcagggagc 1440

gtgggcgaaa gtcgttgtca tccttctgtt ggccgccggg gtggacgcgc gcacccatac 1500

tgttgggggt tctgccgcgg actacaaaga ccatgacggt gattataaag atcatgacat 1560

cgattacaag gatgacgatg acaagggagg cggtagctta tttgacatgg gccccaggca 1620

gaaaatccag ctcgttaaca ccaatggcag ctggcacatc aaccgcaccg ccctgaactg 1680

caatgactcc ttgcacaccg gctttatcgc gtctctgttc tacacccaca gcttcaactc 1740

gtcaggatgt cccgaacgca tgtccgcctg ccgcagtatc gaggccttcc gggtgggatg 1800

gggcgccttg caatatgagg ataatgtcac caatccagag gatatgagac cctattgctg 1860

gcactaccca ccaaggcagt gtggcgtggt ctccgcgaag actgtgtgtg gcccagtgta 1920

ctgtttcacc cccagcccag tggtagtggg cacgaccgac aggcttggag cgcccactta 1980

cacgtggggg gagaaagaga cagatgtctt cctattgaac agcactcgac caccgctggg 2040

gtcatggttc ggctgcacgt ggatgaactc ttctggctac accaagactt gcggcgcacc 2100

accctgccgt actagagctg acttcaacgc cagcacggac ctgttgtgcc ccacggactg 2160

ttttaggaag catcctgata ccacttacct caaatgcggc tctgggccct ggctcacgcc 2220

aaggtgcctg atcgactacc cctacaggct ctggcattac ccctgcacag ttaactatac 2280

catcttcaaa ataaggatgt atgtgggagg ggttgagcac aggctcacgg ctgcatgcaa 2340

tttcactcgt ggggatcgtt gcaacttgga ggacagagac agaagtcaac tgtctccttt 2400

gttgcactcc accacggaat gggccatttt accttgctct tactcggacc tgcccgcctt 2460

gtcgactggt cttctccacc tccaccaaaa catcgtggac gtacaattca tgtatggcct 2520

atcacctgcc ctcacaaaat acatcgtccg atgggagtgg gtaatactct tattcctgct 2580

cttagcggac gccagggttt gcgcctgctt atggatgctc atcttgttgg gccaggccga 2640

agcagcacta gagaagctgg tcatcttgca cgctgcgagc gcagctagct gcaatggctt 2700

cctatatttt gtcatctttt tcgtggctgc ttggtacatc aagggtcggg tagtcccctt 2760

agctacctat tccctcactg gcctgtggtc ctttagccta ctgctcctag cattgcccca 2820

acaggcttat gcttatgacg catctgtgca tggccagata ggagcggctc tgctggtaat 2880

gatcaccctc ttcacactca ccccggggta taagaccctc ctcggccagt gtctgtggtg 2940

gttgtgctat ctcctgaccc tgggggaagc catgattcag gagtgggtac cacccatgca 3000

ggtgcgcggc ggccgcgatg gcatcgcgtg ggccgtcact atattctgcc cgggtgtggt 3060

gtttgacatt accaaatggc ttttggcgtt gcttgggcct gcttacctct taagggccgc 3120

tttgacacat gtgccgtact tcgtcagagc tcacgctctg ataagggtat gcgctttggt 3180

gaagcagctc gcggggggta ggtatgttca ggtggcgcta ttggcccttg gcaggtggac 3240

tggcacctac atctatgacc acctcacacc tatgtcggac tgggccgcta gcggcctgcg 3300

cgacttagcg gtcgccgtgg aacccatcat cttcagtccg atggagaaga aggtcatcgt 3360

ctggggagcg gagacggctg catgtgggga cattctacat ggacttcccg tgtccgcccg 3420

actcggccag gagatcctcc tcggcccagc tgatggctac acctccaagg ggtggaagct 3480

ccttgctccc atcactgctt atgcccagca aacacgaggc ctcctgggcg ccatagtggt 3540

gagtatgacg gggcgtgaca ggacagaaca ggccggggaa gtccaaatcc tgtccacagt 3600

ctctcagtcc ttcctcggaa caaccatctc ggggggtttg tggactgttt accacggagc 3660

tggcaacaag actctagccg gcttacgggg tccggtcacg cagatgtact cgagtgctga 3720

gggggacttg gtaggctggc ccagcccccc tgggaccaag tctttggagc cgtgcaagtg 3780

tggagccgtc gacctatatc tggtcacgcg gaacgctgat gtcatcccgg ctcggagacg 3840

cggggacaag cggggagcat tgctctcccc gagacccatt tcgaccttga aggggtcctc 3900

gggggggccg gtgctctgcc ctaggggcca cgtcgttggg ctcttccgag cagctgtgtg 3960

ctctcggggc gtggccaaat ccatcgattt catccccgtt gagacactcg acgttgttac 4020

aaggtctccc actttcagtg acaacagcac gccaccggct gtgccccaga cctatcaggt 4080

cgggtacttg catgctccaa ctggcagtgg aaagagcacc aaggtccctg tcgcgtatgc 4140

cgcccagggg tacaaagtac tagtgcttaa cccctcggta gctgccaccc tggggtttgg 4200

ggcgtaccta tccaaggcac atggcatcaa tcccaacatt aggactggag tcaggaccgt 4260

gatgaccggg gaggccatca cgtactccac atatggcaaa tttctcgccg atgggggctg 4320

cgctagcggc gcctatgaca tcatcatatg cgatgaatgc cacgctgtgg atgctacctc 4380

cattctcggc atcggaacgg tccttgatca agcagagaca gccggggtca gactaactgt 4440

gctggctacg gccacacccc ccgggtcagt gacaaccccc catcccgata tagaagaggt 4500

aggcctcggg cgggagggtg agatcccctt ctatgggagg gcgattcccc tatcctgcat 4560

caagggaggg agacacctga ttttctgcca ctcaaagaaa aagtgtgacg agctcgcggc 4620

ggcccttcgg ggcatgggct tgaatgccgt ggcatactat agagggttgg acgtctccat 4680

aataccagct cagggagatg tggtggtcgt cgccaccgac gccctcatga cggggtacac 4740

tggagacttt gactccgtga tcgactgcaa tgtagcggtc acccaagctg tcgacttcag 4800

cctggacccc accttcacta taaccacaca gactgtccca caagacgctg tctcacgcag 4860

tcagcgccgc gggcgcacag gtagaggaag acagggcact tataggtatg tttccactgg 4920

tgaacgagcc tcaggaatgt ttgacagtgt agtgctttgt gagtgctacg acgcaggggc 4980

tgcgtggtac gatctcacac cagcggagac caccgtcagg cttagagcgt atttcaacac 5040

gcccggccta cccgtgtgtc aagaccatct tgaattttgg gaggcagttt tcaccggcct 5100

cacacacata gacgcccact tcctctccca aacaaagcaa gcgggggaga acttcgcgta 5160

cctagtagcc taccaagcta cggtgtgcgc cagagccaag gcccctcccc cgtcctggga 5220

cgccatgtgg aagtgcctgg cccgactcaa gcctacgctt gcgggcccca cacctctcct 5280

gtaccgtttg ggccctatta ccaatgaggt caccctcaca caccctggga cgaagtacat 5340

cgccacatgc atgcaagctg accttgaggt catgaccagc acgtgggtcc tagctggagg 5400

agtcctggca gccgtcgccg catattgcct ggcgactgga tgcgtttcca tcatcggccg 5460

cttgcacgtc aaccagcgag tcgtcgttgc gccggataag gaggtcctgt atgaggcttt 5520

tgatgagatg gaggaatgcg cctctagggc ggctctcatc gaagaggggc agcggatagc 5580

cgagatgttg aagtccaaga tccaaggctt gctgcagcag gcctctaagc aggcccagga 5640

catacaaccc gctatgcagg cttcatggcc caaagtggaa caattttggg ccagacacat 5700

gtggaacttc attagcggca tccaatacct cgcaggattg tcaacactgc cagggaaccc 5760

cgcggtggct tccatgatgg cattcagtgc cgccctcacc agtccgttgt cgaccagtac 5820

caccatcctt ctcaacatca tgggaggctg gttagcgtcc cagatcgcac cacccgcggg 5880

ggccaccggc tttgtcgtca gtggcctggt gggggctgcc gtgggcagca taggcctggg 5940

taaggtgctg gtggacatcc tggcaggata tggtgcgggc atttcggggg ccctcgtcgc 6000

attcaagatc atgtctggcg agaagccctc tatggaagat gtcatcaatc tactgcctgg 6060

gatcctgtct ccgggagccc tggtggtggg ggtcatctgc gcggccattc tgcgccgcca 6120

cgtgggaccg ggggagggcg cggtccaatg gatgaacagg cttattgcct ttgcttccag 6180

aggaaaccac gtcgccccta ctcactacgt gacggagtcg gatgcgtcgc agcgtgtgac 6240

ccaactactt ggctctctta ctataaccag cctactcaga agactccaca attggataac 6300

tgaggactgc cccatcccat gctccggatc ctggctccgc gacgtgtggg actgggtttg 6360

caccatcttg acagacttca aaaattggct gacctctaaa ttgttcccca agctgcccgg 6420

cctccccttc atctcttgtc aaaaggggta caagggtgtg tgggccggca ctggcatcat 6480

gaccacgcgc tgcccttgcg gcgccaacat ctctggcaat gtccgcctgg gctctatgag 6540

gatcacaggg cctaaaacct gcatgaacac ctggcagggg acctttccta tcaattgcta 6600

cacggagggc cagtgcgcgc cgaaaccccc cacgaactac aagaccgcca tctggagggt 6660

ggcggcctcg gagtacgcgg aggtgacgca gcatgggtcg tactcctatg taacaggact 6720

gaccactgac aatctgaaaa ttccttgcca actaccttct ccagagtttt tctcctgggt 6780

ggacggtgtg cagatccata ggtttgcacc cacaccaaag ccgtttttcc gggatgaggt 6840

ctcgttctgc gttgggctta attcctatgc tgtcgggtcc cagcttccct gtgaacctga 6900

gcccgacgca gacgtattga ggtccatgct aacagatccg ccccacatca cggcggagac 6960

tgcggcgcgg cgcttggcac ggggatcacc tccatctgag gcgagctcct cagtgagcca 7020

gctatcagca ccgtcgctgc gggccacctg caccacccac agcaacacct atgacgtgga 7080

catggtcgat gccaacctgc tcatggaggg cggtgtggct cagacagagc ctgagtccag 7140

ggtgcccgtt ctggactttc tcgagccaat ggccgaggaa gagagcgacc ttgagccctc 7200

aataccatcg gagtgcatgc tccccaggag cgggtttcca cgggccttac cggcttgggc 7260

acggcctgac tacaacccgc cgctcgtgga atcgtggagg aggccagatt accaaccgcc 7320

caccgttgct ggttgtgctc tccccccccc caagaaggcc ccgacgcctc ccccaaggag 7380

acgccggaca gtgggtctga gcgagagcac catatcagaa gccctccagc aactggccat 7440

caagaccttt ggccagcccc cctcgagcgg tgatgcaggc tcgtccacgg gggcgggcgc 7500

cgccgaatcc ggcggtccga cgtcccctgg tgagccggcc ccctcagaga caggttccgc 7560

ctcctctatg ccccccctcg agggggagcc tggagatccg gacctggagt ctgatcaggt 7620

agagcttcaa cctccccccc aggggggggg ggtagctccc ggttcgggct cggggtcttg 7680

gtctacttgc tccgaggagg acgataccac cgtgtgctgc tccatgtcat actcctggac 7740

cggggctcta ataactccct gtagccccga agaggaaaag ttgccaatca accctttgag 7800

taactcgctg ttgcgatacc ataacaaggt gtactgtaca acatcaaaga gcgcctcaca 7860

gagggctaaa aaggtaactt ttgacaggac gcaagtgctc gacgcccatt atgactcagt 7920

cttaaaggac atcaagctag cggcttccaa ggtcagcgca aggctcctca ccttggagga 7980

ggcgtgccag ttgactccac cccattctgc aagatccaag tatggattcg gggccaagga 8040

ggtccgcagc ttgtccggga gggccgttaa ccacatcaag tccgtgtgga aggacctcct 8100

ggaagaccca caaacaccaa ttcccacaac catcatggcc aaaaatgagg tgttctgcgt 8160

ggaccccgcc aaggggggta agaaaccagc tcgcctcatc gtttaccctg acctcggcgt 8220

ccgggtctgc gagaaaatgg ccctctatga cattacacaa aagcttcctc aggcggtaat 8280

gggagcttcc tatggcttcc agtactcccc tgcccaacgg gtggagtatc tcttgaaagc 8340

atgggcggaa aagaaggacc ccatgggttt ttcgtatgat acccgatgct tcgactcaac 8400

cgtcactgag agagacatca ggaccgagga gtccatatac caggcctgct ccctgcccga 8460

ggaggcccgc actgccatac actcgctgac tgagagactt tacgtaggag ggcccatgtt 8520

caacagcaag ggtcaaacct gcggttacag acgttgccgc gccagcgggg tgctaaccac 8580

tagcatgggt aacaccatca catgctatgt gaaagcccta gcggcctgca aggctgcggg 8640

gatagttgcg cccacaatgc tggtatgcgg cgatgaccta gtagtcatct cagaaagcca 8700

ggggactgag gaggacgagc ggaacctgag agccttcacg gaggccatga ccaggtactc 8760

tgcccctcct ggtgatcccc ccagaccgga atatgacctg gagctaataa catcctgttc 8820

ctcaaatgtg tctgtggcgt tgggcccgcg gggccgccgc agatactacc tgaccagaga 8880

cccaaccact ccactcgccc gggctgcctg ggaaacagtt agacactccc ctatcaattc 8940

atggctggga aacatcatcc agtatgctcc aaccatatgg gttcgcatgg tcctaatgac 9000

acacttcttc tccattctca tggtccaaga caccctggac cagaacctca actttgagat 9060

gtatggatca gtatactccg tgaatccttt ggaccttcca gccataattg agaggttaca 9120

cgggcttgac gccttttcta tgcacacata ctctcaccac gaactgacgc gggtggcttc 9180

agccctcaga aaacttgggg cgccacccct cagggtgtgg aagagtcggg ctcgcgcagt 9240

cagggcgtcc ctcatctccc gtggagggaa agcggccgtt tgcggccgat atctcttcaa 9300

ttgggcggtg aagaccaagc tcaaactcac tccattgccg gaggcgcgcc tactggactt 9360

atccagttgg ttcaccgtcg gcgccggcgg gggcgacatt tttcacagcg tgtcgcgcgc 9420

ccgaccccgc tcattactct tcggcctact cctacttttc gtaggggtag gcctcttcct 9480

actccccgct cggtagagcg gcacacacta ggtacactcc atagctaact gttccttttt 9540

tttttttttt tttttttttt tttttttttt ttttttttct tttttttttt tttccctctt 9600

tcttcccttc tcatcttatt ctactttctt tcttggtggc tccatcttag ccctagtcac 9660

ggctagctgt gaaaggtccg tgagccgcat gactgcagag agtgccgtaa ctggtctctc    9720

tgcagatcat gtctaga                                                   9737

<210>  22
<211>  30
<212>  PRT
<213>  Artificial

<220>
<223>  N terminal sequence of E2 protein encoded in pJFH-1

<400>  22

Gly Thr Thr Thr Val Gly Gly Ala Val Ala Arg Ser Thr Asn Val Ile
1               5                   10                  15

Ala Gly Val Phe Ser His Gly Pro Gln Gln Asn Ile Gln Leu
            20                  25                  30

<210>  23
<211>  34
<212>  PRT
<213>  Artificial

<220>
<223>  N terminal sequence of E2 protein encoded in pJFH-1(1XFLAG)

<400>  23

Gly Thr Thr Thr Val Gly Gly Ala Val Ala Asp Tyr Lys Asp Asp Asp
1               5                   10                  15

Asp Lys Gly Gly Gly Gly Val Phe Ser His Gly Pro Gln Gln Asn Ile
            20                  25                  30

Gln Leu

<210> 24
<211> 48
<212> PRT
<213> Artificial

<220>
<223> N terminal sequence of E2 protein encoded in pJFH-1 (3×FLAG)

<400> 24

Gly Thr Thr Thr Val Gly Gly Ala Val Ala Asp Tyr Lys Asp His Asp
1               5                   10                  15

Gly Asp Tyr Lys Asp His Asp Ile Asp Tyr Lys Asp Asp Asp Asp Lys
            20                  25                  30

Gly Gly Gly Gly Val Phe Ser His Gly Pro Gln Gln Asn Ile Gln Leu
        35                  40                  45

<210> 25
<211> 23
<212> DNA
<213> Artificial

<220>
<223> primer(JFH1-F)

<400> 25
ccggaattct aatacgactc act                                              23

<210> 26
<211> 44
<212> DNA
<213> Artificial

<220>
<223>  primer(JFH1-1F-R)

<400>  26
cccttgtcat cgtcatcctt gtaatctgca acagcgcctc caac                44


<210>  27
<211>  44
<212>  DNA
<213>  Artificial

<220>
<223>  primer(JFH1-1F-F)

<400>  27
aggatgacga tgacaaggga ggcggtggcg tgttcagcca tggc                44


<210>  28
<211>  65
<212>  DNA
<213>  Artificial

<220>
<223>  primer(JFH1-3F-R)

<400>  28
taatcgatgt catgatcttt ataatcaccg tcatggtctt tgtagtctgc aacagcgcct      60

ccaac                                                                  65


<210>  29
<211>  65
<212>  DNA
<213>  Artificial

<220>
<223>  primer(JFH1-3F-F)

<400> 29
aagatcatga catcgattac aaggatgacg atgacaaggg aggcggtggc gtgttcagcc 60

atggc 65

<210> 30
<211> 9690
<212> DNA
<213> Artificial

<220>
<223> pJFH-1(1XFLAG)

<400> 30
acctgcccct aataggggcg acactccgcc atgaatcact cccctgtgag gaactactgt 60

cttcacgcag aaagcgccta gccatggcgt tagtatgagt gtcgtacagc ctccaggccc 120

ccccctcccg ggagagccat agtggtctgc ggaaccggtg agtacaccgg aattgccggg 180

aagactgggt cctttcttgg ataaacccac tctatgcccg gccatttggg cgtgcccccg 240

caagactgct agccgagtag cgttgggttg cgaaaggcct tgtggtactg cctgataggg 300

cgcttgcgag tgccccggga ggtctcgtag accgtgcacc atgagcacaa atcctaaacc 360

tcaaagaaaa accaaaagaa acaccaaccg tcgcccagaa gacgttaagt tcccgggcgg 420

cggccagatc gttggcggag tatacttgtt gccgcgcagg ggccccaggt tgggtgtgcg 480

cacgacaagg aaaacttcgg agcggtccca gccacgtggg agacgccagc ccatccccaa 540

agatcggcgc tccactggca aggcctgggg aaaaccaggt cgcccctggc ccctatatgg 600

gaatgaggga ctcggctggg caggatggct cctgtccccc cgaggctctc gcccctcctg 660

gggccccact gacccccggc ataggtcgcg caacgtgggt aaagtcatcg acaccctaac 720

gtgtggcttt gccgacctca tggggtacat ccccgtcgta ggcgccccgc ttagtggcgc 780

cgccagagct gtcgcgcacg gcgtgagagt cctggaggac ggggttaatt atgcaacagg 840

gaacctaccc ggtttcccct tttctatctt cttgctggcc ctgttgtcct gcatcaccgt 900

tccggtctct gctgcccagg tgaagaatac cagtagcagc tacatggtga ccaatgactg 960

ctccaatgac agcatcactt ggcagctcga ggctgcggtt ctccacgtcc ccgggtgcgt 1020

cccgtgcgag agagtgggga atacgtcacg gtgttgggtg ccagtctcgc caaacatggc 1080

tgtgcggcag cccggtgccc tcacgcaggg tctgcggacg cacatcgata tggttgtgat 1140

gtccgccacc ttctgctctg ctctctacgt gggggacctc tgtggcgggg tgatgctcgc 1200

ggcccaggtg ttcatcgtct cgccgcagta ccactggttt gtgcaagaat gcaattgctc 1260

catctaccct ggcaccatca ctggacaccg catggcatgg gacatgatga tgaactggtc 1320

gcccacggcc accatgatcc tggcgtacgt gatgcgcgtc cccgaggtca tcatagacat 1380

cgttagcggg gctcactggg gcgtcatgtt cggcttggcc tacttctcta tgcagggagc 1440

gtgggcgaag gtcattgtca tccttctgct ggccgctggg gtggacgcgg gcaccaccac 1500

cgttggaggc gctgttgcag attacaagga tgacgatgac aagggaggcg gtggcgtgtt 1560

cagccatggc cctcagcaga acattcagct cattaacacc aacggcagtt ggcacatcaa 1620

ccgtactgcc ttgaattgca atgactcctt gaacaccggc tttctcgcgg ccttgttcta 1680

caccaaccgc tttaactcgt cagggtgtcc agggcgcctg tccgcctgcc gcaacatcga 1740

ggctttccgg atagggtggg gcaccctaca gtacgaggat aatgtcacca atccagagga 1800

tatgaggccg tactgctggc actacccccc aaagccgtgt ggcgtagtcc ccgcgaggtc 1860

tgtgtgtggc ccagtgtact gtttcacccc cagcccggta gtagtgggca cgaccgacag 1920

acgtggagtg cccacctaca catggggaga gaatgagaca gatgtcttcc tactgaacag 1980

cacccgaccg ccgcagggct catggttcgg ctgcacgtgg atgaactcca ctggtttcac 2040

caagacttgt ggcgcgccac cttgccgcac cagagctgac ttcaacgcca gcacggactt 2100

gttgtgccct acggattgtt ttaggaagca tcctgatgcc acttatatta agtgtggttc 2160

tgggccctgg ctcacaccaa agtgcctggt ccactaccct tacagactct ggcattaccc 2220

ctgcacagtc aattttacca tcttcaagat aagaatgtat gtaggggggg ttgagcacag 2280

gctcacggcc gcatgcaact tcactcgtgg ggatcgctgc gacttggagg acagggacag 2340

gagtcagctg tctcctctgt tgcactctac cacggaatgg gccatcctgc cctgcaccta 2400

ctcagactta cccgctttgt caactggtct tctccacctt caccagaaca tcgtggacgt 2460

acaatacatg tatggcctct cacctgctat cacaaaatac gtcgttcgat gggagtgggt 2520

ggtactctta ttcctgctct tagcggacgc cagagtctgc gcctgcttgt ggatgctcat 2580

cttgttgggc caggccgaag cagcattgga gaagttggtc gtcttgcacg ctgcgagtgc 2640

ggctaactgc catggcctcc tatattttgc catcttcttc gtggcagctt ggcacatcag 2700

gggtcgggtg gtccccttga ccacctattg cctcactggc ctatggccct tctgcctact 2760

gctcatggca ctgccccggc aggcttatgc ctatgacgca cctgtgcacg gacagatagg 2820

cgtgggtttg ttgatattga tcaccctctt cacactcacc ccggggtata agaccctcct 2880

cggccagtgt ctgtggtggt tgtgctatct cctgaccctg ggggaagcca tgattcagga 2940

gtgggtacca cccatgcagg tgcgcggcgg ccgcgatggc atcgcgtggg ccgtcactat 3000

attctgcccg ggtgtggtgt ttgacattac caaatggctt ttggcgttgc ttgggcctgc 3060

ttacctctta agggccgctt tgacacatgt gccgtacttc gtcagagctc acgctctgat 3120

aagggtatgc gctttggtga agcagctcgc ggggggtagg tatgttcagg tggcgctatt 3180

ggcccttggc aggtggactg gcacctacat ctatgaccac ctcacaccta tgtcggactg 3240

ggccgctagc ggcctgcgcg acttagcggt cgccgtggaa cccatcatct tcagtccgat 3300

ggagaagaag gtcatcgtct ggggagcgga gacggctgca tgtggggaca ttctacatgg 3360

acttcccgtg tccgcccgac tcggccagga gatcctcctc ggcccagctg atggctacac 3420

ctccaagggg tggaagctcc ttgctcccat cactgcttat gcccagcaaa cacgaggcct 3480

cctgggcgcc atagtggtga gtatgacggg gcgtgacagg acagaacagg ccggggaagt 3540

ccaaatcctg tccacagtct ctcagtcctt cctcggaaca accatctcgg gggttttgtg 3600

gactgtttac cacggagctg gcaacaagac tctagccggc ttacggggtc cggtcacgca 3660

gatgtactcg agtgctgagg gggacttggt aggctggccc agcccccctg ggaccaagtc 3720

tttggagccg tgcaagtgtg gagccgtcga cctatatctg gtcacgcgga acgctgatgt 3780

catcccggct cggagacgcg gggacaagcg gggagcattg ctctccccga gacccatttc 3840

gaccttgaag gggtcctcgg gggggccggt gctctgccct aggggccacg tcgttgggct 3900

cttccgagca gctgtgtgct ctcggggcgt ggccaaatcc atcgatttca tccccgttga 3960

gacactcgac gttgttacaa ggtctcccac tttcagtgac aacagcacgc caccggctgt 4020

gccccagacc tatcaggtcg ggtacttgca tgctccaact ggcagtggaa agagcaccaa 4080

ggtccctgtc gcgtatgccg cccaggggta caaagtacta gtgcttaacc cctcggtagc 4140

tgccaccctg gggtttgggg cgtacctatc caaggcacat ggcatcaatc ccaacattag 4200

gactggagtc aggaccgtga tgaccgggga ggccatcacg tactccacat atggcaaatt 4260

tctcgccgat gggggctgcg ctagcggcgc ctatgacatc atcatatgcg atgaatgcca 4320

cgctgtggat gctacctcca ttctcggcat cggaacggtc cttgatcaag cagagacagc 4380

cggggtcaga ctaactgtgc tggctacggc cacacccccc gggtcagtga caacccccca 4440

tcccgatata gaagaggtag gcctcgggcg ggagggtgag atccccttct atgggagggc 4500

gattccccta tcctgcatca agggagggag acacctgatt ttctgccact caaagaaaaa 4560

gtgtgacgag ctcgcggcgg cccttcgggg catgggcttg aatgccgtgg catactatag . 4620

agggttggac gtctccataa taccagctca gggagatgtg gtggtcgtcg ccaccgacgc 4680

cctcatgacg gggtacactg gagactttga ctccgtgatc gactgcaatg tagcggtcac 4740

ccaagctgtc gacttcagcc tggaccccac cttcactata accacacaga ctgtcccaca 4800

agacgctgtc tcacgcagtc agcgccgcgg gcgcacaggt agaggaagac agggcactta 4860

taggtatgtt tccactggtg aacgagcctc aggaatgttt gacagtgtag tgctttgtga 4920

gtgctacgac gcaggggctg cgtggtacga tctcacacca gcggagacca ccgtcaggct 4980

tagagcgtat ttcaacacgc ccggcctacc cgtgtgtcaa gaccatcttg aattttggga 5040

ggcagttttc accggcctca cacacataga cgcccacttc ctctcccaaa caaagcaagc 5100

gggggagaac ttcgcgtacc tagtagccta ccaagctacg gtgtgcgcca gagccaaggc 5160

ccctcccccg tcctgggacg ccatgtggaa gtgcctggcc cgactcaagc ctacgcttgc 5220

gggccccaca cctctcctgt accgtttggg ccctattacc aatgaggtca ccctcacaca 5280

ccctgggacg aagtacatcg ccacatgcat gcaagctgac cttgaggtca tgaccagcac 5340

gtgggtccta gctggaggag tcctggcagc cgtcgccgca tattgcctgg cgactggatg 5400

cgtttccatc atcggccgct tgcacgtcaa ccagcgagtc gtcgttgcgc cggataagga 5460

ggtcctgtat gaggcttttg atgagatgga ggaatgcgcc tctagggcgg ctctcatcga 5520

agaggggcag cggatagccg agatgttgaa gtccaagatc caaggcttgc tgcagcaggc 5580

ctctaagcag gcccaggaca tacaacccgc tatgcaggct tcatggccca aagtggaaca 5640

attttgggcc agacacatgt ggaacttcat tagcggcatc caatacctcg caggattgtc 5700

aacactgcca gggaaccccg cggtggcttc catgatggca ttcagtgccg ccctcaccag 5760

tccgttgtcg accagtacca ccatccttct caacatcatg ggaggctggt tagcgtccca 5820

gatcgcacca cccgcggggg ccaccggctt tgtcgtcagt ggcctggtgg gggctgccgt 5880

gggcagcata ggcctgggta aggtgctggt ggacatcctg gcaggatatg gtgcgggcat 5940

ttcgggggcc ctcgtcgcat tcaagatcat gtctggcgag aagccctcta tggaagatgt 6000

catcaatcta ctgcctggga tcctgtctcc gggagccctg gtggtggggg tcatctgcgc 6060

ggccattctg cgccgccacg tgggaccggg ggagggcgcg gtccaatgga tgaacaggct 6120

tattgccttt gcttccagag gaaaccacgt cgcccctact cactacgtga cggagtcgga 6180

tgcgtcgcag cgtgtgaccc aactacttgg ctctcttact ataaccagcc tactcagaag 6240

actccacaat tggataactg aggactgccc catcccatgc tccggatcct ggctccgcga 6300

cgtgtgggac tgggtttgca ccatcttgac agacttcaaa aattggctga cctctaaatt 6360

gttccccaag ctgcccggcc tccccttcat ctcttgtcaa aaggggtaca agggtgtgtg 6420

ggccggcact ggcatcatga ccacgcgctg cccttgcggc gccaacatct ctggcaatgt 6480

ccgcctgggc tctatgagga tcacagggcc taaaacctgc atgaacacct ggcaggggac 6540

ctttcctatc aattgctaca cggagggcca gtgcgcgccg aaacccccca cgaactacaa 6600

gaccgccatc tggagggtgg cggcctcgga gtacgcggag gtgacgcagc atgggtcgta 6660

ctcctatgta acaggactga ccactgacaa tctgaaaatt ccttgccaac taccttctcc 6720

agagtttttc tcctgggtgg acggtgtgca gatccatagg tttgcaccca caccaaagcc 6780

gtttttccgg gatgaggtct cgttctgcgt tgggcttaat tcctatgctg tcgggtccca 6840

gcttccctgt gaacctgagc ccgacgcaga cgtattgagg tccatgctaa cagatccgcc 6900

ccacatcacg gcggagactg cggcgcggcg cttggcacgg ggatcacctc catctgaggc 6960

gagctcctca gtgagccagc tatcagcacc gtcgctgcgg gccacctgca ccacccacag 7020

caacacctat gacgtggaca tggtcgatgc caacctgctc atggagggcg gtgtggctca 7080

gacagagcct gagtccaggg tgcccgttct ggactttctc gagccaatgg ccgaggaaga 7140

gagcgacctt gagccctcaa taccatcgga gtgcatgctc cccaggagcg ggtttccacg 7200

ggccttaccg gcttgggcac ggcctgacta caacccgccg ctcgtggaat cgtggaggag 7260

gccagattac caaccgccca ccgttgctgg ttgtgctctc ccccccccca agaaggcccc 7320

gacgcctccc ccaaggagac gccggacagt gggtctgagc gagagcacca tatcagaagc 7380

cctccagcaa ctggccatca agacctttgg ccagcccccc tcgagcggtg atgcaggctc 7440

gtccacgggg gcgggcgccg ccgaatccgg cggtccgacg tcccctggtg agccggcccc 7500

ctcagagaca ggttccgcct cctctatgcc ccccctcgag ggggagcctg gagatccgga 7560

cctggagtct gatcaggtag agcttcaacc tcccccccag gggggggggg tagctcccgg 7620

ttcgggctcg gggtcttggt ctacttgctc cgaggaggac gataccaccg tgtgctgctc 7680

catgtcatac tcctggaccg gggctctaat aactccctgt agccccgaag aggaaaagtt 7740

gccaatcaac cctttgagta actcgctgtt gcgataccat aacaaggtgt actgtacaac 7800

atcaaagagc gcctcacaga gggctaaaaa ggtaactttt gacaggacgc aagtgctcga 7860

cgcccattat gactcagtct taaaggacat caagctagcg gcttccaagg tcagcgcaag 7920

gctcctcacc ttggaggagg cgtgccagtt gactccaccc cattctgcaa gatccaagta 7980

tggattcggg gccaaggagg tccgcagctt gtccgggagg gccgttaacc acatcaagtc 8040

cgtgtggaag gacctcctgg aagacccaca aacaccaatt cccacaacca tcatggccaa 8100

aaatgaggtg ttctgcgtgg accccgccaa ggggggtaag aaaccagctc gcctcatcgt 8160

ttaccctgac ctcggcgtcc gggtctgcga gaaaatggcc ctctatgaca ttacacaaaa 8220

gcttcctcag gcggtaatgg gagcttccta tggcttccag tactcccctg cccaacgggt 8280

ggagtatctc ttgaaagcat gggcggaaaa gaaggacccc atgggttttt cgtatgatac 8340

ccgatgcttc gactcaaccg tcactgagag agacatcagg accgaggagt ccatatacca 8400

ggcctgctcc ctgcccgagg aggcccgcac tgccatacac tcgctgactg agagacttta 8460

cgtaggaggg cccatgttca acagcaaggg tcaaacctgc ggttacagac gttgccgcgc 8520

cagcggggtg ctaaccacta gcatgggtaa caccatcaca tgctatgtga aagccctagc 8580

ggcctgcaag gctgcgggga tagttgcgcc cacaatgctg gtatgcggcg atgacctagt 8640

agtcatctca gaaagccagg ggactgagga ggacgagcgg aacctgagag ccttcacgga 8700

ggccatgacc aggtactctg cccctcctgg tgatcccccc agaccggaat atgacctgga 8760

gctaataaca tcctgttcct caaatgtgtc tgtggcgttg ggcccgcggg gccgccgcag 8820

atactacctg accagagacc caaccactcc actcgcccgg gctgcctggg aaacagttag 8880

acactcccct atcaattcat ggctgggaaa catcatccag tatgctccaa ccatatgggt 8940

tcgcatggtc ctaatgacac acttcttctc cattctcatg gtccaagaca ccctggacca 9000

gaacctcaac tttgagatgt atggatcagt atactccgtg aatcctttgg accttccagc 9060

cataattgag aggttacacg ggcttgacgc cttttctatg cacacatact ctcaccacga 9120

actgacgcgg gtggcttcag ccctcagaaa acttggggcg ccacccctca gggtgtggaa 9180

gagtcgggct cgcgcagtca gggcgtccct catctcccgt ggagggaaag cggccgtttg 9240

cggccgatat ctcttcaatt gggcggtgaa gaccaagctc aaactcactc cattgccgga 9300

ggcgcgccta ctggacttat ccagttggtt caccgtcggc gccggcgggg gcgacatttt 9360

tcacagcgtg tcgcgcgccc gaccccgctc attactcttc ggcctactcc tacttttcgt 9420

aggggtaggc ctcttcctac tccccgctcg gtagagcggc acacactagg tacactccat 9480

agctaactgt tccttttttt tttttttttt tttttttttt tttttttttt tttttctttt 9540

tttttttttt tccctctttc ttcccttctc atcttattct actttctttc ttggtggctc 9600

catcttagcc ctagtcacgg ctagctgtga aaggtccgtg agccgcatga ctgcagagag 9660

tgccgtaact ggtctctctg cagatcatgt 9690

<210> 31
<211> 9732
<212> DNA
<213> Artificial

<220>
<223> pJFH-1(3XFLAG)

<400> 31
acctgcccct aataggggcg acactccgcc atgaatcact cccctgtgag gaactactgt 60

cttcacgcag aaagcgccta gccatggcgt tagtatgagt gtcgtacagc ctccaggccc 120

ccccctcccg ggagagccat agtggtctgc ggaaccggtg agtacaccgg aattgccggg 180

aagactgggt cctttcttgg ataaacccac tctatgcccg gccatttggg cgtgcccccg 240

caagactgct agccgagtag cgttgggttg cgaaaggcct tgtggtactg cctgataggg 300

cgcttgcgag tgccccggga ggtctcgtag accgtgcacc atgagcacaa atcctaaacc 360

tcaaagaaaa accaaaagaa acaccaaccg tcgcccagaa gacgttaagt tcccgggcgg 420

cggccagatc gttggcggag tatacttgtt gccgcgcagg ggccccaggt tgggtgtgcg 480

cacgacaagg aaaacttcgg agcggtccca gccacgtggg agacgccagc ccatccccaa 540

agatcggcgc tccactggca aggcctgggg aaaaccaggt cgcccctggc ccctatatgg 600

gaatgaggga ctcggctggg caggatggct cctgtccccc cgaggctctc gcccctcctg 660

gggccccact gacccccggc ataggtcgcg caacgtgggt aaagtcatcg acaccctaac 720

gtgtggcttt gccgacctca tggggtacat ccccgtcgta ggcgccccgc ttagtggcgc 780

cgccagagct gtcgcgcacg gcgtgagagt cctggaggac ggggttaatt atgcaacagg 840

gaacctaccc ggtttcccct ttctatcttt cttgctggcc ctgttgtcct gcatcaccgt 900

tccggtctct gctgcccagg tgaagaatac cagtagcagc tacatggtga ccaatgactg 960

ctccaatgac agcatcactt ggcagctcga ggctgcggtt ctccacgtcc ccgggtgcgt 1020

cccgtgcgag agagtgggga atacgtcacg gtgttgggtg ccagtctcgc caaacatggc 1080

tgtgcggcag cccggtgccc tcacgcaggg tctgcggacg cacatcgata tggttgtgat 1140

gtccgccacc ttctgctctg ctctctacgt gggggacctc tgtggcgggg tgatgctcgc 1200

ggcccaggtg ttcatcgtct cgccgcagta ccactggttt gtgcaagaat gcaattgctc 1260

catctaccct ggcaccatca ctggacaccg catggcatgg gacatgatga tgaactggtc 1320

gcccacggcc accatgatcc tggcgtacgt gatgcgcgtc cccgaggtca tcatagacat 1380

cgttagcggg gctcactggg gcgtcatgtt cggcttggcc tacttctcta tgcagggagc 1440

gtgggcgaag gtcattgtca tccttctgct ggccgctggg gtggacgcgg gcaccaccac 1500

cgttggaggc gctgttgcag actacaaaga ccatgacggt gattataaag atcatgacat 1560

cgattacaag gatgacgatg acaagggagg cggtggcgtg ttcagccatg gccctcagca 1620

gaacattcag ctcattaaca ccaacggcag ttggcacatc aaccgtactg ccttgaattg 1680

caatgactcc ttgaacaccg gctttctcgc ggccttgttc tacaccaacc gctttaactc 1740

gtcagggtgt ccagggcgcc tgtccgcctg ccgcaacatc gaggctttcc ggatagggtg 1800

gggcacccta cagtacgagg ataatgtcac caatccagag gatatgaggc cgtactgctg 1860

gcactacccc ccaaagccgt gtggcgtagt ccccgcgagg tctgtgtgtg gcccagtgta 1920

ctgtttcacc cccagcccgg tagtagtggg cacgaccgac agacgtggag tgcccaccta 1980

cacatgggga gagaatgaga cagatgtctt cctactgaac agcacccgac cgccgcaggg 2040

ctcatggttc ggctgcacgt ggatgaactc cactggtttc accaagactt gtggcgcgcc 2100

accttgccgc accagagctg acttcaacgc cagcacggac ttgttgtgcc ctacggattg 2160

ttttaggaag catcctgatg ccacttatat taagtgtggt tctgggccct ggctcacacc 2220

aaagtgcctg gtccactacc cttacagact ctggcattac ccctgcacag tcaattttac 2280

catcttcaag ataagaatgt atgtaggggg ggttgagcac aggctcacgg ccgcatgcaa 2340

cttcactcgt ggggatcgct gcgacttgga ggacagggac aggagtcagc tgtctcctct 2400

gttgcactct accacggaat gggccatcct gccctgcacc tactcagact tacccgcttt 2460

gtcaactggt cttctccacc ttcaccagaa catcgtggac gtacaataca tgtatggcct 2520

ctcacctgct atcacaaaat acgtcgttcg atgggagtgg gtggtactct tattcctgct 2580

cttagcggac gccagagtct gcgcctgctt gtggatgctc atcttgttgg gccaggccga 2640

agcagcattg gagaagttgg tcgtcttgca cgctgcgagt gcggctaact gccatggcct 2700

cctatatttt gccatcttct tcgtggcagc ttggcacatc aggggtcggg tggtcccctt 2760

gaccacctat tgcctcactg gcctatggcc cttctgccta ctgctcatgg cactgccccg 2820

gcaggcttat gcctatgacg cacctgtgca cggacagata ggcgtgggtt tgttgatatt 2880

gatcaccctc ttcacactca ccccggggta taagaccctc ctcggccagt gtctgtggtg 2940

gttgtgctat ctcctgaccc tgggggaagc catgattcag gagtgggtac cacccatgca 3000

ggtgcgcggc ggccgcgatg gcatcgcgtg ggccgtcact atattctgcc cgggtgtggt 3060

gtttgacatt accaaatggc ttttggcgtt gcttgggcct gcttacctct taagggccgc 3120

tttgacacat gtgccgtact tcgtcagagc tcacgctctg ataagggtat gcgctttggt 3180

gaagcagctc gcggggggta ggtatgttca ggtggcgcta ttggcccttg gcaggtggac 3240

tggcacctac atctatgacc acctcacacc tatgtcggac tgggccgcta gcggcctgcg 3300

cgacttagcg gtcgccgtgg aacccatcat cttcagtccg atggagaaga aggtcatcgt 3360

ctggggagcg gagacggctg catgtgggga cattctacat ggacttcccg tgtccgcccg 3420

actcggccag gagatcctcc tcggcccagc tgatggctac acctccaagg ggtggaagct 3480

ccttgctccc atcactgctt atgcccagca aacacgaggc ctcctgggcg ccatagtggt 3540

gagtatgacg gggcgtgaca ggacagaaca ggccggggaa gtccaaatcc tgtccacagt 3600

ctctcagtcc ttcctcggaa caaccatctc ggggggtttg tggactgttt accacggagc 3660

tggcaacaag actctagccg gcttacgggg tccggtcacg cagatgtact cgagtgctga 3720

gggggacttg gtaggctggc ccagcccccc tgggaccaag tctttggagc cgtgcaagtg 3780

tggagccgtc gacctatatc tggtcacgcg gaacgctgat gtcatcccgg ctcggagacg 3840

cggggacaag cggggagcat tgctctcccc gagacccatt tcgaccttga aggggtcctc 3900

gggggggccg gtgctctgcc ctaggggcca cgtcgttggg ctcttccgag cagctgtgtg 3960

ctctcggggc gtggccaaat ccatcgattt catccccgtt gagacactcg acgttgttac 4020

aaggtctccc actttcagtg acaacagcac gccaccggct gtgccccaga cctatcaggt 4080

cgggtacttg catgctccaa ctggcagtgg aaagagcacc aaggtccctg tcgcgtatgc 4140

cgcccagggg tacaaagtac tagtgcttaa cccctcggta gctgccaccc tggggtttgg 4200

ggcgtaccta tccaaggcac atggcatcaa tcccaacatt aggactggag tcaggaccgt 4260

gatgaccggg gaggccatca cgtactccac atatggcaaa tttctcgccg atgggggctg 4320

cgctagcggc gcctatgaca tcatcatatg cgatgaatgc cacgctgtgg atgctacctc 4380

cattctcggc atcggaacgg tccttgatca agcagagaca gccggggtca gactaactgt 4440

gctggctacg gccacacccc ccgggtcagt gacaaccccc catcccgata tagaagaggt 4500

aggcctcggg cgggagggtg agatcccctt ctatgggagg gcgattcccc tatcctgcat 4560

caagggaggg agacacctga ttttctgcca ctcaaagaaa aagtgtgacg agctcgcggc 4620

ggcccttcgg ggcatgggct tgaatgccgt ggcatactat agagggttgg acgtctccat 4680

aataccagct cagggagatg tggtggtcgt cgccaccgac gccctcatga cggggtacac 4740

tggagacttt gactccgtga tcgactgcaa tgtagcggtc acccaagctg tcgacttcag 4800

cctggacccc accttcacta taaccacaca gactgtccca caagacgctg tctcacgcag 4860

tcagcgccgc gggcgcacag gtagaggaag acagggcact tataggtatg tttccactgg 4920

tgaacgagcc tcaggaatgt ttgacagtgt agtgctttgt gagtgctacg acgcaggggc 4980

tgcgtggtac gatctcacac cagcggagac caccgtcagg cttagagcgt atttcaacac 5040

gcccggccta cccgtgtgtc aagaccatct tgaattttgg gaggcagttt tcaccggcct 5100

cacacacata gacgcccact tcctctccca aacaaagcaa gcgggggaga acttcgcgta 5160

cctagtagcc taccaagcta cggtgtgcgc cagagccaag gcccctcccc cgtcctggga 5220

cgccatgtgg aagtgcctgg cccgactcaa gcctacgctt gcgggcccca cacctctcct 5280

gtaccgtttg ggccctatta ccaatgaggt caccctcaca caccctggga cgaagtacat 5340

cgccacatgc atgcaagctg accttgaggt catgaccagc acgtgggtcc tagctggagg 5400

agtcctggca gccgtcgccg catattgcct ggcgactgga tgcgtttcca tcatcggccg 5460

cttgcacgtc aaccagcgag tcgtcgttgc gccggataag gaggtcctgt atgaggcttt 5520

tgatgagatg gaggaatgcg cctctagggc ggctctcatc gaagaggggc agcggatagc 5580

cgagatgttg aagtccaaga tccaaggctt gctgcagcag gcctctaagc aggcccagga 5640

catacaaccc gctatgcagg cttcatggcc caaagtggaa caattttggg ccagacacat 5700

gtggaacttc attagcggca tccaatacct cgcaggattg tcaacactgc cagggaaccc 5760

cgcggtggct tccatgatgg cattcagtgc cgccctcacc agtccgttgt cgaccagtac 5820

caccatcctt ctcaacatca tgggaggctg gttagcgtcc cagatcgcac cacccgcggg 5880

ggccaccggc tttgtcgtca gtggcctggt gggggctgcc gtgggcagca taggcctggg 5940

taaggtgctg gtggacatcc tggcaggata tggtgcgggc atttcggggg ccctcgtcgc 6000

attcaagatc atgtctggcg agaagccctc tatggaagat gtcatcaatc tactgcctgg 6060

gatcctgtct ccgggagccc tggtggtggg ggtcatctgc gcggccattc tgcgccgcca 6120

cgtgggaccg ggggagggcg cggtccaatg gatgaacagg cttattgcct ttgcttccag 6180

aggaaaccac gtcgccccta ctcactacgt gacggagtcg gatgcgtcgc agcgtgtgac 6240

ccaactactt ggctctctta ctataaccag cctactcaga agactccaca attggataac 6300

tgaggactgc cccatcccat gctccggatc ctggctccgc gacgtgtggg actgggtttg 6360

caccatcttg acagacttca aaaattggct gacctctaaa ttgttcccca agctgcccgg 6420

cctccccttc atctcttgtc aaaaggggta caagggtgtg tgggccggca ctggcatcat 6480

gaccacgcgc tgcccttgcg gcgccaacat ctctggcaat gtccgcctgg gctctatgag 6540

gatcacaggg cctaaaacct gcatgaacac ctggcagggg acctttccta tcaattgcta 6600

cacggagggc cagtgcgcgc cgaaaccccc cacgaactac aagaccgcca tctggagggt 6660

ggcggcctcg gagtacgcgg aggtgacgca gcatgggtcg tactcctatg taacaggact 6720

gaccactgac aatctgaaaa ttccttgcca actaccttct ccagagtttt tctcctgggt 6780

ggacggtgtg cagatccata ggtttgcacc cacaccaaag ccgtttttcc gggatgaggt 6840

ctcgttctgc gttgggctta attcctatgc tgtcgggtcc cagcttccct gtgaacctga 6900

gcccgacgca gacgtattga ggtccatgct aacagatccg ccccacatca cggcggagac 6960

tgcggcgcgg cgcttggcac ggggatcacc tccatctgag gcgagctcct cagtgagcca 7020

gctatcagca ccgtcgctgc gggccacctg caccacccac agcaacacct atgacgtgga 7080

catggtcgat gccaacctgc tcatggaggg cggtgtggct cagacagagc ctgagtccag 7140

ggtgcccgtt ctggactttc tcgagccaat ggccgaggaa gagagcgacc ttgagccctc 7200

aataccatcg gagtgcatgc tccccaggag cgggtttcca cgggccttac cggcttgggc 7260

acggcctgac tacaacccgc cgctcgtgga atcgtggagg aggccagatt accaaccgcc 7320

caccgttgct ggttgtgctc tccccccccc caagaaggcc ccgacgcctc ccccaaggag 7380

acgccggaca gtgggtctga gcgagagcac catatcagaa gccctccagc aactggccat 7440

caagaccttt ggccagcccc cctcgagcgg tgatgcaggc tcgtccacgg gggcgggcgc 7500

cgccgaatcc ggcggtccga cgtcccctgg tgagccggcc ccctcagaga caggttccgc 7560

ctcctctatg ccccccctcg agggggagcc tggagatccg gacctggagt ctgatcaggt 7620

agagcttcaa cctccccccc aggggggggg ggtagctccc ggttcgggct cggggtcttg 7680

gtctacttgc tccgaggagg acgataccac cgtgtgctgc tccatgtcat actcctggac 7740

cggggctcta ataactccct gtagccccga agaggaaaag ttgccaatca accctttgag 7800

taactcgctg ttgcgatacc ataacaaggt gtactgtaca acatcaaaga gcgcctcaca 7860

gagggctaaa aaggtaactt ttgacaggac gcaagtgctc gacgcccatt atgactcagt 7920

cttaaaggac atcaagctag cggcttccaa ggtcagcgca aggctcctca ccttggagga 7980

ggcgtgccag ttgactccac cccattctgc aagatccaag tatggattcg gggccaagga 8040

ggtccgcagc ttgtccggga gggccgttaa ccacatcaag tccgtgtgga aggacctcct 8100

ggaagaccca caaacaccaa ttcccacaac catcatggcc aaaaatgagg tgttctgcgt 8160

ggaccccgcc aaggggggta agaaaccagc tcgcctcatc gtttaccctg acctcggcgt 8220

ccgggtctgc gagaaaatgg ccctctatga cattacacaa aagcttcctc aggcggtaat 8280

gggagcttcc tatggcttcc agtactcccc tgcccaacgg gtggagtatc tcttgaaagc 8340

atgggcggaa aagaaggacc ccatgggttt ttcgtatgat acccgatgct tcgactcaac 8400

cgtcactgag agagacatca ggaccgagga gtccatatac caggcctgct ccctgcccga 8460

ggaggcccgc actgccatac actcgctgac tgagagactt tacgtaggag ggcccatgtt 8520

caacagcaag ggtcaaacct gcggttacag acgttgccgc gccagcgggg tgctaaccac 8580

tagcatgggt aacaccatca catgctatgt gaaagcccta gcggcctgca aggctgcggg 8640

gatagttgcg cccacaatgc tggtatgcgg cgatgaccta gtagtcatct cagaaagcca 8700

ggggactgag gaggacgagc ggaacctgag agccttcacg gaggccatga ccaggtactc 8760

tgcccctcct ggtgatcccc ccagaccgga atatgacctg gagctaataa catcctgttc 8820

ctcaaatgtg tctgtggcgt tgggcccgcg gggccgccgc agatactacc tgaccagaga 8880

cccaaccact ccactcgccc gggctgcctg ggaaacagtt agacactccc ctatcaattc 8940

atggctggga aacatcatcc agtatgctcc aaccatatgg gttcgcatgg tcctaatgac 9000

acacttcttc tccattctca tggtccaaga caccctggac cagaacctca actttgagat 9060

gtatggatca gtatactccg tgaatccttt ggaccttcca gccataattg agaggttaca 9120

cgggcttgac gccttttcta tgcacacata ctctcaccac gaactgacgc gggtggcttc 9180

agccctcaga aaacttgggg cgccacccct cagggtgtgg aagagtcggg ctcgcgcagt 9240

cagggcgtcc ctcatctccc gtggagggaa agcggccgtt tgcggccgat atctcttcaa 9300

ttgggcggtg aagaccaagc tcaaactcac tccattgccg gaggcgcgcc tactggactt 9360

atccagttgg ttcaccgtcg gcgccggcgg gggcgacatt tttcacagcg tgtcgcgcgc 9420

ccgaccccgc tcattactct tcggcctact cctacttttc gtaggggtag gcctcttcct 9480

actccccgct cggtagagcg gcacacacta ggtacactcc atagctaact gttccttttt 9540

tttttttttt tttttttttt tttttttttt tttttttctt tttttttttt tttccctctt 9600

tcttcccttc tcatcttatt ctactttctt tcttggtggc tccatcttag ccctagtcac 9660

ggctagctgt gaaaggtccg tgagccgcat gactgcagag agtgccgtaa ctggtctctc 9720

tgcagatcat gt                                                          9732

<210>  32
<211>  30
<212>  PRT
<213>  Artificial

<220>
<223>  N terminal sequence of E2 protein encoded in pTH/JFH1

<400>  32

Ala Thr Tyr Val Thr Gly Gly Ser Glu Ala Arg Gly Ala Ser Gly Leu
1               5                   10                  15

Ala Asn Leu Phe Ser Phe Gly Ala Ser Gln Lys Ile Gln Leu
            20                  25                  30

<210>  33
<211>  34
<212>  PRT
<213>  Artificial

<220>
<223>  N terminal sequence of E2 protein encoded in pTH/JFH-1(1XFLAG)

<400>  33

Ala Thr Tyr Val Thr Gly Gly Ser Glu Ala Asp Tyr Lys Asp Asp Asp
1               5                   10                  15

Asp Lys Gly Gly Gly Asn Leu Phe Ser Phe Gly Ala Ser Gln Lys Ile
            20                  25                  30

Gln Leu

<210> 34
<211> 48
<212> PRT
<213> Artificial

<220>
<223> N terminal sequence of E2 protein encoded in pTH/JFH-1(3XFLAG)

<400> 34

Ala Thr Tyr Val Thr Gly Gly Ser Glu Ala Asp Tyr Lys Asp His Asp
1               5                   10                  15

Gly Asp Tyr Lys Asp His Asp Ile Asp Tyr Lys Asp Asp Asp Asp Lys
            20                  25                  30

Gly Gly Gly Asn Leu Phe Ser Phe Gly Ala Ser Gln Lys Ile Gln Leu
        35                  40                  45

<210> 35
<211> 20
<212> DNA
<213> Artificial

<220>
<223> primer(TH-F)

<400> 35
aggcgacaac ctatccccaa                                                20

<210> 36
<211> 44
<212> DNA
<213> Artificial

<220>

<223>  primer(TH-1F-R)

<400>  36
cccttgtcat cgtcatcctt gtaatcggct tccgaccccc ccgt                44

<210>  37
<211>  44
<212>  DNA
<213>  Artificial

<220>
<223>  primer(TH-1F-F)

<400>  37
aggatgacga tgacaaggga ggcggtaacc tcttttcatt tggg                44

<210>  38
<211>  65
<212>  DNA
<213>  Artificial

<220>
<223>  primer(TH-3F-R)

<400>  38
taatcgatgt catgatcttt ataatcaccg tcatggtctt tgtagtcggc ttccgacccc      60

cccgt                                                                  65

<210>  39
<211>  65
<212>  DNA
<213>  Artificial

<220>
<223>  primer(TH-3F-F)

<400>  39
aagatcatga catcgattac aaggatgacg atgacaaggg aggcggtaac ctcttttcat      60

ttggg 65

<210> 40
<211> 9669
<212> DNA
<213> Artificial

<220>
<223> pTH/JFH1

<400> 40
   acctgcccct aataggggcg acactccgcc atgaatcact cccctgtgag gaactactgt 60
   cttcacgcag aaagcgccta gccatggcgt tagtatgagt gtcgtacagc ctccaggccc 120
   ccccctcccg ggagagccat agtggtctgc ggaaccggtg agtacaccgg aattgccggg 180
   aagactgggt cctttcttgg ataaacccac tctatgcccg gccatttggg cgtgcccccg 240
   caagactgct agccgagtag cgttgggttg cgaaaggcct tgtggtactg cctgataggg 300
   cgcttgcgag tgccccggga ggtctcgtag accgtgcacc atgagcacga atcctaaacc 360
   tcaaagaaaa accaaacgta acaccaaccg ccgcccacag gacgtcaagt tcccgggcgg 420
   tggccagatc gttggtggag tttacctgtt gccgcgcagg ggccccaggt tgggtgtgcg 480
   cgcgactagg aagacttccg agcggtcgca acctcgtgga aggcgacaac ctatccccaa 540
   ggatcgccga cccgagggca gggcctgggc tcagcctggg tacccttggc ccctctatgg 600
   caacgagggc atggggtggg caggatggct cctgtcaccc cgtggctccc ggcctagttg     660
   gggccccaat gacccccggc gcaggtcgcg taatttgggt aaagtcatcg atacccttac     720
   atgcggcttc gccgacctca tggggtacat tccgctcgtc ggcgctccct tggggggcgc 780
   tgccagggcc ttggcgcatg gcgtccgggt tctggaggac ggcgtgaact atgcaacagg 840
   gaatctgccc ggttgctctt tctctatctt cctcttggct ctgctgtcct gtctaaccat 900
   cccagcttcc gcttatgaag tgcgcaacgt gtccggggtg taccatgtca cgaacgactg 960
   ctccaactcg agcattgtgt acgagacagg ggacatgatt atgcacaccc ctgggtgcgt 1020
   gccctgtgtt cgggagaaca actcctcccg ctgctgggca gcgctcactc ccacgctcgc 1080
   ggccaggaac gccagcgtcc ccaccacgac aatacggcgc cacgtcgatt tgctcgttgg 1140
   ggcggctgct ttctgctccg ctatgtacgt gggggatctc tgcggatctg ttttcctcgt 1200
   ctcccagttg ttcaccttct cgcctcgccg gcatgagaca gtgcaggact gcaattgttc 1260
   aatctatccc ggccacgtat caggtcaccg catggcttgg gatatgatga tgaactggtc 1320
   acctacaaca gccctactgg tatcgcagtt actccggatc ccacaagccg tcgtggacat 1380
   ggtggcgggg gcccactggg gagtcctggc gggccttgcc tactattcca tggcggggaa 1440
   ctgggctaag gttttgattg tgctgctact ctttgccggc gttgatgggg cgacctacgt 1500
   gacggggggg tcggaagcca gaggggcctc tggcttagca aacctctttt catttggggc 1560
   gtctcagaag atccagctca taaataccaa cggcagttgg cacatcaata gaactgccct 1620
   gaactgcaat gactccctcc acactgggtt tcttgccgcg ctattctaca cacacaaatt 1680
   caacgcgtcc ggatgtccag agcgcatggc cagctgccgc cccattgaag agttcgctca 1740
   ggggtatggt cccatcactt atgctgagcc ctccccctcg gaccagaggc cctattgctg 1800
   gcactacgcg cctcgaccgt gtggtatcat acccgcgtcg caggtgtgtg gtccagtgta 1860
   ctgcttcacc ccaagccctg ttgtggtggg gacgaccgat cgctccggtg cccccacgta 1920
   taattggggg gcgaatgaga cggacgtgct gtatctcaac aacacgcggc cgccgcaagg 1980
   caactggttc ggctgcacat ggatgaatgg caccgggttc accaagacgt gcgggggccc 2040
   cccgtgcaac atcggggggg gcggcaacaa caacaccttg acctgcccca cggactgttt 2100
   ccggaaacac cccgaggcca cctacaccaa atgtggttcg ggaccttggt tgacacctag 2160
   gtgcatggtc gactacccat acaggctctg gcactacccc tgcaccgtta actttaccat 2220
   ctttaaggtt aggatgtacg tgggaggtgt ggagcacagg ctcaacgccg catgcaattg 2280
   gacccgagga gagcgttgta acttagagga cagggataga tcagagctta gcccgctgct 2340
   gctgtcaaca acagagtggc aggtgctacc ttgttccttc accaccctac cggctctgtc 2400
   cactggtttg atccatctcc accagaacat cgtggacgtg caatacctgt acggtatagg 2460
   gtcggcggtt gtctcctatg caatcaaatg ggaatatgtc ttgttgctct tcctcctcct 2520
   ggcagacgcg cgcgtctgcg cctgcttgtg gatgatgctg ctgatagctc aagctgaggc 2580
   cgccttagag aacctggtgg tcctcaatgc ggcgtccctg gctggagcgc atggccttct 2640
   ctctttcctt gtgttcttct gtgccgcttg gtacatcaag ggcaggttga tccccggggc 2700
   ggcgtatgct ttttacggcg tatggccgct gctcctactc ctgctggcgt taccaccacg 2760
   agcatacgcc atggaccggg agatggctgc atcgtgcgga ggcgcggttt ttgtaggtct 2820
   ggcattcctg accttgtcac cacactataa ggcattcctc gccaagctcc tgtggtggtt 2880
   gtgctatctc ctgaccctgg gggaagccat gattcaggag tgggtaccac ccatgcaggt 2940
   gcgcggcggc cgcgatggca tcgcgtgggc cgtcactata ttctgcccgg gtgtggtgtt 3000
   tgacattacc aaatggcttt tggcgttgct tgggcctgct tacctcttaa gggccgcttt 3060
   gacacatgtg ccgtacttcg tcagagctca cgctctgata agggtatgcg ctttggtgaa 3120
   gcagctcgcg gggggtaggt atgttcaggt ggcgctattg gcccttggca ggtggactgg 3180
   cacctacatc tatgaccacc tcacacctat gtcggactgg gccgctagcg gcctgcgcga 3240
   cttagcggtc gccgtggaac ccatcatctt cagtccgatg gagaagaagg tcatcgtctg 3300
   gggagcggag acggctgcat gtggggacat tctacatgga cttcccgtgt ccgcccgact 3360
   cggccaggag atcctcctcg gcccagctga tggctacacc tccaaggggt ggaagctcct 3420
   tgctcccatc actgcttatg cccagcaaac acgaggcctc ctgggcgcca tagtggtgag 3480
   tatgacgggg cgtgacagga cagaacaggc cggggaagtc caaatcctgt ccacagtctc 3540
   tcagtccttc ctcggaacaa ccatctcggg ggttttgtgg actgtttacc acggagctgg 3600
   caacaagact ctagccggct tacggggtcc ggtcacgcag atgtactcga gtgctgaggg 3660
   ggacttggta ggctggccca gcccccctgg gaccaagtct ttggagccgt gcaagtgtgg 3720
   agccgtcgac ctatatctgg tcacgcggaa cgctgatgtc atcccggctc ggagacgcgg 3780
   ggacaagcgg ggagcattgc tctccccgag acccatttcg accttgaagg ggtcctcggg 3840
   ggggccggtg ctctgcccta ggggccacgt cgttgggctc ttccgagcag ctgtgtgctc 3900
   tcggggcgtg gccaaatcca tcgatttcat ccccgttgag acactcgacg ttgttacaag 3960
   gtctcccact ttcagtgaca acagcacgcc accggctgtg ccccagacct atcaggtcgg 4020
   gtacttgcat gctccaactg gcagtggaaa gagcaccaag gtccctgtcg cgtatgccgc 4080
   ccaggggtac aaagtactag tgcttaaccc ctcggtagct gccaccctgg ggtttggggc 4140
   gtacctatcc aaggcacatg gcatcaatcc caacattagg actggagtca ggaccgtgat 4200
   gaccggggag gccatcacgt actccacata tggcaaattt ctcgccgatg ggggctgcgc 4260
   tagcggcgcc tatgacatca tcatatgcga tgaatgccac gctgtggatg ctacctccat 4320
   tctcggcatc ggaacggtcc ttgatcaagc agagacagcc ggggtcagac taactgtgct 4380
   ggctacggcc acaccccccg ggtcagtgac aaccccccat cccgatatag aagaggtagg 4440
   cctcgggcgg gagggtgaga tccccttcta tgggagggcg attcccctat cctgcatcaa 4500
   gggagggaga cacctgattt tctgccactc aaagaaaaag tgtgacgagc tcgcggcggc 4560
   ccttcggggc atgggcttga atgccgtggc atactataga gggttggacg tctccataat 4620
   accagctcag ggagatgtgg tggtcgtcgc caccgacgcc ctcatgacgg ggtacactgg 4680
   agactttgac tccgtgatcg actgcaatgt agcggtcacc caagctgtcg acttcagcct 4740
   ggaccccacc ttcactataa ccacacagac tgtcccacaa gacgctgtct cacgcagtca 4800
   gcgccgcggg cgcacaggta gaggaagaca gggcacttat aggtatgttt ccactggtga 4860
   acgagcctca ggaatgtttg acagtgtagt gctttgtgag tgctacgacg caggggctgc 4920
   gtggtacgat ctcacaccag cggagaccac cgtcaggctt agagcgtatt tcaacacgcc 4980
   cggcctaccc gtgtgtcaag accatcttga attttgggag gcagttttca ccggcctcac 5040
   acacatagac gcccacttcc tctcccaaac aaagcaagcg ggggagaact tcgcgtacct 5100
   agtagcctac caagctacgg tgtgcgccag agccaaggcc cctcccccgt cctgggacgc 5160
   catgtggaag tgcctggccc gactcaagcc tacgcttgcg ggccccacac ctctcctgta 5220
   ccgtttgggc cctattacca atgaggtcac cctcacacac cctgggacga agtacatcgc 5280
   cacatgcatg caagctgacc ttgaggtcat gaccagcacg tgggtcctag ctggaggagt 5340
   cctggcagcc gtcgccgcat attgcctggc gactggatgc gtttccatca tcggccgctt 5400
   gcacgtcaac cagcgagtcg tcgttgcgcc ggataaggag gtcctgtatg aggcttttga 5460
   tgagatggag gaatgcgcct ctagggcggc tctcatcgaa gaggggcagc ggatagccga 5520
   gatgttgaag tccaagatcc aaggcttgct gcagcaggcc tctaagcagg cccaggacat 5580
   acaacccgct atgcaggctt catggcccaa agtggaacaa ttttgggcca gacacatgtg 5640
   gaacttcatt agcggcatcc aatacctcgc aggattgtca acactgccag ggaaccccgc 5700
   ggtggcttcc atgatggcat tcagtgccgc cctcaccagt ccgttgtcga ccagtaccac 5760
   catccttctc aacatcatgg gaggctggtt agcgtcccag atcgcaccac ccgcgggggc 5820
   caccggcttt gtcgtcagtg gcctggtggg ggctgccgtg ggcagcatag gcctgggtaa 5880
   ggtgctggtg gacatcctgg caggatatgg tgcgggcatt tcgggggccc tcgtcgcatt 5940
   caagatcatg tctggcgaga agccctctat ggaagatgtc atcaatctac tgcctgggat 6000
   cctgtctccg ggagccctgg tggtgggggt catctgcgcg gccattctgc gccgccacgt 6060
   gggaccgggg gagggcgcgg tccaatggat gaacaggctt attgcctttg cttccagagg 6120
   aaaccacgtc gcccctactc actacgtgac ggagtcggat gcgtcgcagc gtgtgaccca 6180
   actacttggc tctcttacta taaccagcct actcagaaga ctccacaatt ggataactga 6240
   ggactgcccc atcccatgct ccggatcctg gctccgcgac gtgtgggact gggtttgcac 6300
   catcttgaca gacttcaaaa attggctgac ctctaaattg ttccccaagc tgcccggcct 6360
   ccccttcatc tcttgtcaaa aggggtacaa gggtgtgtgg gccggcactg gcatcatgac 6420
   cacgcgctgc ccttgcggcg ccaacatctc tggcaatgtc cgcctgggct ctatgaggat 6480
   cacagggcct aaaacctgca tgaacacctg gcaggggacc tttcctatca attgctacac 6540
   ggagggccag tgcgcgccga aaccccccac gaactacaag accgccatct ggagggtggc 6600
   ggcctcggag tacgcggagg tgacgcagca tgggtcgtac tcctatgtaa caggactgac 6660
   cactgacaat ctgaaaattc cttgccaact accttctcca gagtttttct cctgggtgga 6720
   cggtgtgcag atccataggt ttgcacccac accaaagccg tttttccggg atgaggtctc 6780
   gttctgcgtt gggcttaatt cctatgctgt cgggtcccag cttccctgtg aacctgagcc 6840
   cgacgcagac gtattgaggt ccatgctaac agatccgccc cacatcacgg cggagactgc 6900
   ggcgcggcgc ttggcacggg gatcacctcc atctgaggcg agctcctcag tgagccagct 6960
   atcagcaccg tcgctgcggg ccacctgcac cacccacagc aacacctatg acgtggacat 7020
   ggtcgatgcc aacctgctca tggagggcgg tgtggctcag acagagcctg agtccagggt 7080
   gcccgttctg gactttctcg agccaatggc cgaggaagag agcgaccttg agccctcaat 7140
   accatcggag tgcatgctcc ccaggagcgg gtttccacgg gccttaccgg cttgggcacg 7200
   gcctgactac aacccgccgc tcgtggaatc gtggaggagg ccagattacc aaccgcccac 7260
   cgttgctggt tgtgctctcc ccccccccaa gaaggccccg acgcctcccc caaggagacg 7320
   ccggacagtg ggtctgagcg agagcaccat atcagaagcc ctccagcaac tggccatcaa 7380
   gacctttggc cagcccccct cgagcggtga tgcaggctcg tccacggggg cgggcgccgc 7440
   cgaatccggc ggtccgacgt cccctggtga gccggccccc tcagagacag gttccgcctc 7500
   ctctatgccc cccctcgagg gggagcctgg agatccggac ctggagtctg atcaggtaga 7560
   gcttcaacct cccccccagg gggggggggt agctcccggt tcgggctcgg ggtcttggtc 7620
   tacttgctcc gaggaggacg ataccaccgt gtgctgctcc atgtcatact cctggaccgg 7680
   ggctctaata actccctgta gccccgaaga ggaaaagttg ccaatcaacc ctttgagtaa 7740
   ctcgctgttg cgataccata acaaggtgta ctgtacaaca tcaaagagcg cctcacagag 7800
   ggctaaaaag gtaacttttg acaggacgca agtgctcgac gcccattatg actcagtctt 7860
   aaaggacatc aagctagcgg cttccaaggt cagcgcaagg ctcctcacct tggaggaggc 7920
   gtgccagttg actccacccc attctgcaag atccaagtat ggattcgggg ccaaggaggt 7980
   ccgcagcttg tccgggaggg ccgttaacca catcaagtcc gtgtggaagg acctcctgga 8040
   agacccacaa acaccaattc ccacaaccat catggccaaa aatgaggtgt tctgcgtgga 8100
   ccccgccaag gggggtaaga aaccagctcg cctcatcgtt taccctgacc tcggcgtccg 8160
   ggtctgcgag aaaatggccc tctatgacat tacacaaaag cttcctcagg cggtaatggg 8220
   agcttcctat ggcttccagt actcccctgc ccaacgggtg gagtatctct tgaaagcatg 8280
   ggcggaaaag aaggacccca tgggtttttc gtatgatacc cgatgcttcg actcaaccgt 8340
   cactgagaga gacatcagga ccgaggagtc catataccag gcctgctccc tgcccgagga 8400
   ggcccgcact gccatacact cgctgactga gagactttac gtaggagggc ccatgttcaa 8460
   cagcaagggt caaacctgcg gttacagacg ttgccgcgcc agcggggtgc taaccactag 8520
   catgggtaac accatcacat gctatgtgaa agccctagcg gcctgcaagg ctgcggggat 8580
   agttgcgccc acaatgctgg tatgcggcga tgacctagta gtcatctcag aaagccaggg 8640
   gactgaggag gacgagcgga acctgagagc cttcacggag gccatgacca ggtactctgc 8700
   ccctcctggt gatcccccca gaccggaata tgacctggag ctaataacat cctgttcctc 8760
   aaatgtgtct gtggcgttgg gcccgcgggg ccgccgcaga tactacctga ccagagaccc 8820
   aaccactcca ctcgcccggg ctgcctggga aacagttaga cactccccta tcaattcatg 8880
   gctgggaaac atcatccagt atgctccaac catatgggtt cgcatggtcc taatgacaca 8940
   cttcttctcc attctcatgg tccaagacac cctggaccag aacctcaact ttgagatgta 9000
   tggatcagta tactccgtga atcctttgga ccttccagcc ataattgaga ggttacacgg 9060
   gcttgacgcc ttttctatgc acacatactc tcaccacgaa ctgacgcggg tggcttcagc 9120
   cctcagaaaa cttggggcgc cacccctcag ggtgtggaag agtcgggctc gcgcagtcag 9180
   ggcgtccctc atctcccgtg gagggaaagc ggccgtttgc ggccgatatc tcttcaattg 9240
   ggcggtgaag accaagctca aactcactcc attgccggag gcgcgcctac tggacttatc 9300
   cagttggttc accgtcggcg ccggcggggg cgacattttt cacagcgtgt cgcgcgcccg 9360
   accccgctca ttactcttcg gcctactcct acttttcgta ggggtaggcc tcttcctact 9420
   ccccgctcgg tagagcggca cacactaggt acactccata gctaactgtt cctttttttt 9480
   tttttttttt tttttttttt tttttttttt tttttctttt tttttttttt ccctctttct 9540
   tcccttctca tcttattcta ctttctttct tggtggctcc atcttagccc tagtcacggc 9600
   tagctgtgaa aggtccgtga gccgcatgac tgcagagagt gccgtaactg gtctctctgc 9660
   agatcatgt 9669

<210> 41
<211> 9681
<212> DNA
<213> Artificial

<220>
<223> pTH/JFH1(1XFLAG)

<400> 41
   acctgcccct aataggggcg acactccgcc atgaatcact cccctgtgag gaactactgt 60
   cttcacgcag aaagcgccta gccatggcgt tagtatgagt gtcgtacagc ctccaggccc 120
   ccccctcccg ggagagccat agtggtctgc ggaaccggtg agtacaccgg aattgccggg 180
   aagactgggt cctttcttgg ataaacccac tctatgcccg gccatttggg cgtgcccccg 240
   caagactgct agccgagtag cgttgggttg cgaaaggcct tgtggtactg cctgataggg 300
   cgcttgcgag tgccccggga ggtctcgtag accgtgcacc atgagcacga atcctaaacc 360
   tcaaagaaaa accaaacgta acaccaaccg ccgcccacag gacgtcaagt tcccgggcgg 420
   tggccagatc gttggtggag tttacctgtt gccgcgcagg ggccccaggt tgggtgtgcg 480
   cgcgactagg aagacttccg agcggtcgca acctcgtgga aggcgacaac ctatccccaa 540
   ggatcgccga cccgagggca gggcctgggc tcagcctggg tacccttggc ccctctatgg 600
   caacgagggc atggggtggg caggatggct cctgtcaccc cgtggctccc ggcctagttg 660
   gggccccaat gacccccggc gcaggtcgcg taatttgggt aaagtcatcg atacccttac 720
   atgcggcttc gccgacctca tggggtacat tccgctcgtc ggcgctccct tggggggcgc 780
   tgccagggcc ttggcgcatg gcgtccgggt tctggaggac ggcgtgaact atgcaacagg 840
   gaatctgccc ggttgctctt tctctatctt cctcttggct ctgctgtcct gtctaaccat 900
   cccagcttcc gcttatgaag tgcgcaacgt gtccggggtg taccatgtca cgaacgactg 960
   ctccaactcg agcattgtgt acgagacagg ggacatgatt atgcacaccc ctgggtgcgt 1020
   gccctgtgtt cgggagaaca actcctcccg ctgctgggca gcgctcactc ccacgctcgc 1080
   ggccaggaac gccagcgtcc ccaccacgac aatacggcgc cacgtcgatt tgctcgttgg 1140
   ggcggctgct ttctgctccg ctatgtacgt gggggatctc tgcggatctg ttttcctcgt 1200
   ctcccagttg ttcaccttct cgcctcgccg gcatgagaca gtgcaggact gcaattgttc 1260
   aatctatccc ggccacgtat caggtcaccg catggcttgg gatatgatga tgaactggtc 1320
   acctacaaca gccctactgg tatcgcagtt actccggatc ccacaagccg tcgtggacat 1380
   ggtggcgggg gcccactggg gagtcctggc gggccttgcc tactattcca tggcggggaa 1440
   ctgggctaag gttttgattg tgctgctact ctttgccggc gttgatgggg cgacctacgt 1500
   gacggggggg tcggaagccg attacaagga tgacgatgac aagggaggcg gtaacctctt 1560
   ttcatttggg gcgtctcaga agatccagct cataaatacc aacggcagtt ggcacatcaa 1620
   tagaactgcc ctgaactgca atgactccct ccacactggg tttcttgccg cgctattcta 1680
   cacacacaaa ttcaacgcgt ccggatgtcc agagcgcatg gccagctgcc gccccattga 1740
   agagttcgct caggggtatg gtcccatcac ttatgctgag ccctccccct cggaccagag 1800
   gccctattgc tggcactacg cgcctcgacc gtgtggtatc atacccgcgt cgcaggtgtg 1860
   tggtccagtg tactgcttca ccccaagccc tgttgtggtg gggacgaccg atcgctccgg 1920
   tgcccccacg tataattggg gggcgaatga gacggacgtg ctgtatctca acaacacgcg 1980
   gccgccgcaa ggcaactggt tcggctgcac atggatgaat ggcaccgggt tcaccaagac 2040
   gtgcgggggc cccccgtgca acatcggggg gggcggcaac aacaacacct tgacctgccc 2100
   cacggactgt ttccggaaac accccgaggc cacctacacc aaatgtggtt cgggaccttg 2160
   gttgacacct aggtgcatgg tcgactaccc atacaggctc tggcactacc cctgcaccgt 2220
   taactttacc atctttaagg ttaggatgta cgtgggaggt gtggagcaca ggctcaacgc 2280
   cgcatgcaat tggacccgag gagagcgttg taacttagag gacagggata gatcagagct 2340
   tagcccgctg ctgctgtcaa caacagagtg gcaggtgcta ccttgttcct tcaccaccct 2400
   accggctctg tccactggtt tgatccatct ccaccagaac atcgtggacg tgcaatacct 2460
   gtacggtata gggtcggcgg ttgtctccta tgcaatcaaa tgggaatatg tcttgttgct 2520
   cttcctcctc ctggcagacg cgcgcgtctg cgcctgcttg tggatgatgc tgctgatagc 2580
   tcaagctgag gccgccttag agaacctggt ggtcctcaat gcggcgtccc tggctggagc 2640
   gcatggcctt ctctctttcc ttgtgttctt ctgtgccgct tggtacatca agggcaggtt 2700
   gatccccggg gcggcgtatg ctttttacgg cgtatggccg ctgctcctac tcctgctggc 2760
   gttaccacca cgagcatacg ccatggaccg ggagatggct gcatcgtgcg gaggcgcggt 2820
   ttttgtaggt ctggcattcc tgaccttgtc accacactat aaggcattcc tcgccaagct 2880
   cctgtggtgg ttgtgctatc tcctgaccct gggggaagcc atgattcagg agtgggtacc 2940
   acccatgcag gtgcgcggcg gccgcgatgg catcgcgtgg gccgtcacta tattctgccc 3000
   gggtgtggtg tttgacatta ccaaatggct tttggcgttg cttgggcctg cttacctctt 3060
   aagggccgct ttgacacatg tgccgtactt cgtcagagct cacgctctga taagggtatg 3120
   cgctttggtg aagcagctcg cggggggtag gtatgttcag gtggcgctat tggcccttgg 3180
   caggtggact ggcacctaca tctatgacca cctcacacct atgtcggact gggccgctag 3240
   cggcctgcgc gacttagcgg tcgccgtgga acccatcatc ttcagtccga tggagaagaa 3300
   ggtcatcgtc tggggagcgg agacggctgc atgtggggac attctacatg gacttcccgt 3360
   gtccgcccga ctcggccagg agatcctcct cggcccagct gatggctaca cctccaaggg 3420
   gtggaagctc cttgctccca tcactgctta tgcccagcaa acacgaggcc tcctgggcgc 3480
   catagtggtg agtatgacgg ggcgtgacag gacagaacag gccggggaag tccaaatcct 3540
   gtccacagtc tctcagtcct tcctcggaac aaccatctcg ggggttttgt ggactgttta 3600
   ccacggagct ggcaacaaga ctctagccgg cttacggggt ccggtcacgc agatgtactc 3660
   gagtgctgag ggggacttgg taggctggcc cagcccccct gggaccaagt ctttggagcc 3720
   gtgcaagtgt ggagccgtcg acctatatct ggtcacgcgg aacgctgatg tcatcccggc 3780
   tcggagacgc ggggacaagc ggggagcatt gctctccccg agacccattt cgaccttgaa 3840
   ggggtcctcg ggggggccgg tgctctgccc taggggccac gtcgttgggc tcttccgagc 3900
   agctgtgtgc tctcggggcg tggccaaatc catcgatttc atccccgttg agacactcga 3960
   cgttgttaca aggtctccca ctttcagtga caacagcacg ccaccggctg tgccccagac 4020
   ctatcaggtc gggtacttgc atgctccaac tggcagtgga aagagcacca aggtccctgt 4080
   cgcgtatgcc gcccaggggt acaaagtact agtgcttaac ccctcggtag ctgccaccct 4140
   ggggtttggg gcgtacctat ccaaggcaca tggcatcaat cccaacatta ggactggagt 4200
   caggaccgtg atgaccgggg aggccatcac gtactccaca tatggcaaat ttctcgccga 4260
   tgggggctgc gctagcggcg cctatgacat catcatatgc gatgaatgcc acgctgtgga 4320
   tgctacctcc attctcggca tcggaacggt ccttgatcaa gcagagacag ccggggtcag 4380
   actaactgtg ctggctacgg ccacaccccc cgggtcagtg acaacccccc atcccgatat 4440
   agaagaggta ggcctcgggc gggagggtga gatccccttc tatgggaggg cgattcccct 4500
   atcctgcatc aagggaggga gacacctgat tttctgccac tcaaagaaaa agtgtgacga 4560
   gctcgcggcg gcccttcggg gcatgggctt gaatgccgtg gcatactata gagggttgga 4620
   cgtctccata ataccagctc agggagatgt ggtggtcgtc gccaccgacg ccctcatgac 4680
   ggggtacact ggagactttg actccgtgat cgactgcaat gtagcggtca cccaagctgt 4740
   cgacttcagc ctggacccca ccttcactat aaccacacag actgtcccac aagacgctgt 4800
   ctcacgcagt cagcgccgcg ggcgcacagg tagaggaaga cagggcactt ataggtatgt 4860
   ttccactggt gaacgagcct caggaatgtt tgacagtgta gtgctttgtg agtgctacga 4920
   cgcaggggct gcgtggtacg atctcacacc agcggagacc accgtcaggc ttagagcgta 4980
   tttcaacacg cccggcctac ccgtgtgtca agaccatctt gaattttggg aggcagtttt 5040
   caccggcctc acacacatag acgcccactt cctctcccaa acaaagcaag cgggggagaa 5100
   cttcgcgtac ctagtagcct accaagctac ggtgtgcgcc agagccaagg cccctccccc 5160
   gtcctgggac gccatgtgga agtgcctggc ccgactcaag cctacgcttg cgggccccac 5220
   acctctcctg taccgtttgg gccctattac caatgaggtc accctcacac accctgggac 5280
   gaagtacatc gccacatgca tgcaagctga ccttgaggtc atgaccagca cgtgggtcct 5340
   agctggagga gtcctggcag ccgtcgccgc atattgcctg gcgactggat gcgtttccat 5400
   catcggccgc ttgcacgtca accagcgagt cgtcgttgcg ccggataagg aggtcctgta 5460
   tgaggctttt gatgagatgg aggaatgcgc ctctagggcg gctctcatcg aagaggggca 5520
   gcggatagcc gagatgttga agtccaagat ccaaggcttg ctgcagcagg cctctaagca 5580
   ggcccaggac atacaacccg ctatgcaggc ttcatggccc aaagtggaac aattttgggc 5640
   cagacacatg tggaacttca ttagcggcat ccaatacctc gcaggattgt caacactgcc 5700
   agggaacccc gcggtggctt ccatgatggc attcagtgcc gccctcacca gtccgttgtc 5760
   gaccagtacc accatccttc tcaacatcat gggaggctgg ttagcgtccc agatcgcacc 5820
   acccgcgggg gccaccggct ttgtcgtcag tggcctggtg ggggctgccg tgggcagcat 5880
   aggcctgggt aaggtgctgg tggacatcct ggcaggatat ggtgcgggca tttcgggggc 5940
   cctcgtcgca ttcaagatca tgtctggcga gaagccctct atggaagatg tcatcaatct 6000
   actgcctggg atcctgtctc cgggagccct ggtggtgggg gtcatctgcg cggccattct 6060
   gcgccgccac gtgggaccgg gggagggcgc ggtccaatgg atgaacaggc ttattgcctt 6120
   tgcttccaga ggaaaccacg tcgcccctac tcactacgtg acggagtcgg atgcgtcgca 6180
   gcgtgtgacc caactacttg gctctcttac tataaccagc ctactcagaa gactccacaa 6240
   ttggataact gaggactgcc ccatcccatg ctccggatcc tggctccgcg acgtgtggga 6300
   ctgggtttgc accatcttga cagacttcaa aaattggctg acctctaaat tgttccccaa 6360
   gctgcccggc ctccccttca tctcttgtca aaaggggtac aagggtgtgt gggccggcac 6420
   tggcatcatg accacgcgct gcccttgcgg cgccaacatc tctggcaatg tccgcctggg 6480
   ctctatgagg atcacagggc ctaaaacctg catgaacacc tggcagggga cctttcctat 6540
   caattgctac acggagggcc agtgcgcgcc gaaacccccc acgaactaca agaccgccat 6600
   ctggagggtg gcggcctcgg agtacgcgga ggtgacgcag catgggtcgt actcctatgt 6660
   aacaggactg accactgaca atctgaaaat tccttgccaa ctaccttctc cagagttttt 6720
   ctcctgggtg gacggtgtgc agatccatag gtttgcaccc acaccaaagc cgtttttccg 6780
   ggatgaggtc tcgttctgcg ttgggcttaa ttcctatgct gtcgggtccc agcttccctg 6840
   tgaacctgag cccgacgcag acgtattgag gtccatgcta acagatccgc cccacatcac 6900
   ggcggagact gcggcgcggc gcttggcacg gggatcacct ccatctgagg cgagctcctc 6960
   agtgagccag ctatcagcac cgtcgctgcg ggccacctgc accacccaca gcaacaccta 7020
   tgacgtggac atggtcgatg ccaacctgct catggagggc ggtgtggctc agacagagcc 7080
   tgagtccagg gtgcccgttc tggactttct cgagccaatg gccgaggaag agagcgacct 7140
   tgagccctca ataccatcgg agtgcatgct ccccaggagc gggtttccac gggccttacc 7200
   ggcttgggca cggcctgact acaacccgcc gctcgtggaa tcgtggagga ggccagatta 7260
   ccaaccgccc accgttgctg gttgtgctct cccccccccc aagaaggccc cgacgcctcc 7320
   cccaaggaga cgccggacag tgggtctgag cgagagcacc atatcagaag ccctccagca 7380
   actggccatc aagacctttg gccagccccc ctcgagcggt gatgcaggct cgtccacggg 7440
   ggcgggcgcc gccgaatccg gcggtccgac gtcccctggt gagccggccc cctcagagac 7500
   aggttccgcc tcctctatgc cccccctcga gggggagcct ggagatccgg acctggagtc 7560
   tgatcaggta gagcttcaac ctccccccca gggggggggg gtagctcccg gttcgggctc 7620
   ggggtcttgg tctacttgct ccgaggagga cgataccacc gtgtgctgct ccatgtcata 7680
   ctcctggacc ggggctctaa taactccctg tagccccgaa gaggaaaagt tgccaatcaa 7740
   ccctttgagt aactcgctgt tgcgatacca taacaaggtg tactgtacaa catcaaagag 7800
   cgcctcacag agggctaaaa aggtaacttt tgacaggacg caagtgctcg acgcccatta 7860
   tgactcagtc ttaaaggaca tcaagctagc ggcttccaag gtcagcgcaa ggctcctcac 7920
   cttggaggag gcgtgccagt tgactccacc ccattctgca agatccaagt atggattcgg 7980
   ggccaaggag gtccgcagct tgtccgggag ggccgttaac cacatcaagt ccgtgtggaa 8040
   ggacctcctg gaagacccac aaacaccaat tcccacaacc atcatggcca aaaatgaggt 8100
   gttctgcgtg gaccccgcca aggggggtaa gaaaccagct cgcctcatcg tttaccctga 8160
   cctcggcgtc cgggtctgcg agaaaatggc cctctatgac attacacaaa agcttcctca 8220
   ggcggtaatg ggagcttcct atggcttcca gtactcccct gcccaacggg tggagtatct 8280
   cttgaaagca tgggcggaaa agaaggaccc catgggtttt tcgtatgata cccgatgctt 8340
   cgactcaacc gtcactgaga gagacatcag gaccgaggag tccatatacc aggcctgctc 8400
   cctgcccgag gaggcccgca ctgccataca ctcgctgact gagagacttt acgtaggagg 8460
   gcccatgttc aacagcaagg gtcaaacctg cggttacaga cgttgccgcg ccagcggggt 8520
   gctaaccact agcatgggta acaccatcac atgctatgtg aaagccctag cggcctgcaa 8580
   ggctgcgggg atagttgcgc ccacaatgct ggtatgcggc gatgacctag tagtcatctc 8640
   agaaagccag gggactgagg aggacgagcg gaacctgaga gccttcacgg aggccatgac 8700
   caggtactct gcccctcctg gtgatccccc cagaccggaa tatgacctgg agctaataac 8760
   atcctgttcc tcaaatgtgt ctgtggcgtt gggcccgcgg ggccgccgca gatactacct 8820
   gaccagagac ccaaccactc cactcgcccg ggctgcctgg gaaacagtta gacactcccc 8880
   tatcaattca tggctgggaa acatcatcca gtatgctcca accatatggg ttcgcatggt 8940
   cctaatgaca cacttcttct ccattctcat ggtccaagac accctggacc agaacctcaa 9000
   ctttgagatg tatggatcag tatactccgt gaatcctttg gaccttccag ccataattga 9060
   gaggttacac gggcttgacg ccttttctat gcacacatac tctcaccacg aactgacgcg 9120
   ggtggcttca gccctcagaa aacttggggc gccacccctc agggtgtgga agagtcgggc 9180
   tcgcgcagtc agggcgtccc tcatctcccg tggagggaaa gcggccgttt gcggccgata 9240
   tctcttcaat tgggcggtga agaccaagct caaactcact ccattgccgg aggcgcgcct 9300
   actggactta tccagttggt tcaccgtcgg cgccggcggg ggcgacattt ttcacagcgt 9360
   gtcgcgcgcc cgaccccgct cattactctt cggcctactc ctacttttcg taggggtagg 9420
   cctcttccta ctccccgctc ggtagagcgg cacacactag gtacactcca tagctaactg 9480
   ttcctttttt tttttttttt tttttttttt tttttttttt tttttttctt tttttttttt 9540
   ttccctcttt cttcccttct catcttattc tactttcttt cttggtggct ccatcttagc 9600
   cctagtcacg gctagctgtg aaaggtccgt gagccgcatg actgcagaga gtgccgtaac 9660
   tggtctctct gcagatcatg t 9681

<210> 42
<211> 9723
<212> DNA
<213> Artificial

<220>
<223> pTH/JFH1(3XFLAG)

<400> 42
acctgcccct aataggggcg acactccgcc atgaatcact cccctgtgag gaactactgt 60

cttcacgcag aaagcgccta gccatggcgt tagtatgagt gtcgtacagc ctccaggccc 120

ccccctcccg ggagagccat agtggtctgc ggaaccggtg agtacaccgg aattgccggg 180

aagactgggt cctttcttgg ataaacccac tctatgcccg gccatttggg cgtgcccccg 240

caagactgct agccgagtag cgttgggttg cgaaaggcct tgtggtactg cctgataggg 300

cgcttgcgag tgccccggga ggtctcgtag accgtgcacc atgagcacga atcctaaacc 360

tcaaagaaaa accaaacgta acaccaaccg ccgcccacag gacgtcaagt tcccgggcgg 420

tggccagatc gttggtggag tttacctgtt gccgcgcagg ggccccaggt tgggtgtgcg 480

cgcgactagg aagacttccg agcggtcgca acctcgtgga aggcgacaac ctatccccaa 540

ggatcgccga cccgagggca gggcctgggc tcagcctggg tacccttggc ccctctatgg 600

caacgagggc atggggtggg caggatggct cctgtcaccc cgtggctccc ggcctagttg 660

gggccccaat gacccccggc gcaggtcgcg taatttgggt aaagtcatcg atacccttac 720

atgcggcttc gccgacctca tggggtacat tccgctcgtc ggcgctccct tggggggcgc 780

tgccagggcc ttggcgcatg gcgtccgggt tctggaggac ggcgtgaact atgcaacagg 840

gaatctgccc ggttgctctt tctctatctt cctcttggct ctgctgtcct gtctaaccat 900

cccagcttcc gcttatgaag tgcgcaacgt gtccggggtg taccatgtca cgaacgactg 960

ctccaactcg agcattgtgt acgagacagg ggacatgatt atgcacaccc ctgggtgcgt 1020

gccctgtgtt cgggagaaca actcctcccg ctgctgggca gcgctcactc ccacgctcgc 1080

ggccaggaac gccagcgtcc ccaccacgac aatacggcgc cacgtcgatt tgctcgttgg 1140

ggcggctgct ttctgctccg ctatgtacgt gggggatctc tgcggatctg ttttcctcgt 1200

ctcccagttg ttcaccttct cgcctcgccg gcatgagaca gtgcaggact gcaattgttc 1260

aatctatccc ggccacgtat caggtcaccg catggcttgg gatatgatga tgaactggtc 1320

acctacaaca gccctactgg tatcgcagtt actccggatc ccacaagccg tcgtggacat 1380

ggtggcgggg gcccactggg gagtcctggc gggccttgcc tactattcca tggcggggaa 1440

ctgggctaag gttttgattg tgctgctact ctttgccggc gttgatgggg cgacctacgt 1500

gacggggggg tcggaagccg actacaaaga ccatgacggt gattataaag atcatgacat 1560

cgattacaag gatgacgatg acaagggagg cggtaacctc ttttcatttg gggcgtctca 1620

gaagatccag ctcataaata ccaacggcag ttggcacatc aatagaactg ccctgaactg 1680

caatgactcc ctccacactg ggtttcttgc cgcgctattc tacacacaca aattcaacgc 1740

gtccggatgt ccagagcgca tggccagctg ccgccccatt gaagagttcg ctcaggggta 1800

tggtcccatc acttatgctg agccctcccc ctcggaccag aggccctatt gctggcacta 1860

cgcgcctcga ccgtgtggta tcatacccgc gtcgcaggtg tgtggtccag tgtactgctt 1920

caccccaagc cctgttgtgg tggggacgac cgatcgctcc ggtgccccca cgtataattg 1980

gggggcgaat gagacggacg tgctgtatct caacaacacg cggccgccgc aaggcaactg 2040

gttcggctgc acatggatga atggcaccgg gttcaccaag acgtgcgggg gccccccgtg 2100

caacatcggg gggggcggca acaacaacac cttgacctgc cccacggact gtttccggaa 2160

acaccccgag gccacctaca ccaaatgtgg ttcgggacct tggttgacac ctaggtgcat 2220

ggtcgactac ccatacaggc tctggcacta cccctgcacc gttaacttta ccatctttaa 2280

ggttaggatg tacgtgggag gtgtggagca caggctcaac gccgcatgca attggacccg 2340

aggagagcgt tgtaacttag aggacaggga tagatcagag cttagcccgc tgctgctgtc 2400

aacaacagag tggcaggtgc taccttgttc cttcaccacc ctaccggctc tgtccactgg 2460

tttgatccat ctccaccaga acatcgtgga cgtgcaatac ctgtacggta tagggtcggc 2520

ggttgtctcc tatgcaatca aatgggaata tgtcttgttg ctcttcctcc tcctggcaga 2580

cgcgcgcgtc tgcgcctgct tgtggatgat gctgctgata gctcaagctg aggccgcctt 2640

agagaacctg gtggtcctca atgcggcgtc cctggctgga gcgcatggcc ttctctcttt 2700

ccttgtgttc ttctgtgccg cttggtacat caagggcagg ttgatccccg gggcggcgta 2760

tgctttttac ggcgtatggc cgctgctcct actcctgctg gcgttaccac cacgagcata 2820

cgccatggac cgggagatgg ctgcatcgtg cggaggcgcg gtttttgtag gtctggcatt 2880

cctgaccttg tcaccacact ataaggcatt cctcgccaag ctcctgtggt ggttgtgcta 2940

tctcctgacc ctgggggaag ccatgattca ggagtgggta ccacccatgc aggtgcgcgg 3000

cggccgcgat ggcatcgcgt gggccgtcac tatattctgc ccgggtgtgg tgtttgacat 3060

taccaaatgg cttttggcgt tgcttgggcc tgcttacctc ttaagggccg ctttgacaca 3120

tgtgccgtac ttcgtcagag ctcacgctct gataagggta tgcgctttgg tgaagcagct 3180

cgcggggggt aggtatgttc aggtggcgct attggccctt ggcaggtgga ctggcaccta 3240

catctatgac cacctcacac ctatgtcgga ctgggccgct agcggcctgc gcgacttagc 3300

ggtcgccgtg gaacccatca tcttcagtcc gatggagaag aaggtcatcg tctggggagc 3360

ggagacggct gcatgtgggg acattctaca tggacttccc gtgtccgccc gactcggcca 3420

ggagatcctc ctcggcccag ctgatggcta cacctccaag gggtggaagc tccttgctcc 3480

catcactgct tatgcccagc aaacacgagg cctcctgggc gccatagtgg tgagtatgac 3540

ggggcgtgac aggacagaac aggccgggga agtccaaatc ctgtccacag tctctcagtc 3600

cttcctcgga acaaccatct cgggggtttt gtggactgtt taccacggag ctggcaacaa 3660

gactctagcc ggcttacggg gtccggtcac gcagatgtac tcgagtgctg agggggactt 3720

ggtaggctgg cccagccccc ctgggaccaa gtctttggag ccgtgcaagt gtggagccgt 3780

cgacctatat ctggtcacgc ggaacgctga tgtcatcccg gctcggagac gcggggacaa 3840

gcggggagca ttgctctccc cgagacccat ttcgaccttg aaggggtcct cgggggggcc 3900

ggtgctctgc cctaggggcc acgtcgttgg gctcttccga gcagctgtgt gctctcgggg 3960

cgtggccaaa tccatcgatt tcatccccgt tgagacactc gacgttgtta caaggtctcc 4020

cactttcagt gacaacagca cgccaccggc tgtgccccag acctatcagg tcgggtactt 4080

gcatgctcca actggcagtg gaaagagcac caaggtccct gtcgcgtatg ccgcccaggg 4140

gtacaaagta ctagtgctta acccctcggt agctgccacc ctggggtttg gggcgtacct 4200

atccaaggca catggcatca atcccaacat taggactgga gtcaggaccg tgatgaccgg 4260

ggaggccatc acgtactcca catatggcaa atttctcgcc gatgggggct gcgctagcgg 4320

cgcctatgac atcatcatat gcgatgaatg ccacgctgtg gatgctacct ccattctcgg 4380

catcggaacg gtccttgatc aagcagagac agccggggtc agactaactg tgctggctac 4440

ggccacaccc cccgggtcag tgacaacccc ccatcccgat atagaagagg taggcctcgg 4500

gcgggagggt gagatcccct tctatgggag ggcgattccc ctatcctgca tcaagggagg 4560

gagacacctg attttctgcc actcaaagaa aaagtgtgac gagctcgcgg cggcccttcg 4620

gggcatgggc ttgaatgccg tggcatacta tagagggttg gacgtctcca taataccagc 4680

tcagggagat gtggtggtcg tcgccaccga cgccctcatg acggggtaca ctggagactt 4740

tgactccgtg atcgactgca atgtagcggt cacccaagct gtcgacttca gcctggaccc 4800

caccttcact ataaccacac agactgtccc acaagacgct gtctcacgca gtcagcgccg 4860

cgggcgcaca ggtagaggaa gacagggcac ttataggtat gtttccactg gtgaacgagc 4920

ctcaggaatg tttgacagtg tagtgctttg tgagtgctac gacgcagggg ctgcgtggta 4980

cgatctcaca ccagcggaga ccaccgtcag gcttagagcg tatttcaaca cgcccggcct 5040

acccgtgtgt caagaccatc ttgaattttg ggaggcagtt ttcaccggcc tcacacacat 5100

agacgcccac ttcctctccc aaacaaagca agcgggggag aacttcgcgt acctagtagc 5160

ctaccaagct acggtgtgcg ccagagccaa ggcccctccc ccgtcctggg acgccatgtg 5220

gaagtgcctg gcccgactca agcctacgct tgcgggcccc acacctctcc tgtaccgttt 5280

gggccctatt accaatgagg tcaccctcac acaccctggg acgaagtaca tcgccacatg 5340

catgcaagct gaccttgagg tcatgaccag cacgtgggtc ctagctggag gagtcctggc 5400

agccgtcgcc gcatattgcc tggcgactgg atgcgtttcc atcatcggcc gcttgcacgt 5460

caaccagcga gtcgtcgttg cgccggataa ggaggtcctg tatgaggctt ttgatgagat 5520

ggaggaatgc gcctctaggg cggctctcat cgaagagggg cagcggatag ccgagatgtt 5580

gaagtccaag atccaaggct tgctgcagca ggcctctaag caggcccagg acatacaacc 5640

cgctatgcag gcttcatggc ccaaagtgga acaattttgg gccagacaca tgtggaactt 5700

cattagcggc atccaatacc tcgcaggatt gtcaacactg ccagggaacc ccgcggtggc 5760

ttccatgatg gcattcagtg ccgccctcac cagtccgttg tcgaccagta ccaccatcct 5820

tctcaacatc atgggaggct ggttagcgtc ccagatcgca ccacccgcgg gggccaccgg 5880

ctttgtcgtc agtggcctgg tgggggctgc cgtgggcagc ataggcctgg gtaaggtgct 5940

ggtggacatc ctggcaggat atggtgcggg catttcgggg gccctcgtcg cattcaagat 6000

catgtctggc gagaagccct ctatggaaga tgtcatcaat ctactgcctg ggatcctgtc 6060

tccgggagcc ctggtggtgg gggtcatctg cgcggccatt ctgcgccgcc acgtgggacc 6120

gggggagggc gcggtccaat ggatgaacag gcttattgcc tttgcttcca gaggaaacca 6180

cgtcgcccct actcactacg tgacggagtc ggatgcgtcg cagcgtgtga cccaactact 6240

tggctctctt actataacca gcctactcag aagactccac aattggataa ctgaggactg 6300

ccccatccca tgctccggat cctggctccg cgacgtgtgg gactgggttt gcaccatctt 6360

gacagacttc aaaaattggc tgacctctaa attgttcccc aagctgcccg gcctcccctt 6420

catctcttgt caaaaggggt acaagggtgt gtgggccggc actggcatca tgaccacgcg 6480

ctgcccttgc ggcgccaaca tctctggcaa tgtccgcctg ggctctatga ggatcacagg 6540

gcctaaaacc tgcatgaaca cctggcaggg gacctttcct atcaattgct acacggaggg 6600

ccagtgcgcg ccgaaacccc ccacgaacta caagaccgcc atctggaggg tggcggcctc 6660

ggagtacgcg gaggtgacgc agcatgggtc gtactcctat gtaacaggac tgaccactga 6720

caatctgaaa attccttgcc aactaccttc tccagagttt ttctcctggg tggacggtgt 6780

gcagatccat aggtttgcac ccacaccaaa gccgtttttc cgggatgagg tctcgttctg 6840

cgttgggctt aattcctatg ctgtcgggtc ccagcttccc tgtgaacctg agcccgacgc 6900

agacgtattg aggtccatgc taacagatcc gccccacatc acggcggaga ctgcggcgcg 6960

gcgcttggca cggggatcac ctccatctga ggcgagctcc tcagtgagcc agctatcagc 7020

accgtcgctg cgggccacct gcaccaccca cagcaacacc tatgacgtgg acatggtcga 7080

tgccaacctg ctcatggagg gcggtgtggc tcagacagag cctgagtcca gggtgcccgt 7140

tctggacttt ctcgagccaa tggccgagga agagagcgac cttgagccct caataccatc 7200

ggagtgcatg ctccccagga gcgggtttcc acgggcctta ccggcttggg cacggcctga 7260

ctacaacccg ccgctcgtgg aatcgtggag gaggccagat taccaaccgc ccaccgttgc 7320

tggttgtgct ctcccccccc ccaagaaggc cccgacgcct cccccaagga gacgccggac 7380

agtgggtctg agcgagagca ccatatcaga agccctccag caactggcca tcaagacctt 7440

tggccagccc ccctcgagcg gtgatgcagg ctcgtccacg ggggcgggcg ccgccgaatc 7500

cggcggtccg acgtcccctg gtgagccggc cccctcagag acaggttccg cctcctctat 7560

gccccccctc gagggggagc ctggagatcc ggacctggag tctgatcagg tagagcttca 7620

acctcccccc cagggggggg gggtagctcc cggttcgggc tcggggtctt ggtctacttg 7680

ctccgaggag gacgatacca ccgtgtgctg ctccatgtca tactcctgga ccggggctct 7740

aataactccc tgtagccccg aagaggaaaa gttgccaatc aaccctttga gtaactcgct 7800

gttgcgatac cataacaagg tgtactgtac aacatcaaag agcgcctcac agagggctaa 7860

aaaggtaact tttgacagga cgcaagtgct cgacgcccat tatgactcag tcttaaagga 7920

catcaagcta gcggcttcca aggtcagcgc aaggctcctc accttggagg aggcgtgcca 7980

gttgactcca ccccattctg caagatccaa gtatggattc ggggccaagg aggtccgcag 8040

cttgtccggg agggccgtta accacatcaa gtccgtgtgg aaggacctcc tggaagaccc 8100

acaaacacca attcccacaa ccatcatggc caaaaatgag gtgttctgcg tggaccccgc 8160

caaggggggt aagaaaccag ctcgcctcat cgtttaccct gacctcggcg tccgggtctg 8220

cgagaaaatg gccctctatg acattacaca aaagcttcct caggcggtaa tgggagcttc 8280

ctatggcttc cagtactccc ctgcccaacg ggtggagtat ctcttgaaag catgggcgga 8340

aaagaaggac cccatgggtt tttcgtatga tacccgatgc ttcgactcaa ccgtcactga 8400

gagagacatc aggaccgagg agtccatata ccaggcctgc tccctgcccg aggaggcccg 8460

cactgccata cactcgctga ctgagagact ttacgtagga gggcccatgt tcaacagcaa 8520

gggtcaaacc tgcggttaca gacgttgccg cgccagcggg gtgctaacca ctagcatggg 8580

taacaccatc acatgctatg tgaaagccct agcggcctgc aaggctgcgg ggatagttgc 8640

gcccacaatg ctggtatgcg gcgatgacct agtagtcatc tcagaaagcc aggggactga 8700

ggaggacgag cggaacctga gagccttcac ggaggccatg accaggtact ctgcccctcc 8760

tggtgatccc cccagaccgg aatatgacct ggagctaata acatcctgtt cctcaaatgt 8820

gtctgtggcg ttgggcccgc ggggccgccg cagatactac ctgaccagag acccaaccac 8880

tccactcgcc cgggctgcct gggaaacagt tagacactcc cctatcaatt catggctggg 8940

aaacatcatc cagtatgctc caaccatatg ggttcgcatg gtcctaatga cacacttctt 9000

ctccattctc atggtccaag acaccctgga ccagaacctc aactttgaga tgtatggatc 9060

agtatactcc gtgaatcctt tggaccttcc agccataatt gagaggttac acgggcttga 9120

cgccttttct atgcacacat actctcacca cgaactgacg cgggtggctt cagccctcag 9180

aaaacttggg gcgccacccc tcagggtgtg gaagagtcgg gctcgcgcag tcagggcgtc 9240

cctcatctcc cgtggaggga aagcggccgt ttgcggccga tatctcttca attgggcggt 9300

gaagaccaag ctcaaactca ctccattgcc ggaggcgcgc ctactggact tatccagttg 9360

gttcaccgtc ggcgccggcg ggggcgacat ttttcacagc gtgtcgcgcg cccgaccccg 9420

ctcattactc ttcggcctac tcctactttt cgtaggggta ggcctcttcc tactccccgc 9480

tcggtagagc ggcacacact aggtacactc catagctaac tgttcctttt tttttttttt 9540

tttttttttt tttttttttt tttttttttc tttttttttt ttttccctct ttcttccctt 9600

ctcatcttat tctactttct ttcttggtgg ctccatctta gccctagtca cggctagctg 9660

tgaaaggtcc gtgagccgca tgactgcaga gagtgccgta actggtctct ctgcagatca 9720

tgt 9723

<210> 43
<211> 18
<212> DNA
<213> Artificial

<220>
<223> primer (JFH1-A)

<400> 43
tgtaaaacga cggccagt 18


<210> 44
<211> 40
<212> DNA
<213> Artificial

<220>
<223> primer (JFH1-B)

<400> 44
ggtttaggat tcgtgctcat ggtgcacggt ctacgagacc 40


<210> 45
<211> 40
<212> DNA
<213> Artificial

<220>
<223> primer (TH-C)

<400> 45
ggtctcgtag accgtgcacc atgagcacga atcctaaacc 40


<210> 46
<211> 40
<212> DNA
<213> Artificial

<220>

<223>  primer (TH-D)

<400>  46
agatagcaca accaccacag gagcttggcg aggaatgcct                              40

<210>  47
<211>  40
<212>  DNA
<213>  Artificial

<220>
<223>  primer (JFH1-E)

<400>  47
aggcattcct cgccaagctc ctgtggtggt tgtgctatct                              40

<210>  48
<211>  20
<212>  DNA
<213>  Artificial

<220>
<223>  primer (JFH1-F)

<400>  48
cagctaccga ggggttaagc                                                    20

<210>  49
<211>  11
<212>  PRT
<213>  Artificial

<220>
<223>  Epitopetag peptide (1XFLAG+Linker)

<400>  49
Asp Tyr Lys Asp Asp Asp Asp Lys Gly Gly Gly
1               5                   10

<210> 50
<211> 25
<212> PRT
<213> Artificial

<220>
<223> Epitopetag peptide (3XFLAG+Linker)

<400> 50
Asp Tyr Lys Asp His Asp Gly Asp Tyr Lys Asp His Asp Ile Asp Tyr
1               5                   10                  15

Lys Asp Asp Asp Asp Lys Gly Gly Gly
            20                  25

<210> 51
<211> 8
<212> PRT
<213> Artificial

<220>
<223> Epitopetag peptide (1XFLAG)

<400> 51
Asp Tyr Lys Asp Asp Asp Asp Lys
1               5

<210> 52
<211> 22
<212> PRT
<213> Artificial

<220>
<223> Epitopetag peptide (3XFLAG)

<400> 52
Asp Tyr Lys Asp His Asp Gly Asp Tyr Lys Asp His Asp Ile Asp Tyr
1               5                   10                  15

Lys Asp Asp Asp Asp Lys
            20

<210>  53
<211>  3
<212>  PRT
<213>  Artificial

<220>
<223>  Linker

<400>  53
Gly Gly Gly
1

<210>  54
<211>  25
<212>  PRT
<213>  Artificial

<220>
<223>  Linker

<400>  54
Gly Gly Gly Gly Ser
1               5